(19) Europäisches Patentamt European Patent Office Office européen des brevets

(11) **EP 4 321 625 A1**

(12) **EUROPEAN PATENT APPLICATION**
published in accordance with Art. 153(4) EPC

(43) Date of publication:
**14.02.2024 Bulletin 2024/07**

(21) Application number: **22784930.4**

(22) Date of filing: **06.04.2022**

(51) International Patent Classification (IPC):
**C12Q 1/02** (2006.01)    **G01N 33/574** (2006.01)
**C12Q 1/6886** (2018.01)    **C12Q 1/6869** (2018.01)
**A61P 35/00** (2006.01)

(52) Cooperative Patent Classification (CPC):
**A61P 35/00; C12Q 1/02; C12Q 1/6869;
C12Q 1/6886; G01N 33/574**

(86) International application number:
**PCT/KR2022/004909**

(87) International publication number:
**WO 2022/216021 (13.10.2022 Gazette 2022/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **06.04.2021  KR 20210044488
05.04.2022  KR 20220042365**

(71) Applicant: **Industry Foundation of Chonnam
National University
Gwangju 61186 (KR)**

(72) Inventors:
• **CHO, Jae Ho
Hwasun-gun Jeollanam-do 58121 (KR)**
• **LEE, Sung Woo
Hwasun-gun Jeollanam-do 58136 (KR)**
• **OH, In Jae
Gwangju 62062 (KR)**
• **YANG, Deok Hwan
Gwangju 62068 (KR)**

(74) Representative: **Dehns
St. Bride's House
10 Salisbury Square
London EC4Y 8JD (GB)**

Remarks:
The complete document including Reference
Table(s) and the Sequence Listing(s) can be
downloaded from the EPO website

(54) **METHOD FOR PREDICTING PROGNOSIS AND RESPONSIVENESS TO ANTICANCER THERAPY OF CANCER PATIENTS**

(57)    The present invent ion relates to a method for predicting the prognosis or responsiveness to anticancer therapy of cancer patients, the method enabling accurate prediction of prognosis and prediction of responsiveness to anticancer therapy by measuring the proportion of a specific CD8+ T cells subset. Particularly, the present invention applies machine learning to the prediction method to provide a system capable of rapidly and simply calculating the probability of patients responding to anticancer therapy, and thus can more effectively predict anticancer therapy-responsiveness and the like. Especially, the prediction method according to the present invention uses a non-invasive sample, and thus can rapidly and accurately predict a prognosis without the involvement of cancer tissue analysis and the like.

EP 4 321 625 A1

**FIG. 34**

**Description**

[Technical Field]

The present invention relates to a method of predicting the prognosis or responsiveness to anticancer therapy of cancer patients.

This application claims priority to and the benefit of Korean Patent Application No. 10-2021-0044488, filed on April 6, 2021, and Korean Patent Application No. 10-2022-0042365, filed on April 5, 2022, the disclosures of which are incorporated herein by reference in their entirety.

[Background Art]

Cancer immunotherapy is a cancer treatment method that activates the immune system of the human body to fight cancer cells, and cancer immunotherapeutic agents show anticancer effects by enhancing the specificity, memory, and adaptiveness of the immune system. That is, cancer immunotherapeutic agents have fewer side effects because they precisely attack only cancer cells using the human immune system and use the memory and adaptiveness of the immune system, so patients who are responsiveness to cancer immunotherapeutic agents can benefit from continuous anticancer effects. Examples of the cancer immunotherapeutic agents include immune checkpoint inhibitors, immune cell therapeutic agents, anticancer vaccines, and antibody-drug conjugates.

Some cancer cells avoid immunity by utilizing the immune checkpoints of immune cells, and an immune checkpoint inhibitor blocks an immune evasion signal by binding of cancer cells to binding sites of T cells, thereby preventing the formation of immunological synapses and thus T cells that are not hindered by immune evasion have a mechanism to destroy cancer cells. Immune checkpoint inhibitors include PD-1, PD-L1, CTLA-4, LAG3, TIM3, and BTLA blocking antibodies.

Particularly, interleukin-2 (IL-2) is a pleiotropic cytokine that plays essential roles in the survival, expansion, and function of various lymphocytes including T cells expressing an IL-2 receptor such as a Treg ($Foxp3^+ CD4^+$ regulatory T) cell. Since IL-2 stimulates $CD8^+$ T cells and NK cells having anti-tumor activity, it was clinically used in the US and Europe in the 1990s for the treatment of metastatic melanoma and metastatic renal cancer (Rosenberg, S.A., J Immunol, 2014. 192(12): p. 5451-8). However, IL-2 treatment was effective only in less than 10% of treated cancer patients and accompanied by serious side effects. In addition, there is also a problem that IL-2 administration induces strong expansion of Treg cells that express a high-affinity IL-2 receptor and suppress anti-tumor immunity mediated by $CD8^+$ T and NK cells (Brandenburg, S. et al., Eur J Immunol, 2008. 38(6): p. 1643-53; Facciabene, A. et al., Cancer Res, 2012. 72(9): p. 2162-71).

To address the above disadvantages of IL-2 therapy, studies have been conducted to extend the half-life of IL-2 in vivo and selectively activate CD8+ T cells and NK cells that express low-affinity IL-2 receptors without causing unnatural modifications on IL-2 sequence. Recently, a conjugate of IL-2 and anti-IL-2 monoclonal antibody was developed (Korean Patent No. 10-2099593), and its excellent immunostimulatory effect and potent antitumor effect were confirmed *in vivo.*

However, although many excellent anticancer agents have been developed, there is differences in responsiveness to anticancer treatment for each cancer patient due to resistance to an anticancer agent or the individual characteristics of a patient, and thus the therapeutic effects also vary. Accordingly, predicting the treatment responsiveness and effect of a specific anticancer agent by examining the characteristics of a cancer patient is very important in determining the treatment policy for a cancer patient. However, until now, there is no method that can accurately predict a patient's responsiveness to anticancer treatment.

[Disclosure]

[Technical Problem]

The present invention is provided to solve the above problems, and it was completed by confirming that the proportion of a specific $CD8^+$ T cell subset is related to the prognosis and responsiveness to anticancer treatment of a cancer patient.

Therefore, the present invention is directed to providing a method of predicting the prognosis or responsiveness to anticancer treatment of a cancer patient.

The present invention is also directed to providing a composition for predicting the prognosis or responsiveness to anticancer treatment.

The present invention is also directed to providing a kit for predicting the prognosis or responsiveness to anticancer treatment.

However, technical problems to be solved in the present invention are not limited to the above-described problems, and other problems which are not described herein will be fully understood by those of ordinary skill in the art from the following descriptions.

[Technical Solution]

The present invention provides a method for predicting the prognosis or responsiveness to anticancer treatment of a cancer patient, which includes measuring the proportion of a CD8$^+$ T cell subset in a biological sample isolated from a subject, or a method of providing information therefor. Here, the proportion of a CD8$^+$ T cell subset is the proportion of CD27$^+$ CD28$^+$ CCR7$^-$ CD45RA$^+$CD8$^+$ T cells (DP Temra) among total CCR7$^-$ CD45RA$^+$CD8$^+$ T cells (Temra).

In one embodiment of the present invention, the information providing method may further include measuring a Fitness factor, which is one or more selected from the group consisting of the following, but the present invention is not limited thereto:

(a) the proportion of CD27$^+$ CD28$^+$ CCR7$^-$ CD45RA$^-$ CD8$^+$ T cells (DP Tem) among total CCR7$^-$ CD45RA$^-$ CD8$^+$ T cells (Tem);
(b) the proportion of CD27$^-$ CD28$^-$ CCR7$^-$ CD45RA$^-$ CD8$^+$ T cells (DN Tem) among total CCR7$^-$ CD45RA$^-$ CD8$^+$ T cells (Tem);
(c) the proportion of perforin$^+$ CCR7$^-$ CD45RA$^-$ CD8$^+$ T cells (perforin-expressing Tem) among total CCR7$^-$ CD45RA$^-$ CD8$^+$ T cells (Tem);
(d) the proportion of granzyme B$^+$ CCR7$^-$ CD45RA$^-$ CD8$^+$ T cells (granzyme B-expressing Tem) among total CCR7$^-$ CD45RA$^-$ CD8$^+$ T cells (Tem); and
(e) the proportion of IL-2$^+$ CCR7$^-$ CD45RA$^-$ CD8$^+$ T cells (IL-2-expressing Tem) among total CCR7$^-$ CD45RA$^-$ CD8$^+$ T cells (Tem).

In another embodiment of the present invention, the anticancer treatment may be one or more selected from the group consisting of cancer immunotherapy, chemotherapy, radiation therapy, and a combination of chemotherapy-radiation therapy, but the present invention is not limited thereto.

In still another embodiment of the present invention, the cancer immunotherapy may be treatment with an immune checkpoint inhibitor or an immune cell activator, but the present invention is not limited thereto.

In yet another embodiment of the present invention, the cancer immunotherapy may be one or more selected from the group consisting of anti-PD-L1 treatment, anti-PD-1 treatment, anti-CTLA-4 treatment, anti-LAG3 treatment, anti-TIM3 treatment, anti-BTLA treatment, anti-4-1BB treatment, anti-OX40 treatment, cytokine-mediated treatment, IL-2 treatment, and cytokine receptor treatment, but the present invention is not limited thereto.

In yet another embodiment of the present invention, the anticancer treatment is treatment with an immune checkpoint inhibitor, and when the proportion of CD27$^+$ CD28$^+$ CCR7$^-$ CD45RA$^+$ CD8$^+$ T cells (DP Temra) among total CCR7$^-$ CD45RA$^+$ CD8$^+$ T cells (Temra) is lower than that of the control, the information providing method may further include determining that a subject has a better prognosis, or predicting that the responsiveness to anticancer treatment is higher, but the present invention is not limited thereto.

In yet another embodiment of the present invention, the anticancer treatment is treatment with an immune checkpoint inhibitor, and when one or more of the following characteristics are satisfied, the information providing method may further include determining that a subject has a better prognosis, or predicting that the responsiveness to anticancer treatment is higher, but the present invention is not limited thereto:

(a) the proportion of CD27$^+$ CD28$^+$ CCR7$^-$ CD45RA$^-$ CD8$^+$ T cells (DP Tem) among total CCR7$^-$ CD45RA$^-$ CD8$^+$ T cells (Tern) is higher than that of the control; and
(b) the proportion of CD27$^-$ CD28$^-$ CCR7$^-$ CD45RA$^-$ CD8$^+$ T cells (DN Tem) among total CCR7$^-$ CD45RA$^-$ CD8$^+$ T cells (Tern) is lower than that of the control.

In yet another embodiment of the present invention, the anticancer treatment is treatment with an immune checkpoint inhibitor, and the subject is a subject before receiving anticancer treatment, and when one or more of the following characteristics are satisfied, the information providing method may further include determining that a subject has a better prognosis, or predicting that the responsiveness to anticancer treatment is higher, but the present invention is not limited thereto:

(c) the proportion of perforin$^+$ CCR7$^-$ CD45RA$^-$ CD8$^+$ T cells (perforin-expressing Tem) among total CCR7$^-$ CD45RA$^-$ CD8$^+$ T cells (Tern) is lower than that of the control;
(d) the proportion of granzyme B$^+$ CCR7$^-$ CD45RA$^-$ CD8$^+$ T cells (granzyme B-expressing Tem) among total CCR7$^-$

CD45RA⁻ CD8⁺ T cells (Tern) is lower than that of the control; and

(e) the proportion of IL-2⁺ CCR7⁻ CD45RA⁻ CD8⁺ T cells (IL-2-expressing Tem) among total CCR7⁻ CD45RA⁻ CD8⁺ T cells (Tern) is higher than that of the control.

In yet another embodiment of the present invention, the anticancer treatment is IL-2 treatment, the subject is a subject before receiving anticancer treatment, and when the proportion of CCR7⁻ CD45RA⁺ CD8⁺ T cells (Temra) among total CD8⁺ T cells is higher than that of the control, the information providing method may further include determining that a subject has a better prognosis, or predicting that the responsiveness to anticancer treatment is higher, but the present invention is not limited thereto.

In yet another embodiment of the present invention, the anticancer treatment is IL-2 treatment, and when the proportion of CD27⁺ CD28⁺ CCR7⁻ CD45RA⁻ CD8⁺ T cells (DP Tem) among total CCR7⁻ CD45RA⁻ CD8⁺ T cells (Tern) is lower than that of the control, the information providing method may further include determining that a subject has a better prognosis, or predicting that the responsiveness to anticancer treatment is higher, but the present invention is not limited thereto.

In yet another embodiment of the present invention, the IL-2 treatment may be treatment with a conjugate of IL-2; and an anti-IL-2 antibody or a fragment thereof, but the present invention is not limited thereto.

In yet another embodiment of the present invention, the control may be a biological sample isolated from a healthy subject or a cancer patient showing a complete response or partial response after anticancer treatment, but the present invention is not limited thereto.

In yet another embodiment of the present invention, the sample may be one or more selected from the group consisting of blood, peripheral blood, whole blood, plasma, and cells, but the present invention is not limited thereto.

In yet another embodiment of the present invention, the CD8⁺ T cell subset may be a CD8⁺ T cell subset in peripheral blood, but the present invention is not limited thereto.

In yet another embodiment of the present invention, the cancer may be any one or more selected from the group consisting of melanoma, small cell lung cancer, non-small cell lung cancer, gliomas, liver cancer, thyroid tumors, stomach cancer, prostate cancer, ovarian cancer, bladder cancer, lung cancer, colorectal cancer, breast cancer, Hodgkin's lymphoma, urothelial carcinoma, glioblastoma, endometrial cancer, cervical cancer, kidney cancer, colon cancer, pancreatic cancer, esophageal carcinoma, head and neck cancer, mesothelioma, sarcoma, cholangiocarcinoma, small intestine adenocarcinoma, childhood malignancies, epidermal cancer, testicular cancer, blood cancer, and brain cancer, but the present invention is not limited thereto.

In addition, the present invention provides a composition for predicting the prognosis or responsiveness to anticancer treatment of a cancer patient, which includes an agent for detecting CCR7⁻ CD45RA⁺ CD8⁺ T cells (Temra) and CD27⁺ CD28⁺ CCR7⁻ CD45RA⁺ CD8⁺ T cells (DP Temra) as an active ingredient.

In one embodiment of the present invention, the composition may further include an agent for detecting one or more CD8⁺ T cell subsets selected from the group consisting of the following, but the present invention is not limited thereto:

CCR7⁻ CD45RA⁻ CD8⁺ T cells (Tem);
CD27⁺ CD28⁺ CCR7⁻ CD45RA⁻ CD8⁺ T cells (DP Tern);
CD27⁻ CD28⁻ CCR7⁻ CD45RA⁻ CD8⁺ T cells (DN Tern);
Perforin⁺ CCR7⁻ CD45RA⁻ CD8⁺ T cells (perforin-expressing Tern);
Granzyme B⁺ CCR7⁻ CD45RA⁻ CD8⁺ T cells (granzyme B-expressing Tern); and
IL-2⁺ CCR7⁻ CD45RA⁻ CD8⁺ T cells (IL-2-expressing Tern).

In another embodiment of the present invention, the agent may be an antibody or an aptamer, but the present invention is not limited thereto.

In addition, the present invention provides a kit for predicting the prognosis or responsiveness to anticancer treatment of a cancer patient, which includes the composition according to the present invention.

In one embodiment of the present invention, the kit may further include an agent for detecting total CD8⁺ T cells, but the present invention is not limited thereto.

In another embodiment of the present invention, the kit may further include instructions that describe the method of predicting the prognosis or responsiveness to anticancer treatment of a cancer patient according to the present invention or the information providing method therefor, but the present invention is not limited thereto.

In addition, the present invention provides a use of the composition according to the present invention for predicting the prognosis or responsiveness to anticancer treatment of a cancer patient.

In addition, the present invention provides a use of the composition for preparing an agent for predicting the prognosis or responsiveness to anticancer treatment of a cancer patient.

In addition, the present invention provides a method of treating cancer, which includes the following steps:

(S1) measuring the proportion of CD27$^+$ CD28$^+$ CCR7$^-$ CD45RA$^+$ CD8$^+$ T cells (DP Temra) among total CCR7$^-$ CD45RA$^+$ CD8$^+$ T cells (Temra) in a biological sample isolated from a subject;

(S2) predicting the prognosis or responsiveness to anticancer treatment of a cancer patient by comparing the proportion of DP Temra among total Temra measured in (S1); and

(S3) performing anticancer treatment on the subject who is predicted to have a better prognosis or responsiveness to the anticancer treatment.

[Advantageous Effects]

The present invention relates to a method of predicting the prognosis or responsiveness to anticancer treatment of a cancer patient, and thus prognosis and the responsiveness to anticancer treatment can be accurately predicted by measuring the proportion of a specific CD8$^+$ T cell subset. Particularly, the present invention provides a system capable of rapidly and simply calculating the probability that a patient will show a response to anticancer treatment by applying machine learning to the prediction method, so the responsiveness of a cancer patient to the anticancer treatment can be more efficiently predicted. Particularly, since the prediction method according to the present invention uses a non-invasive sample, rapid and accurate prognosis can be predicted without cancer tissue analysis or the like.

[Description of Drawings]

FIGS. 1A-1H present the characteristics of CD8$^+$ TILs of cancer patients.

FIG. 1A presents a flow cytometry result that distinguishes CD8$^+$ T cell subsets (Tn, Tcm, Tem, and Temra) from PBMCs (left) and TILs (right) using CCR7 and CD45RA.

FIG. 1B present the proportions of CD8$^+$ T cell subsets in TILs.

FIG. 1C presents the relative telomere lengths of tilTem and tilTemra. Telomere lengths were measured by the ratio of a telomere and 36B4. Relative telomere lengths were calculated by dividing with the telomere length of the tilTem obtained from the same tumor.

FIGS. 1D to 1G are flow cytometry results for assessing the proportions of tilTem or tilTemra producing perforin (FIG. 1D), granzyme B (FIG. 1E), IFN-$\gamma$ (FIG. 1F), and IL-2 (FIG. 1G) after restimulating TILs for 4 hours.

FIG. 1H shows the result of stimulating with anti-CD3 (5 $\mu$g/mL), anti-CD28 (2 $\mu$g/mL), and IL-2 (10 ng/mL) for 7 days after purifying tilTem and tilTemra and labeling them with CTV. CTV dilution was assessed by flow cytometry, and cells with diluted CTV were considered as the proliferated cells (n=6). Statistical significance was evaluated by a Student's T test.

FIGS. 2A-2D are transcriptome profile results comparing tilTemra and peripheral Temra (pTemra).

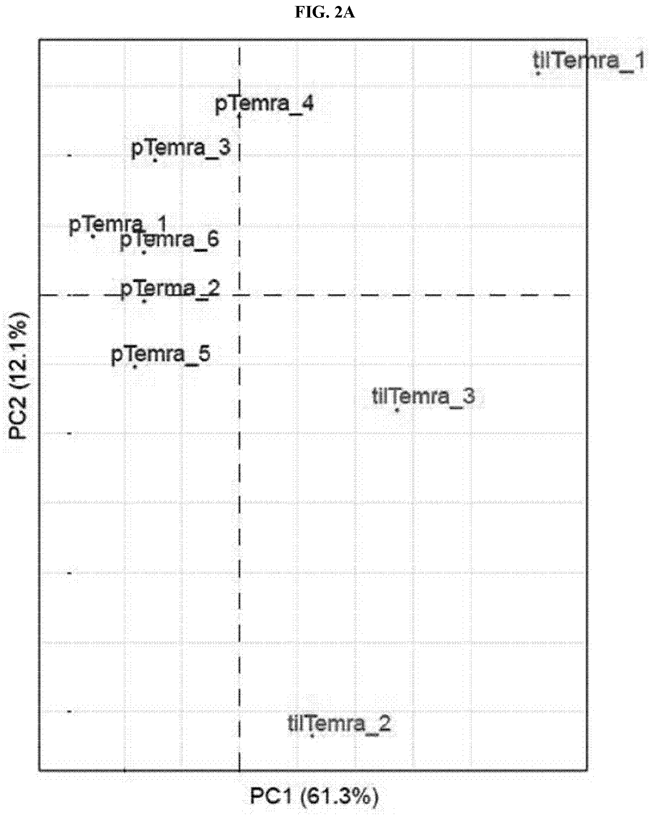

FIG. 2A shows the result of principal component analysis (PCA) for tilTemra and pTemra.

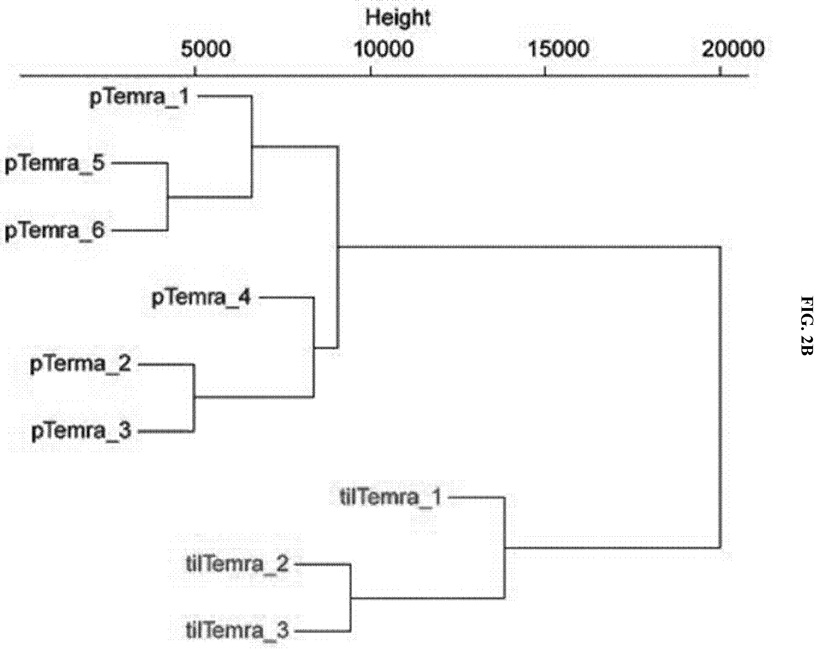

FIG. 2B shows the result of hierarchical clustering analysis.

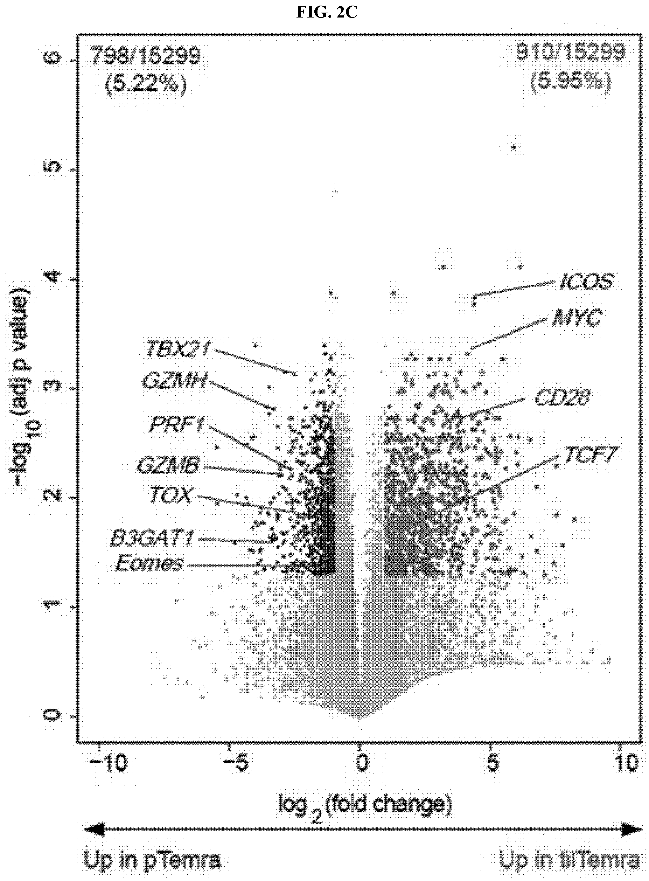

FIG. 2C shows the analysis result for differentially expressed genes (DEGs). Volcano plots were generated based on 15,299 genes with meaningful expression levels (FPKM>0). P values were first analyzed by a t-test and expressed as P values adjusted by the Benjamini-Hochberg procedure. Fold changes were calculated by dividing the mean expression level of pTemra by the mean expression level of tilTemra. Red dots represent genes upregulated in tilTemra (fold change > 2, adjusted P value < 0.05), and blue dots represent genes upregulated in pTemra (fold change > 0.5, adjusted P value < 0.05).

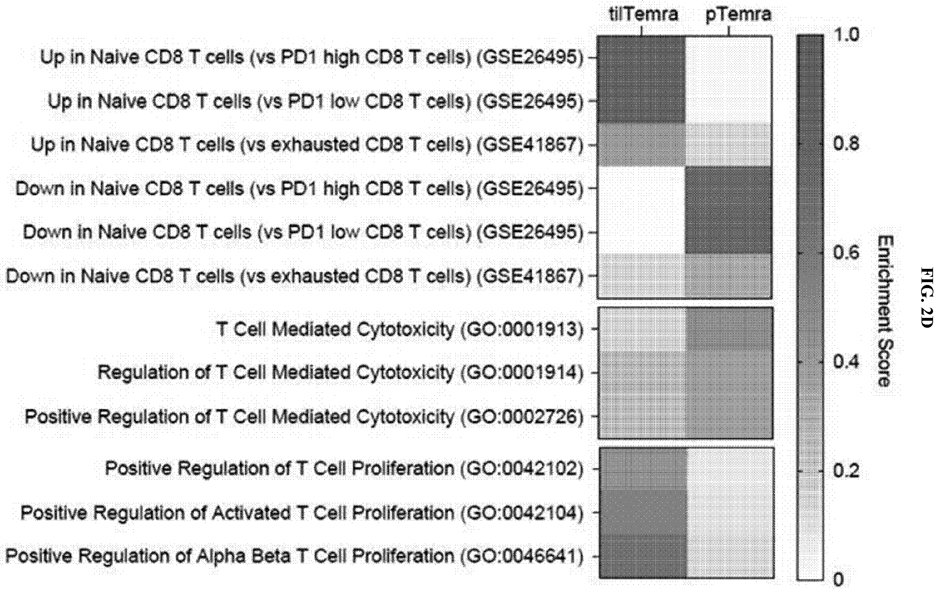

FIG. 2D shows the summary of GSEA results using gene sets obtained from Broad Institute and Gene Ontology. The enrichment score represents the absolute value of the highest or lowest running enrichment score.

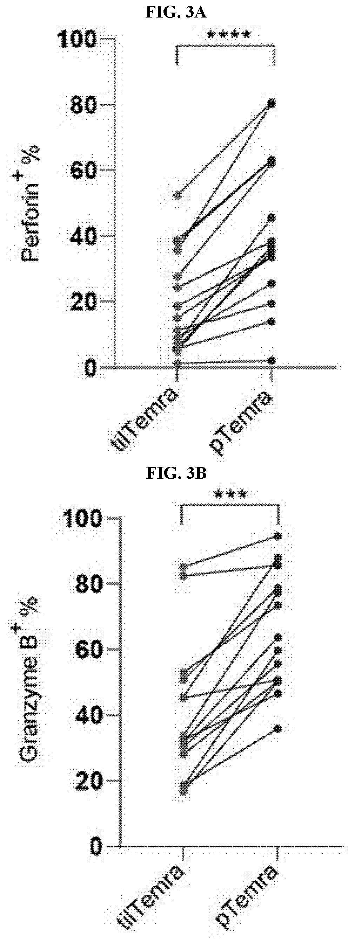

FIGS. 3A-3J are result comparing the functional phenotypes of tilTemra and pTemra.

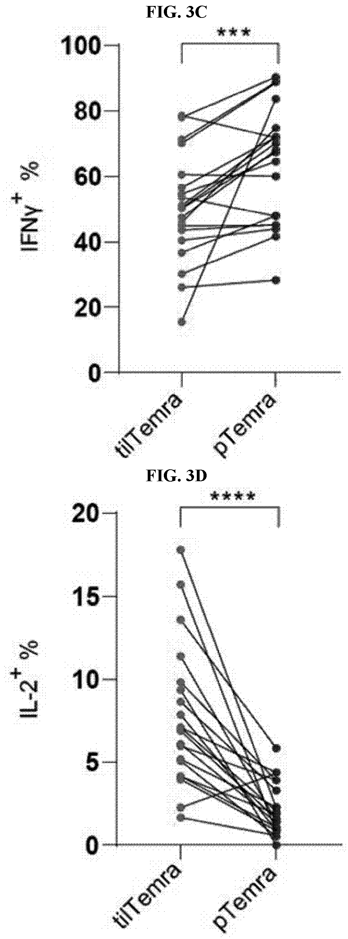

FIGS. 3A-3D show the flow cytometry result of analyzing the proportions of tilTem or tilTemra producing perforin (FIG. 3A), granzyme B (FIG. 3B), IFN-$\gamma$ (FIG. 3C), and IL-2 (FIG. 3D) after restimulating TILs and PBMCs for 4 hours.

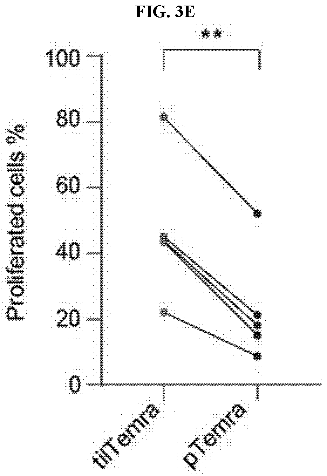

FIG. 3E shows the result of stimulating with anti-CD3 (5 $\mu$g/mL), anti-CD28 (2 $\mu$g/mL), and IL-2 (10 ng/mL) for 7 days after purifying tilTemra and pTemra and labeling them with CTV. The proliferated cells were assessed by flow cytometry.

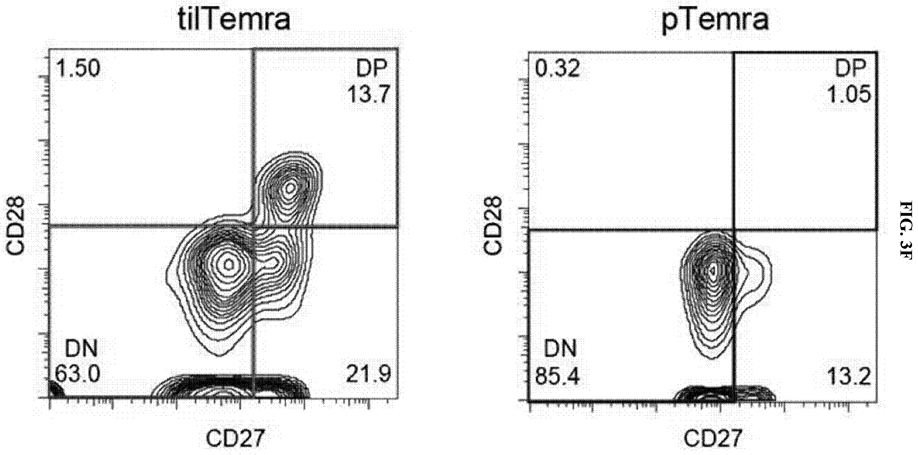

FIG. 3F shows the flow cytometry data for CD27 and CD28 expression levels of tilTemra (left) and pTemra (right).

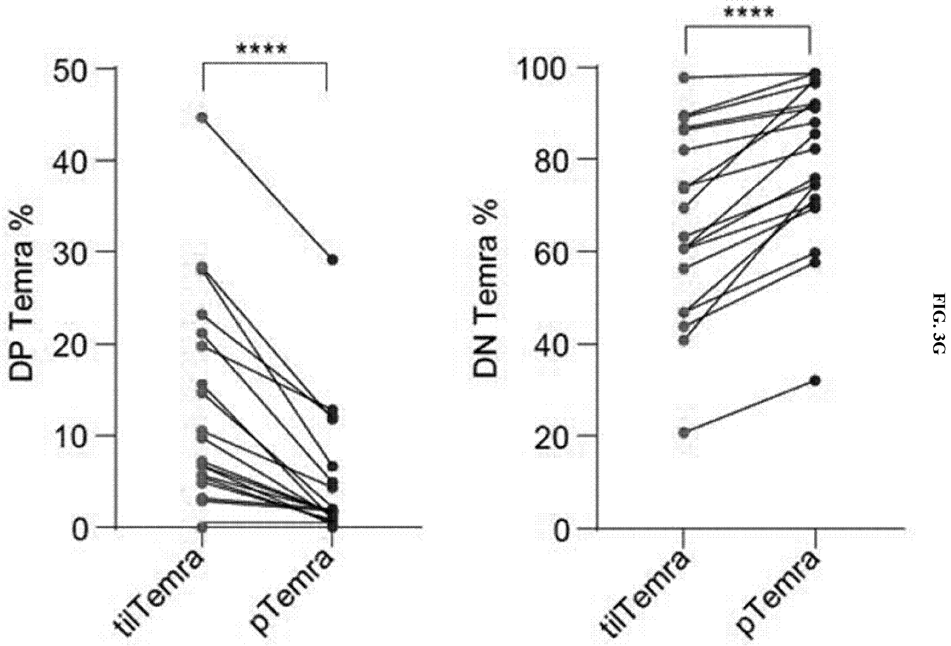

FIG. 3G shows the proportions of the DP (left) and DN (right) phenotypes of tilTemra and pTemra.

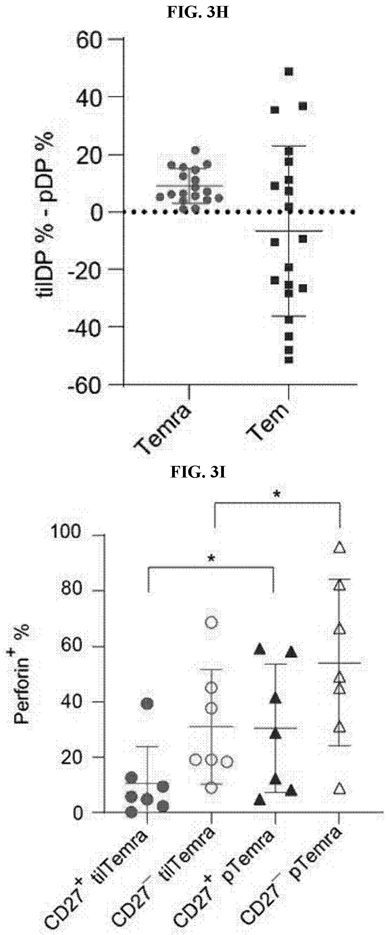

FIG. 3H is a result showing the difference in DP phenotype proportion calculated by subtracting a DP phenotype proportion (pDP %) in PBMCs from the DP phenotype proportion (tilDP %) in TILs. As seen from this drawing, the difference value (tilDP % - pDP %) was always higher than zero in Temra, but not in Tem.

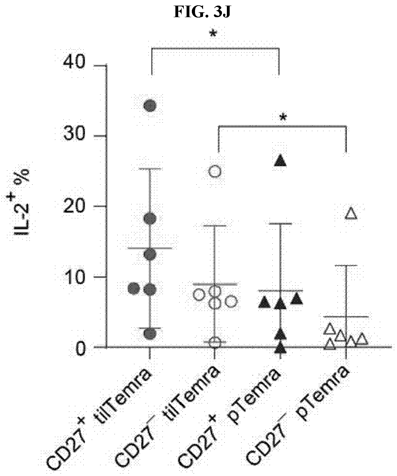

FIGS. 3I and 3J show the proportions of cells generating perforin (FIG. 3I) and IL-2 (FIG. 3J) after restimulating the TILs and PBMCs for 4 hours through flow cytometry.

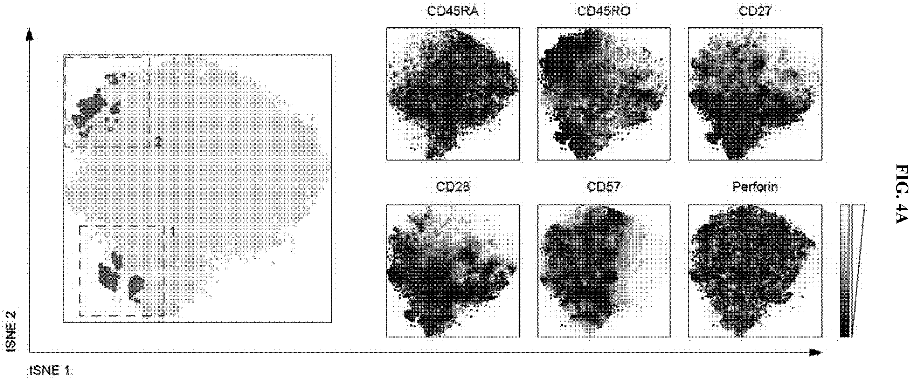

FIGS. 4A-4E show the differentiation trajectory of CD8$^+$ TILs into Temra.

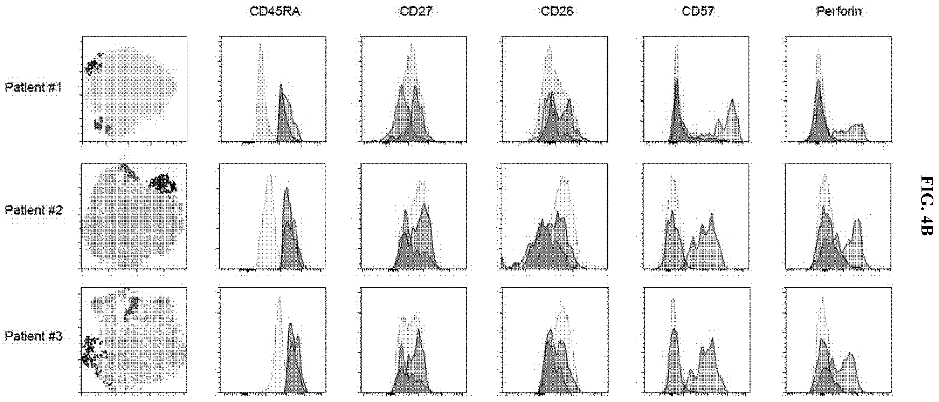

FIGS. 4A and 4B show the result of tSNE analysis of CD8$^+$ TILs. TILs were gated on CD3$^+$CD8$^+$ cells before tSNE analysis. The following parameters were used in tSNE analysis: CD27, CD28, CD45RA, CD45RO, CD8, CD3, CD5, CCR7, perforin, and CD57. Red dots in the left panel of FIG. 4A represent CD45RA$^+$CD45RO$^-$CCR7$^-$ Temra, and two types of tilTemra clustering (shown by 1 and 2) were observed. The relative expression levels of the indicated molecules were represented with a color scale from black (low) to yellow (high). In FIG. 4B, two types of tilTemra clustering (represented in red and blue, respectively) were observed in three patients. The expression of the indicated molecule was shown with a histogram. A gray histogram represents tilTem.

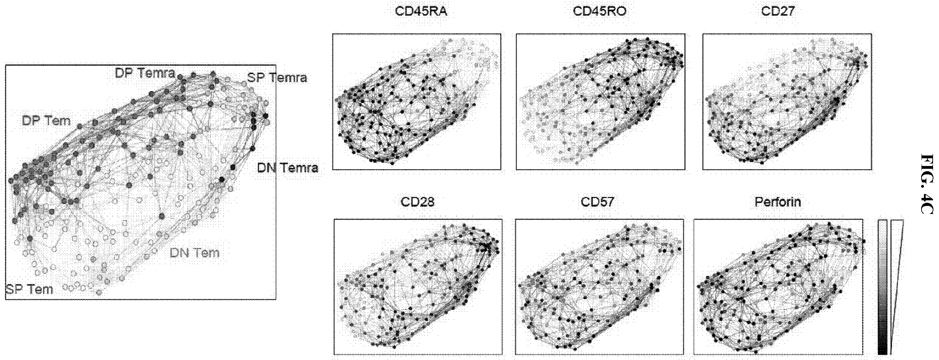

FIG. 4C shows the result of single cell trajectory analysis with FLOW MAP using CD3$^+$CD8$^+$ TILs. The following clustering variables were used for FLOW MAP: CD27, CD28, CD45RA, CD45RO, CD8, CD3, CD5, and CCR7. The relative expression of CD27, CD28, CD45RA, and CD45RO of each node was analyzed using Gephi software: pink, DP Tem; yellow, SP Tem; light blue, DN Tem; red, DP Temra; green, SP Temra; and dark blue, DN Temra. The relative expression levels of the indicated molecules were represented with a color scale from black (low) to yellow (high).

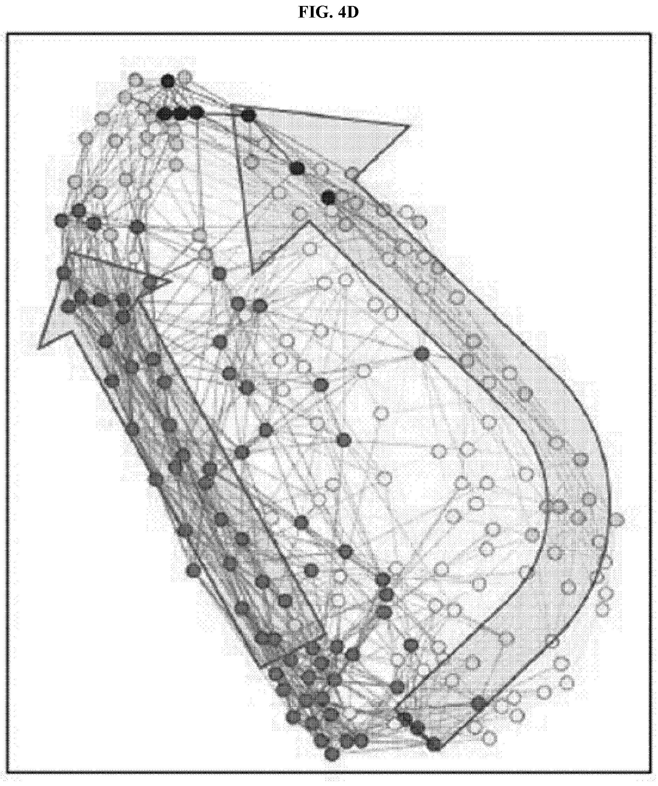

FIG. 4D shows the bifurcated differentiation trajectory shown by two arrows (red and blue).

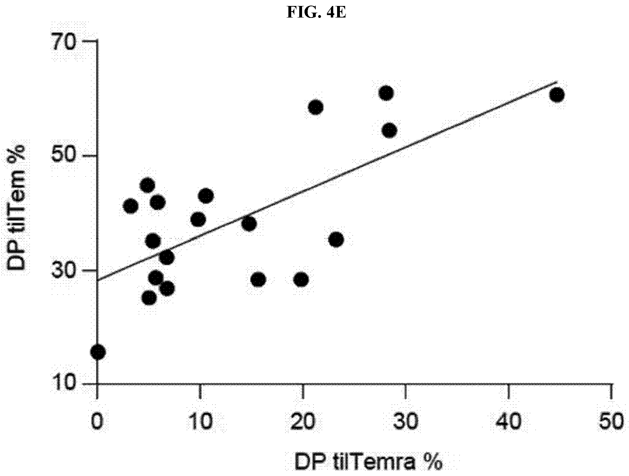

FIG. 4E is the graph showing the correlation between DP tilTem and DP tilTemra.

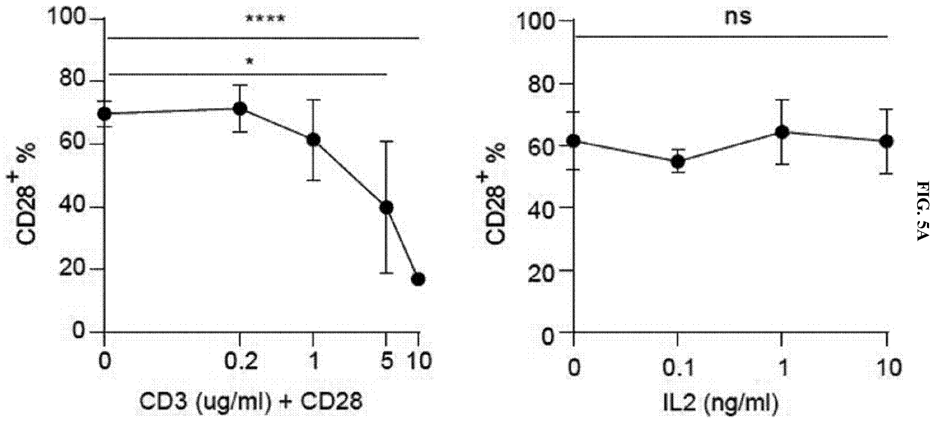

FIGS. 5A-5H are views showing the regulation of the expression of CD27, CD28, and CD45RA in a Temra differentiation process.

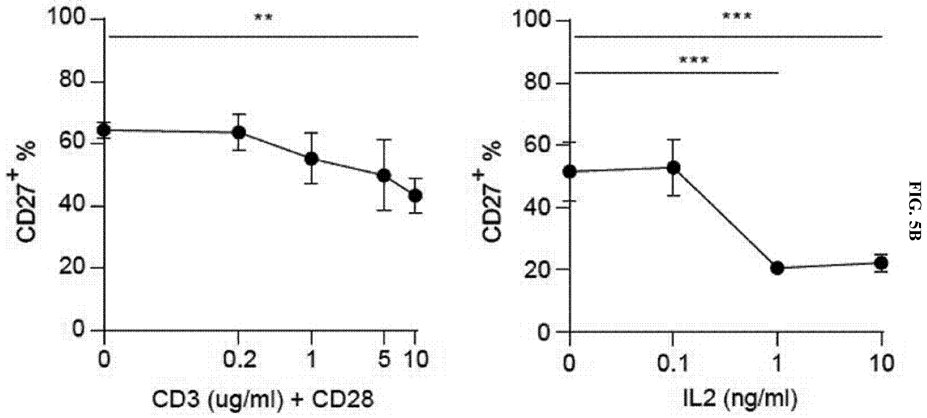

FIGS. 5A and 5B show the result of confirming the expression of CD28 (FIG. 5A) and CD27 (FIG. 5B) after stimulating tilTem with anti-CD3 (0~10 μg/mL) and anti-CD28 (0~4 μg/mL) for 7 days; or stimulating tilTem with IL-2 (0~10 ng/mL) for 7 days.

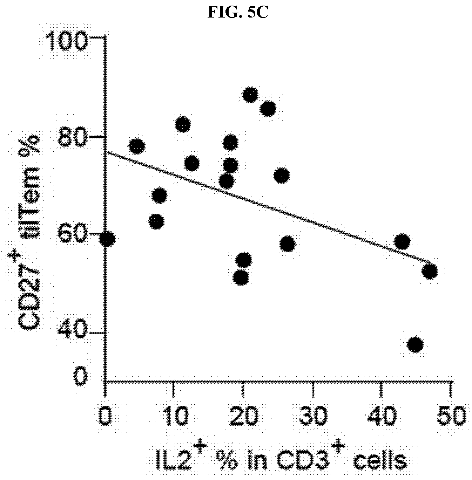

FIG. 5C shows the flow cytometry result of confirming the frequency of IL-2 producing CD3$^+$ cells after restimulating TILs. The proportion of IL-2$^+$ cells in CD3$^+$ cells was plotted together with the proportion of CD27$^+$ cells in tilTem.

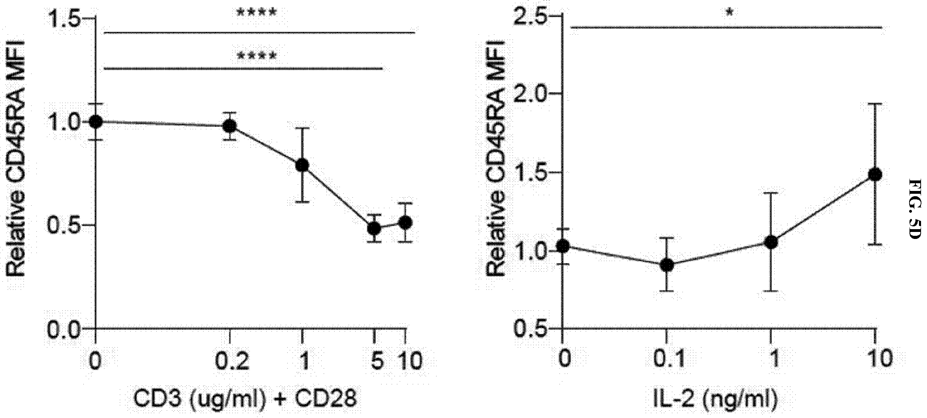

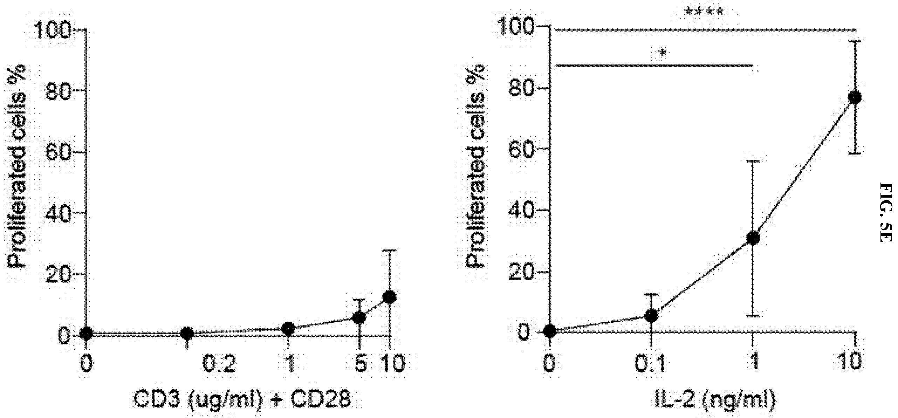

FIGS. 5D and 5E show the results that confirm CD45RA expression (FIG. 5D) and the proportion of proliferated cells (FIG. 5E; confirmed by flow cytometry) after labeling tilTemra with CTV, stimulating it with stimulants indicated in the drawings for 7 days. Relative CD45RA MFI was calculated by division by the mean CD45RA MFI of non-stimulated cells.

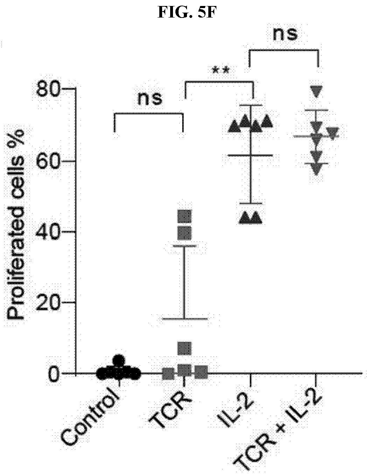

FIG. 5F shows the result of confirming cell proliferation after labeling tilTemra with CTV and stimulating it with TCR (anti-CD3 (5 μg/mL) and anti-CD28 (2 μg/mL)), IL-2 (10 ng/mL), or a combination thereof for 7 days.

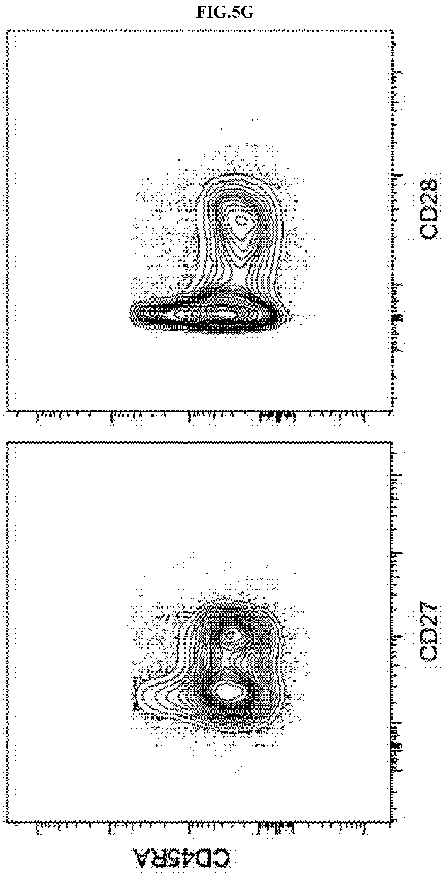

FIG. 5G is a representative diagram of flow cytometry showing CD45RA reexpression in tilTem. CD45RA$^{lo}$CCR7$^-$ tilTem was gated from TILs, and CD45RA expression was assessed with CD27 or CD28.

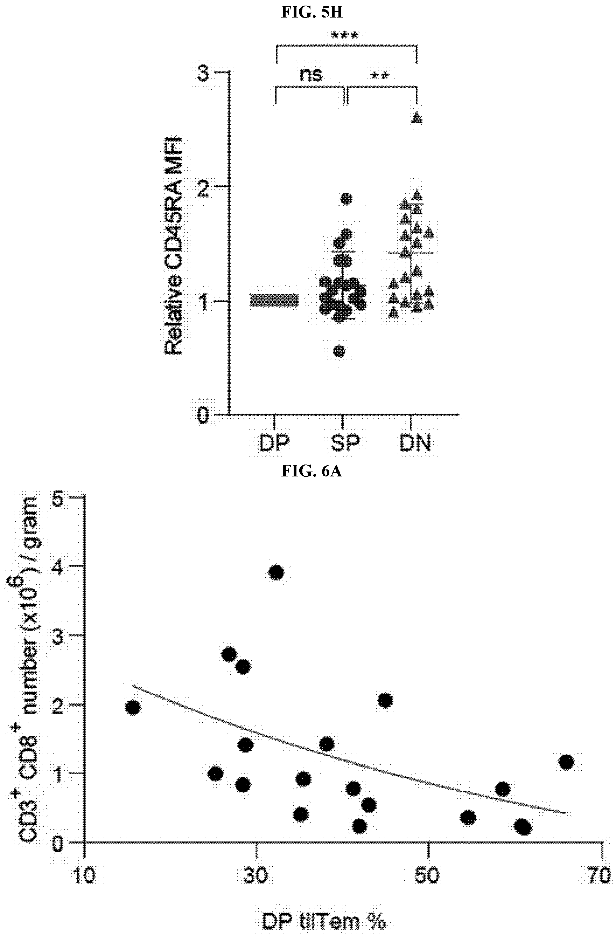

FIG. 5H is the result confirming CD45RA expression of DP, SP, and DN tilTem. Relative CD45RA MFI was calculated by division by the MFI value of DP cells derived from the same tumor.

FIGS. 6A-6I are results showing the correlation between DP Temra; and the number of CD8$^+$ TILs and TMBs.

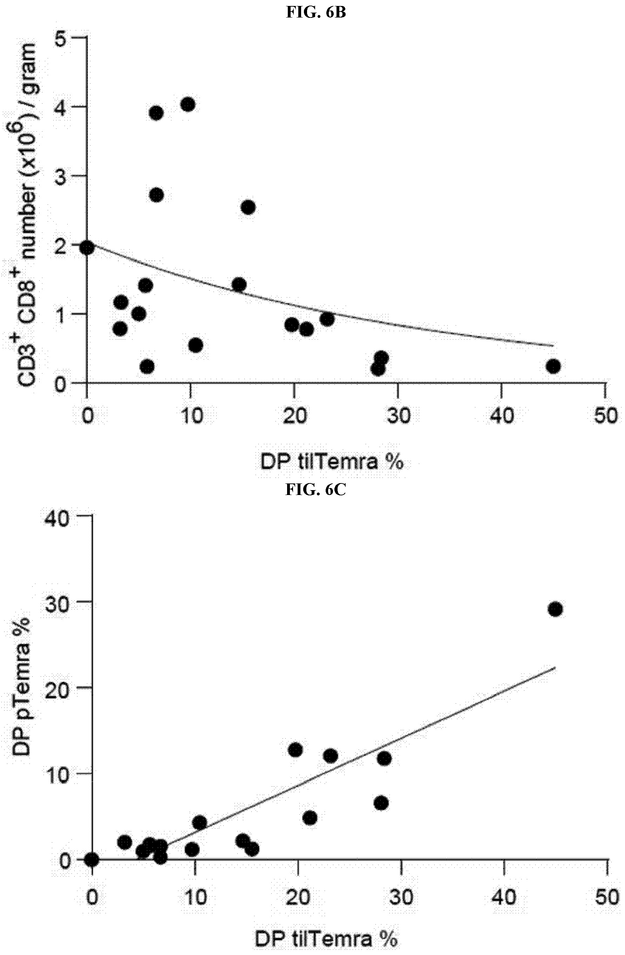

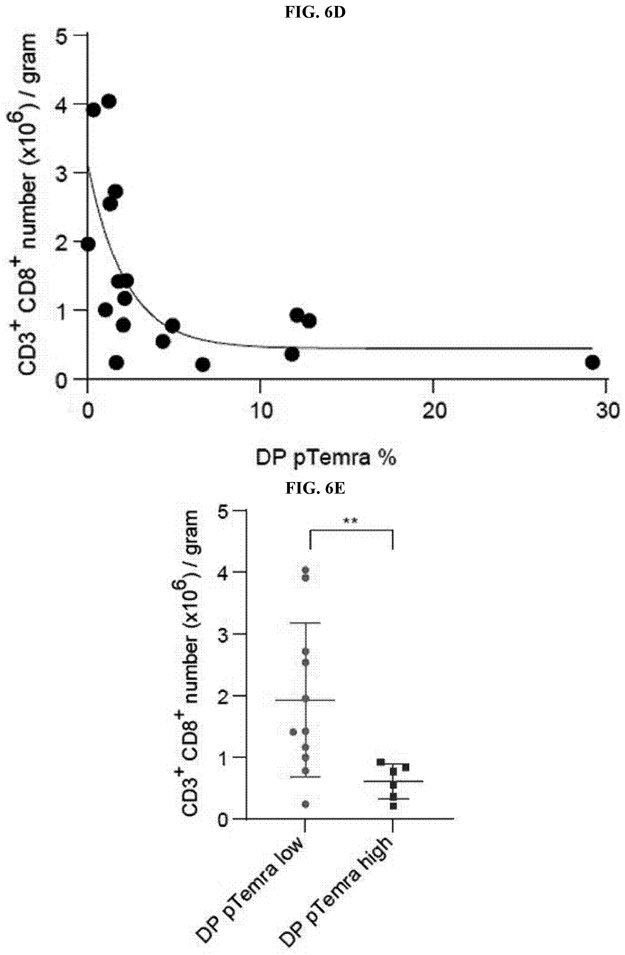

FIGS. 6A-6D are graphs showing the correlation between the number of CD8$^+$ TILs and DP tilTem (FIG. 6A); the correlation of the number of CD8$^+$ TILs and DP tilTemra (FIG. 6B); the correlation between the proportion of DP pTemra and the proportion of DP tilTemra (FIG. 6C); and the correlation between the number of CD8$^+$ TILs and the proportion of DP pTemra (FIG. 6D).

FIG. 6E shows the result of comparing the number of CD8$^+$ TILs in patients with a low proportion of DP pTemra (<6%; DP pTemra low) or a high proportion of DP pTemra (>6%; DP pTemra high).

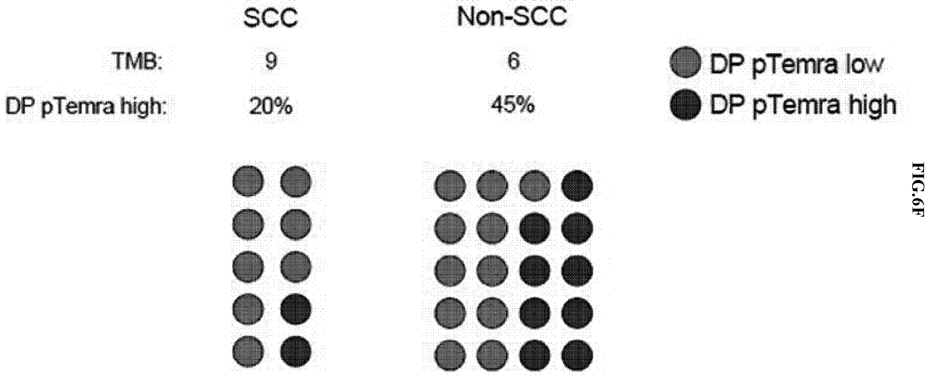

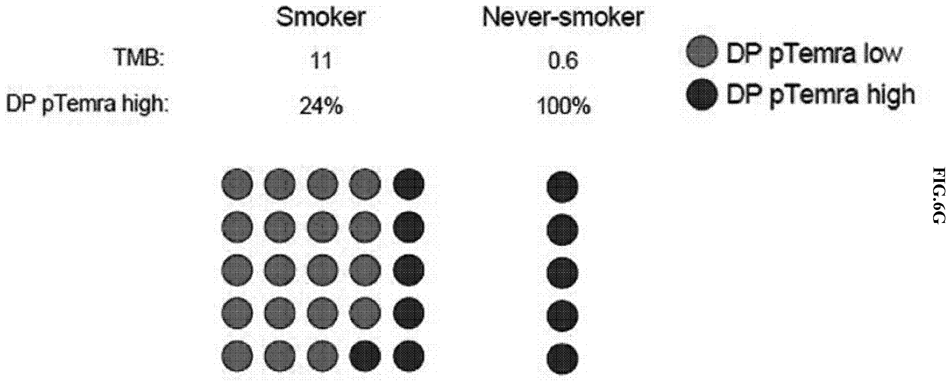

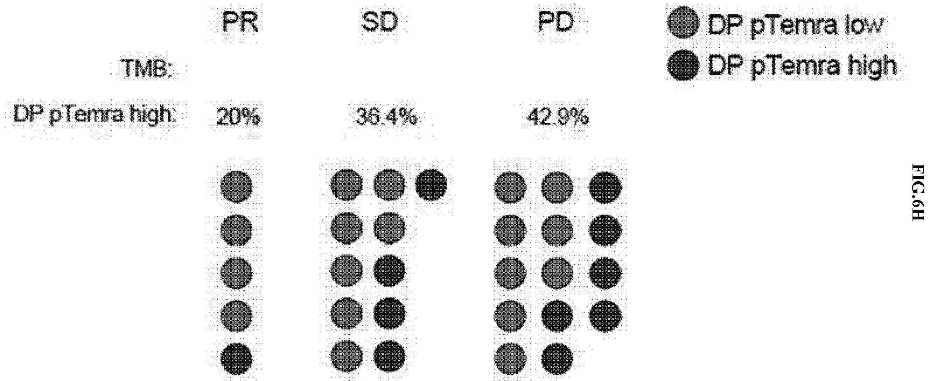

FIGS. 6F-6H show, after analyzing the proportion of DP pTemra in the blood of NSCLC patients through flow cytometry, the result of confirming the proportions of high-DP pTemra patients in squamous cell carcinoma (SCC)(TMB: 9 (9 coding somatic mutations per MB)) and non-SCC)(TMB: 6) patient groups, respectively (FIG. 6F), the result of confirming the proportions of high-DP pTemra patients in smoker (TMB: 11); and non-smoker (TMB: 0.6) patient groups, respectively (FIG. 6G), and the result of confirming the proportions of high-DP pTemra patients in PR, SD, and PD patient groups classified based on the responsiveness to an immune checkpoint inhibitor (FIG. 6H).

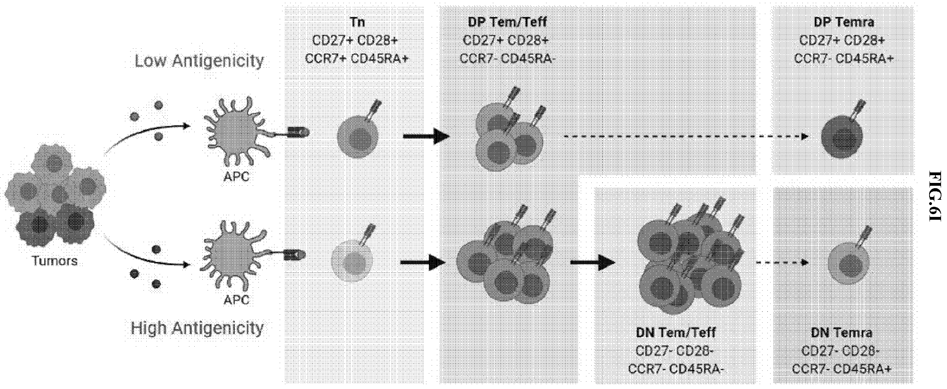

FIG. 6I is a view illustrating the process by which tumor antigens form the differentiation trajectory of CD8$^+$ TILs.

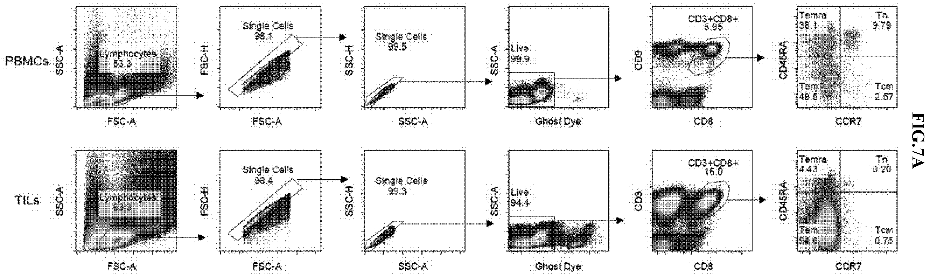

FIGS. 7A-7F are views showing the characteristics of peripheral CD8$^+$ T cell subsets.

FIG. 7A shows the gating strategy to distinguish CD8$^+$ T cell subsets from PBMCs and TILs.

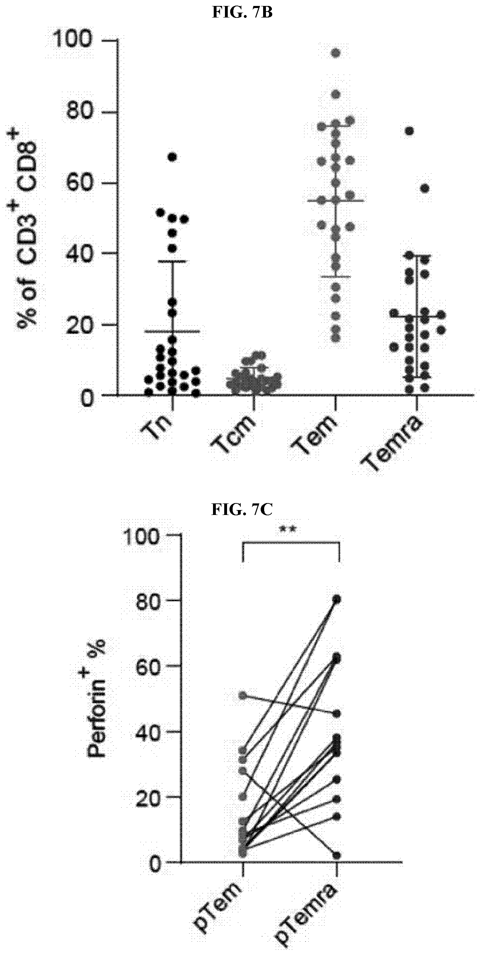

FIG. 7B shows the proportions of CD8$^+$ T cell subsets among PBMC-derived CD8$^+$ T cells.

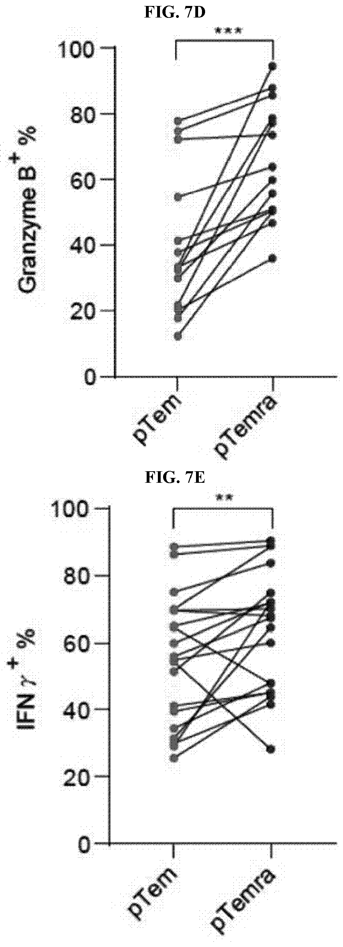

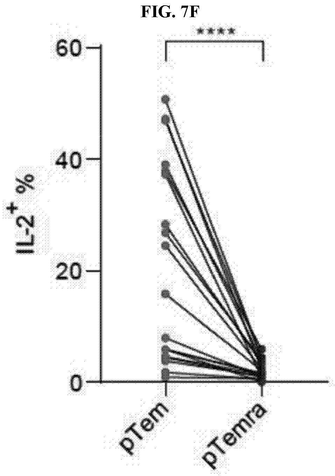

FIGS. 7C-7F show the results assessing the proportions of pTem or pTemra producing perforin (FIG. 7C), granzyme B (FIG. 7D), IFN-γ (FIG. 7E), and IL-2 (FIG. 7F) through flow cytometry, after restimulating PBMCs for 4 hours. Statistic significance was performed with a Wilcoxon matched-pairs signed rank test.

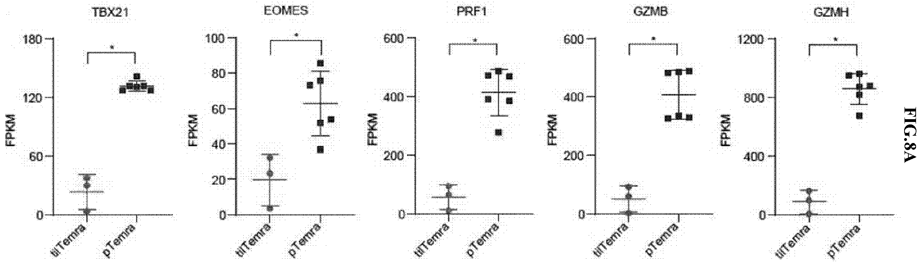

FIGS. 8A-8G are views showing the results of analyzing differentially expressed genes (DEGs) between tilTemra and pTemra.

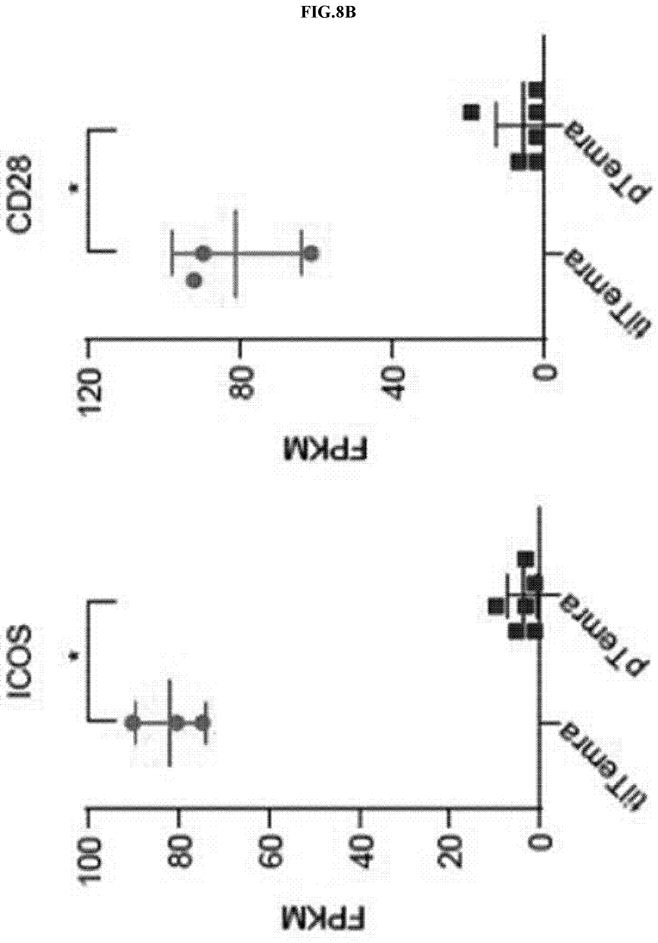

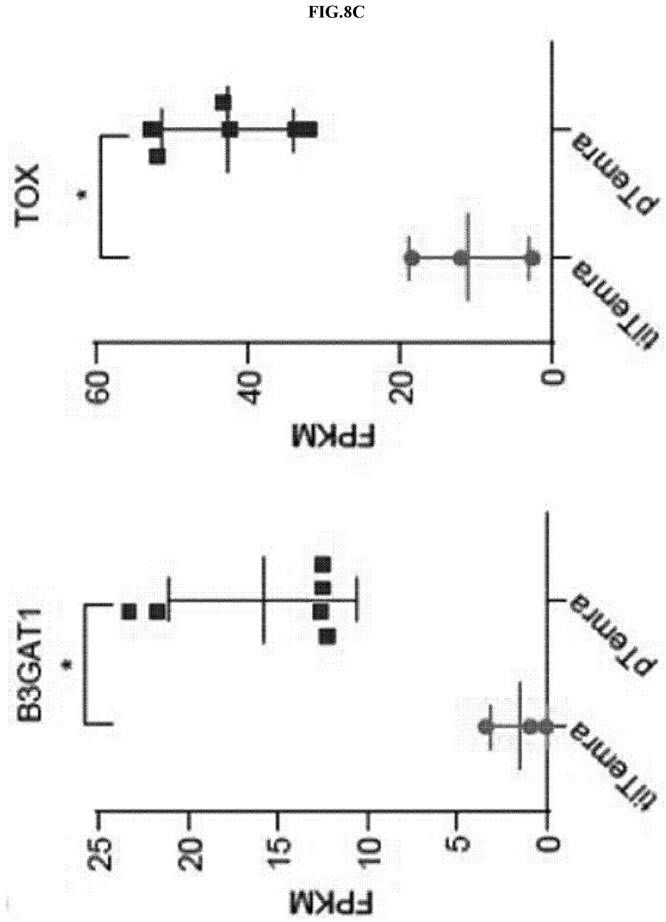

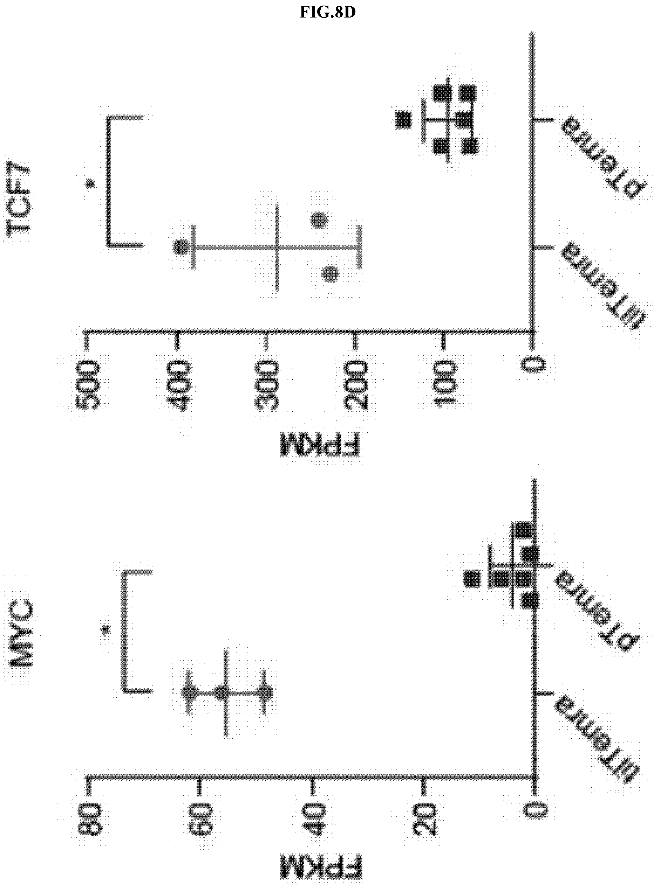

FIGS. 8A-8D show differentiation-related genes (TBX21, EOMES, PRF1, GZMB, GZMH) (FIG. 8A); co-stimulatory molecules (ICOS, CD28) (FIG. 8B); aging or exhaustion-related genes (B3GAT1, TOX) (FIG. 8C); and RNA seq data of proliferation or memory-related genes (MYC, TCF7) (FIG. 8D).

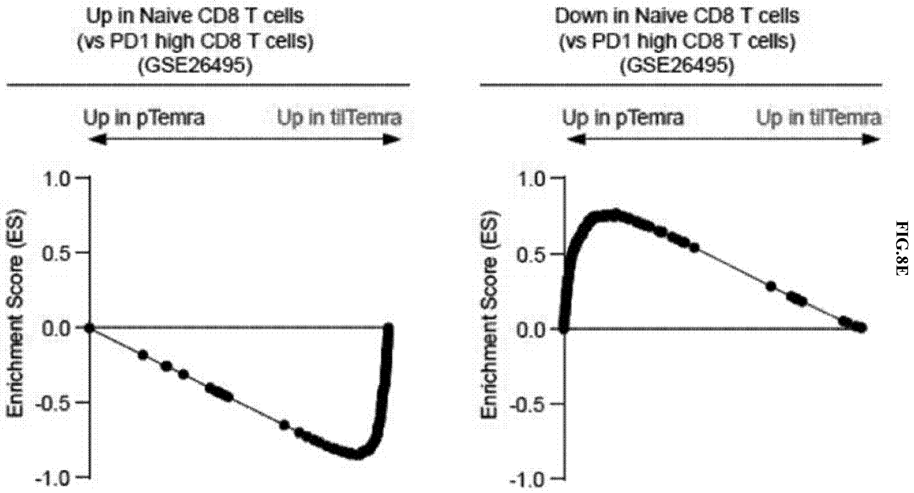

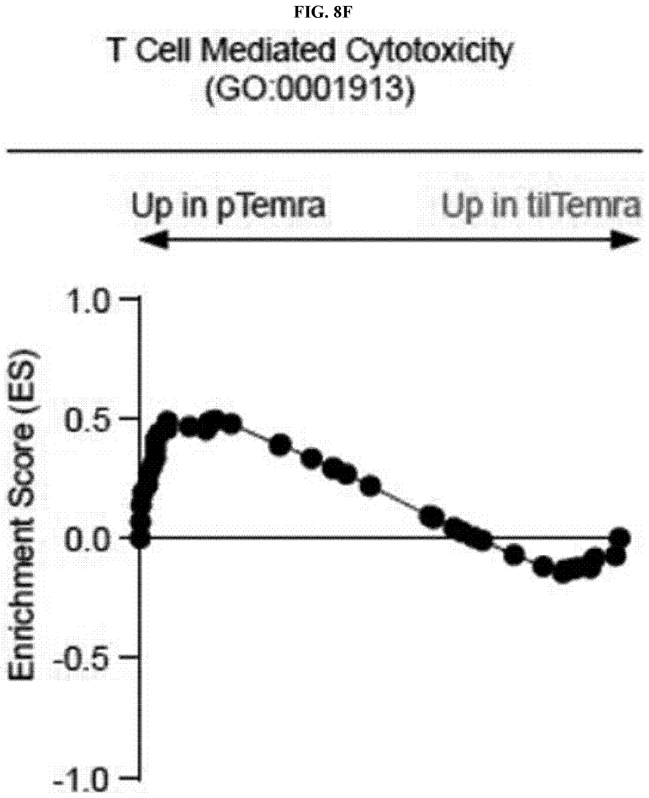

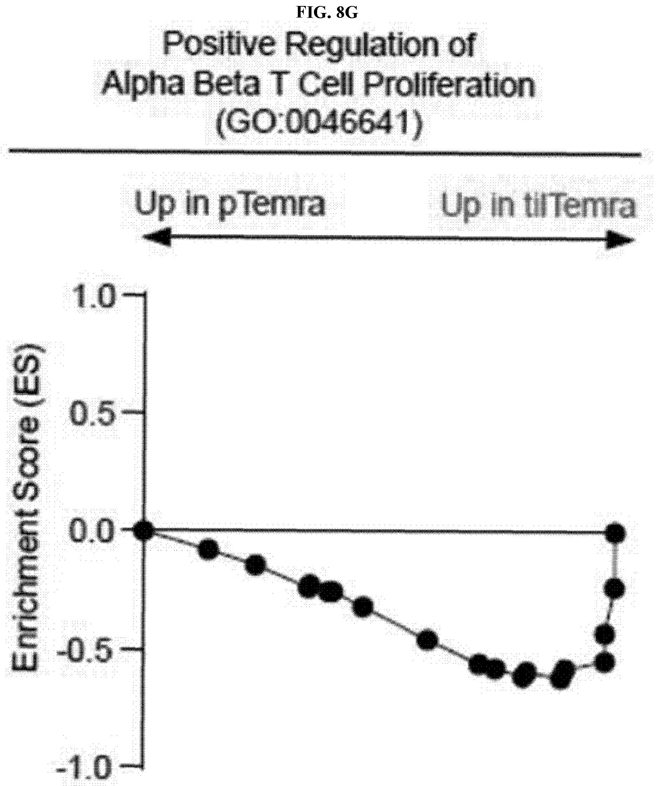

FIGS. 8E-8G show GSEA results of gene sets related to exhaustion (FIG. 8E); cytotoxicity (FIG. 8F); and proliferation (FIG. 8G). Statistic significance was confirmed by a Mann-Whitney test.

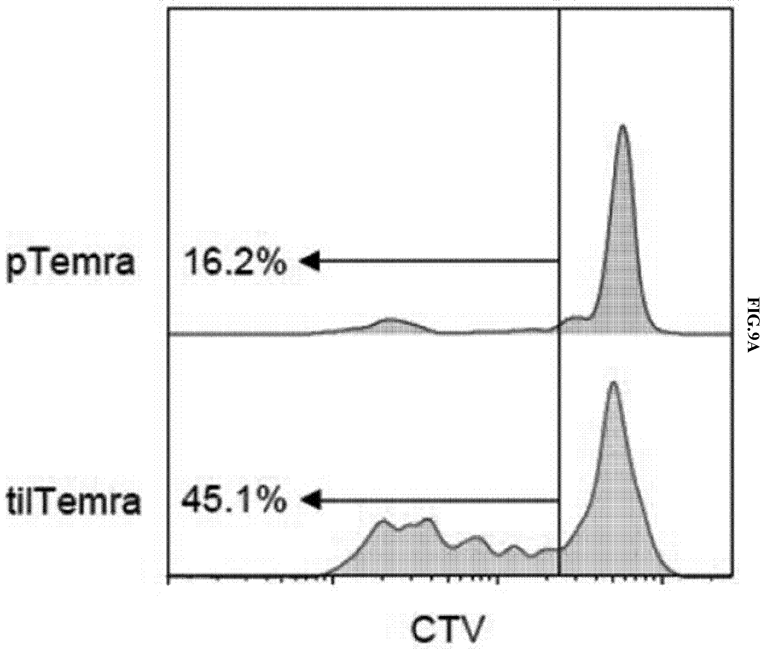

FIGS. 9A-9I are views showing the results of comparing the functional phenotypes of tilTem and pTem.

FIG. 9A is a histogram of CTV dilution of cells stimulated with anti-CD3 (5 $\mu$g/mL), anti-CD28 (2 $\mu$g/mL), and IL-2 (10 ng/mL) for 7 days.

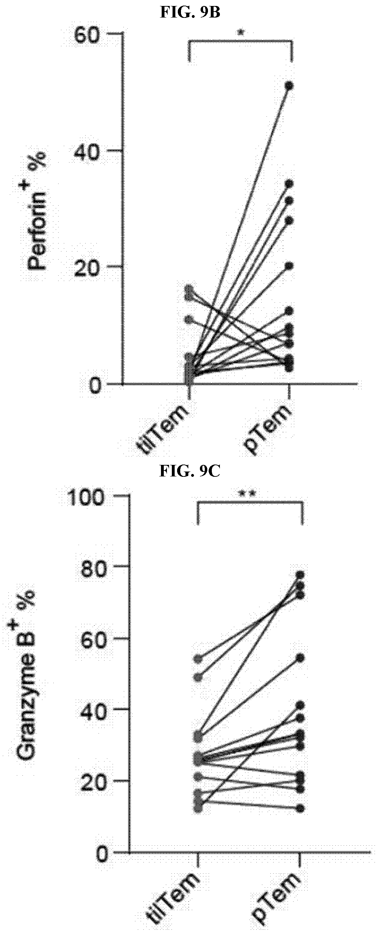

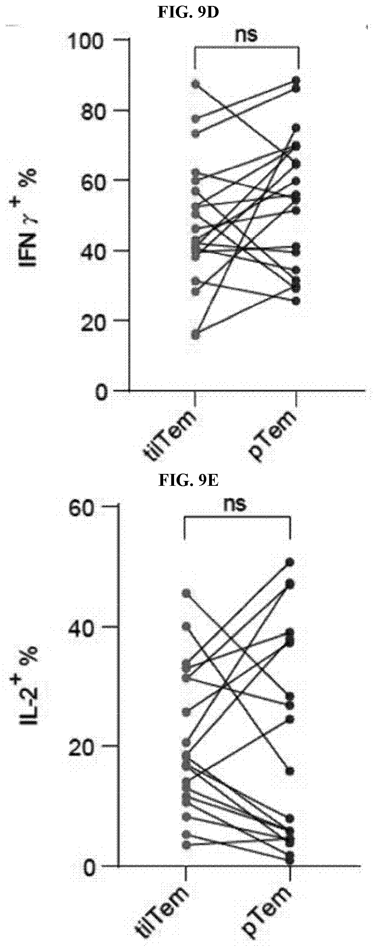

FIGS. 9B-9E show the results that assess the proportions of tilTem or pTem that produce perforin (FIG. 9B), granzyme B (FIG. 9C), IFN-$\gamma$ (FIG. 9D), and IL-2 (FIG. 9E) by flow cytometry.

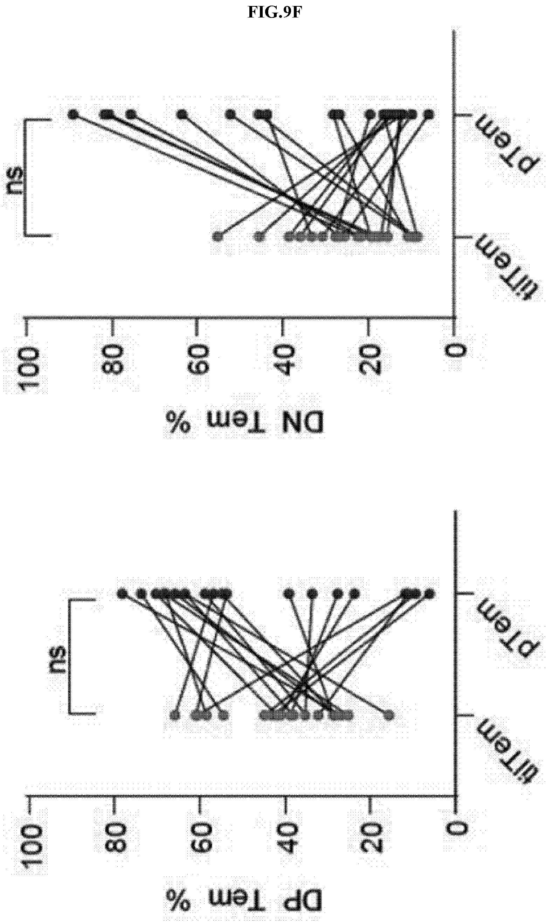

FIG. 9F is a view showing the proportions of DP (left) and DN (right) phenotypes of tilTem and pTem.

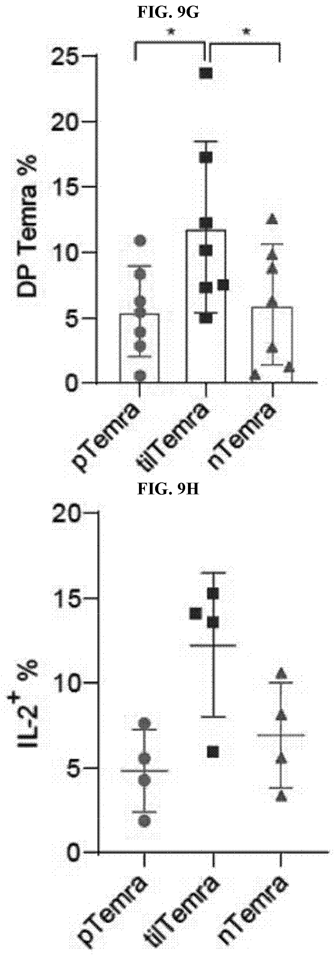

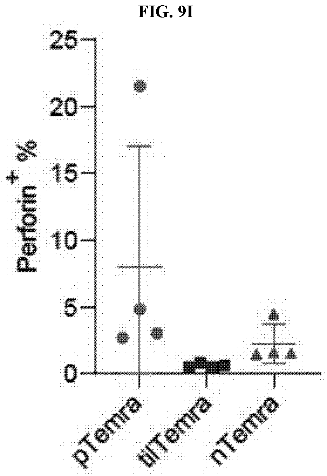

FIGS. 9G and 9H show results that assess DP proportion (FIG. 9G), IL-2 production (FIG. 9H), and perforin production (FIG. 9I) in PBMC-derived Temra cells (pTemra), TILs (tilTemra), and NATs (nTemra).

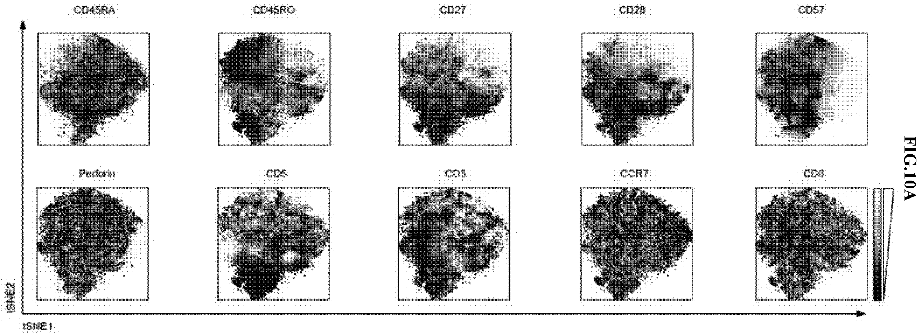

FIGS. 10A-10C are views showing the expression levels of key molecules of CD8$^+$ TILs in tSNE and FLOW MAP.

FIG. 10A shows the results of confirming the expression level of each molecule by tSNE analysis of CD8$^+$ TILs. The relative expression levels of indicated molecules were represented with a color scale from black (low) to yellow (high).

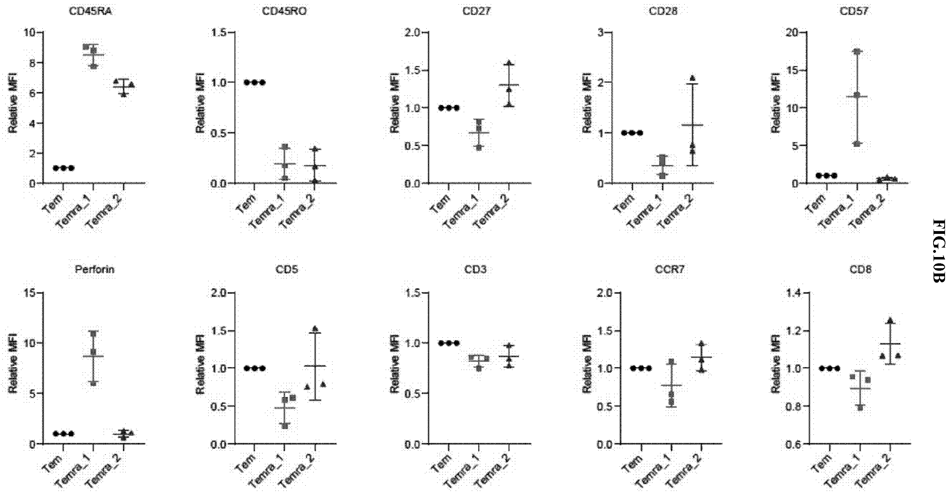

FIG. 10B shows the relative expression level of each molecule in tilTem and two types of clustering of tilTemra.

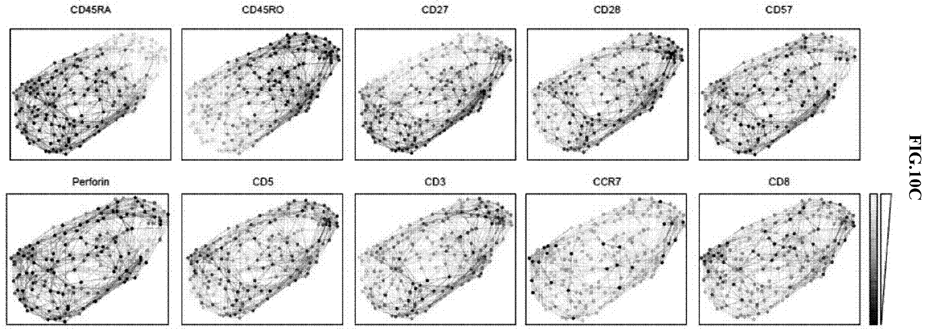

FIG. 10C shows the relative expression levels of molecules confirmed by FLOW MAP. The relative expression levels of the indicated molecules were represented with a color scale from black (low) to yellow (high).

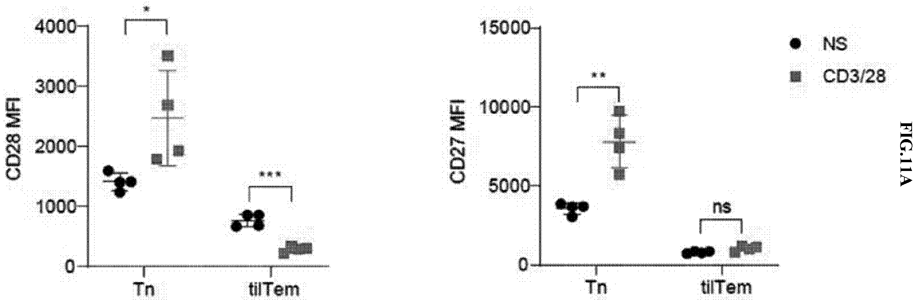

FIGS. 11A-11I are views showing the regulation of the expression of CD27, CD28, and CD45RA,

FIG. 11A shows the results of confirming CD28 and CD27 expression through flow cytometry after purifying Tn and tilTem and treating them with (no stimulation (NS), anti-CD3 (5 $\mu$g/mL) and anti-CD28 (2 $\mu$g/mL) stimulation for 7 days respectively.

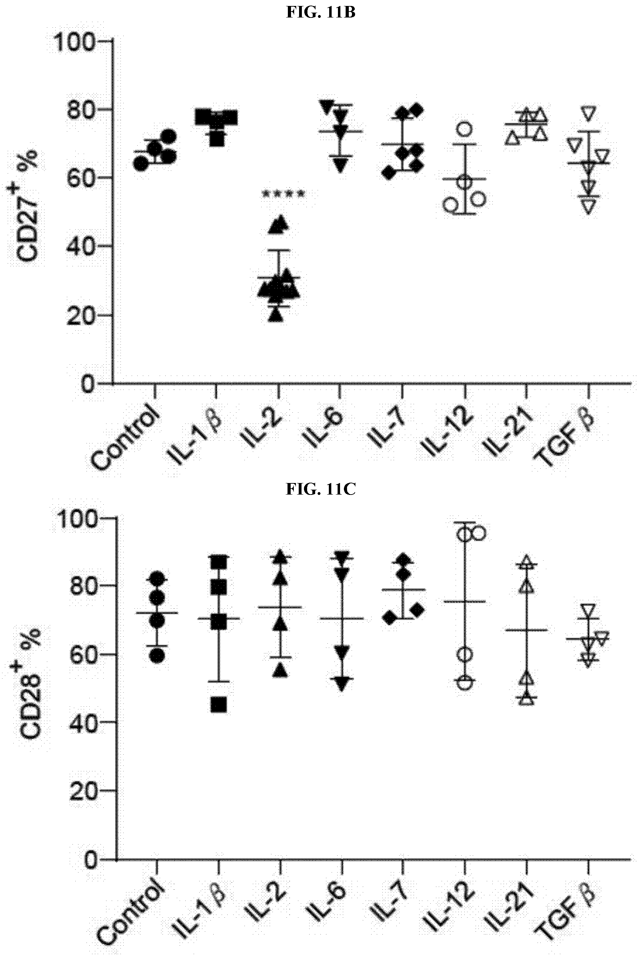

FIGS. 11B and 11C show the results of confirming CD27 (FIG. 11B) and CD28 (FIG. 11C) expression by flow cytometry after stimulating tilTem with the cytokines (20 ng/mL each) indicated on the drawings for 7 days.

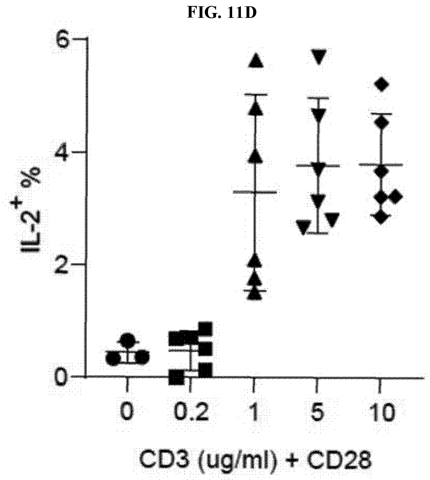

FIG. 11D shows the results of confirming IL-2 producing tilTem through flow cytometry after restimulating TILs with anti-CD3 and anti-CD28 for 5 hours. GolgiPlug was added after the first 1 hour.

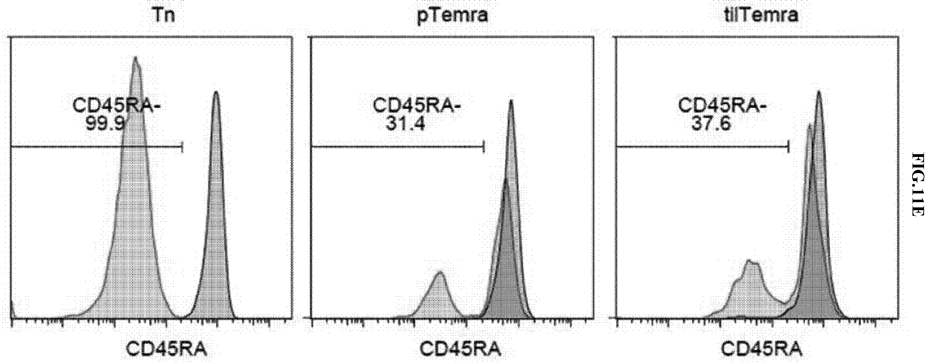

FIG. 11E shows the results of confirming CD45RA expression through flow cytometry after treating Tn, pTemra, and tilTemra with no stimulation (gray) or stimulating them with anti-CD3 (5 $\mu$g/mL) and anti-CD28 (2 $\mu$g/mL) for 7 days.

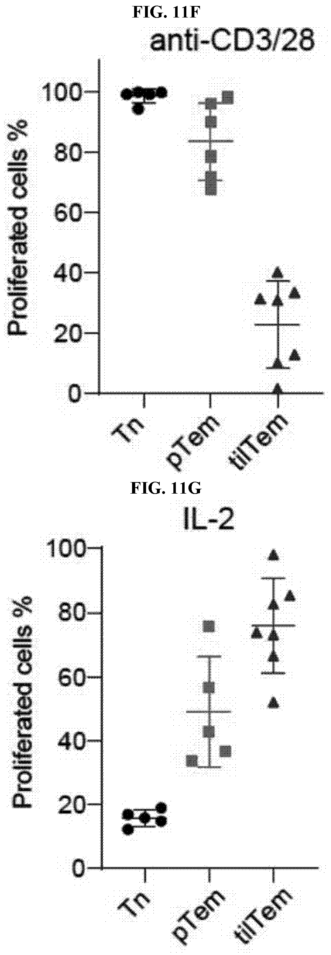

FIGS. 11F and 11G show the results of confirming cell proliferation through flow cytometry after labeling Tn, pTem, and tilTem with CTV, and stimulating them with anti-CD3 (5 $\mu$g/mL) and anti-CD28 (2 $\mu$g/mL) (FIG. 11F); or IL-2 (10 ng/mL) (FIG. 11G) for 7 days.

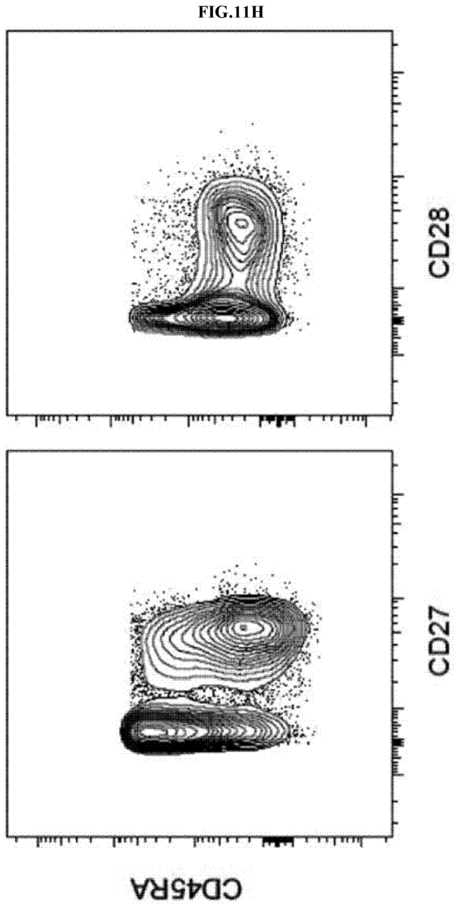

FIG. 11H is a representative diagram of flow cytometry showing the reexpression of CD45RA in pTem. CD45RA$^{lo}$CCR7$^-$ pTem was gated from PBMCs and CD45RA expression was confirmed with CD27 or CD28.

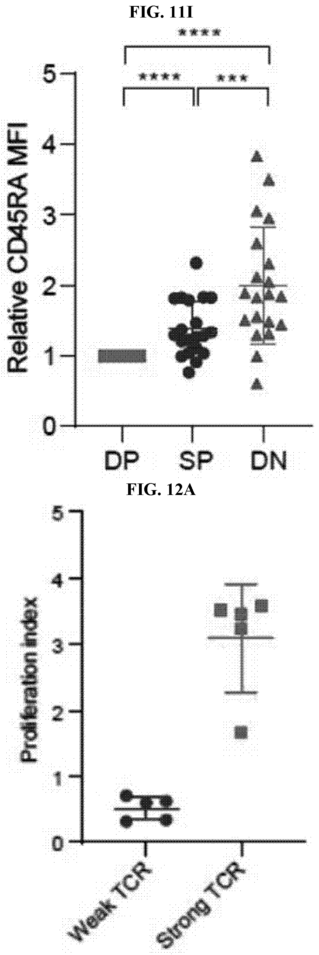

FIG. 11I shows the relative CD45RA expressions of DP, SP, and DN pTem. The relative CD45RA MFI was calculated by dividing the MFI value of DP cells derived from the same PBMCs.

FIGS. 12A-12I are views showing the correlation between DP pTemra proportion and tumor immunogenicity.

FIG. 12A shows the results of confirming proliferated cells through flow cytometry after stimulating CTV-labeled Tn with weak TCR (anti-CD3 (1 $\mu$g/mL) and anti-CD28 (0.4 $\mu$g/mL)) or with strong TCR (anti-CD3 (5 $\mu$g/mL) and anti-CD28 (2 $\mu$g/mL)) for 7 days.

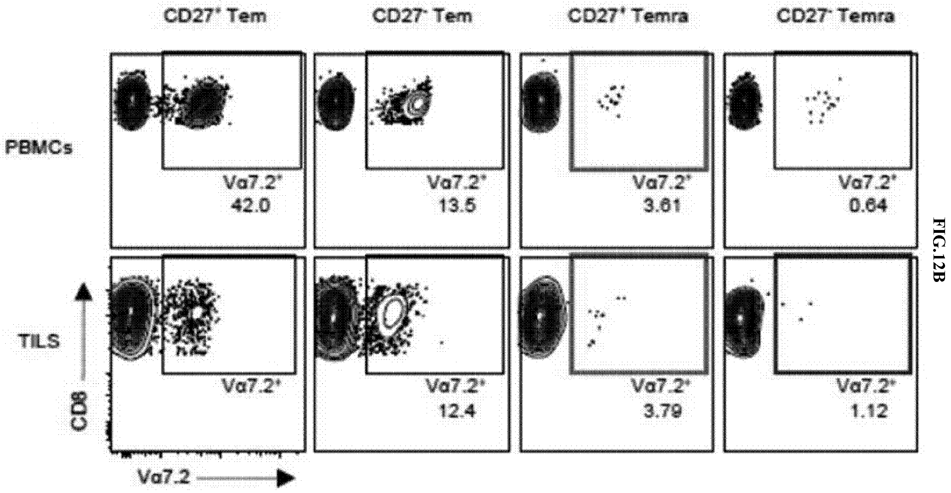

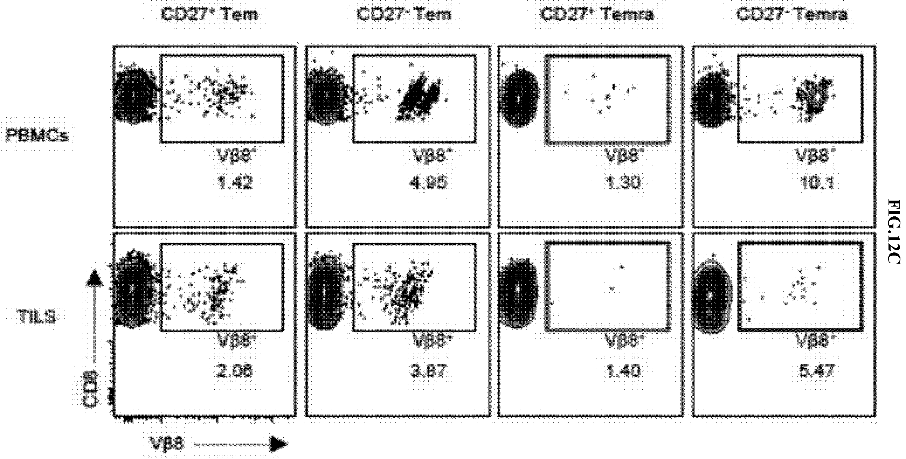

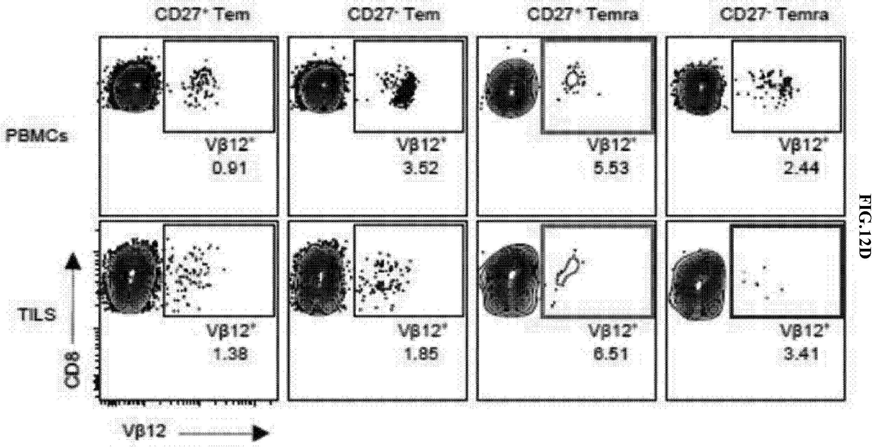

FIGS. 12B-12E show the results of analyzing the use of TCR $\alpha$/$\beta$ in peripheral or tumor-infiltrating CD27$^{+/-}$ Tem and CD27$^{+/-}$ Temra cells (FIG. 12B, the result confirmed using V$\alpha$7.2 use; FIG. 12C, the result confirmed using V$\beta$8 use; and FIG. 12D, the result confirmed using V$\beta$12 use).

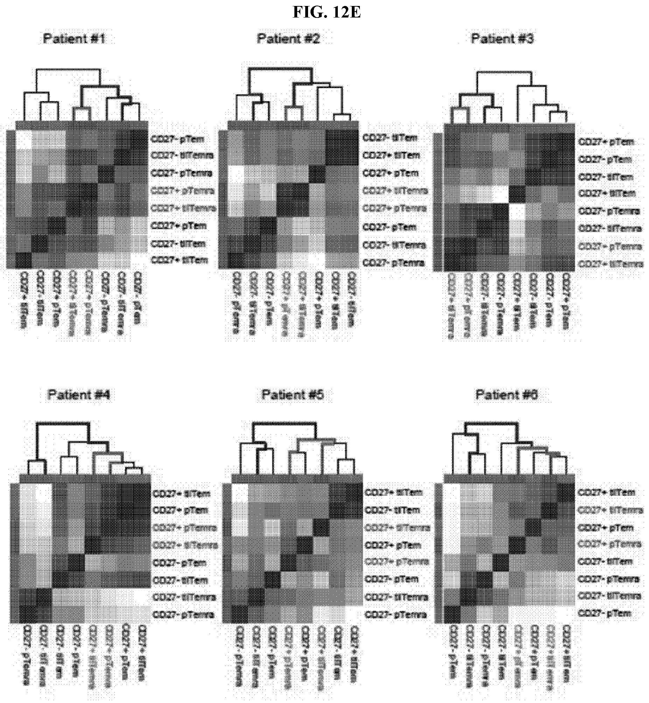

FIG. 12E shows the sample distance matrix generated using 8 kinds of TCR $\alpha$/$\beta$ uses (Va2, V$\alpha$7.2, V$\alpha$12.1, V$\beta$3, V$\beta$5b, V$\beta$8, V$\beta$12, and V$\beta$13.1). Each matrix includes T cell subsets derived from one individual. Blue represents close samples, and white represents distant samples. In the hierarchical clustering above each matrix, a red line indicates the distance between CD27$^+$ tilTemra and CD27$^+$ pTemra, and a blue line indicates the distance between CD27$^-$ tilTemra and CD27$^+$ tilTemra/CD27$^+$ pTemra.

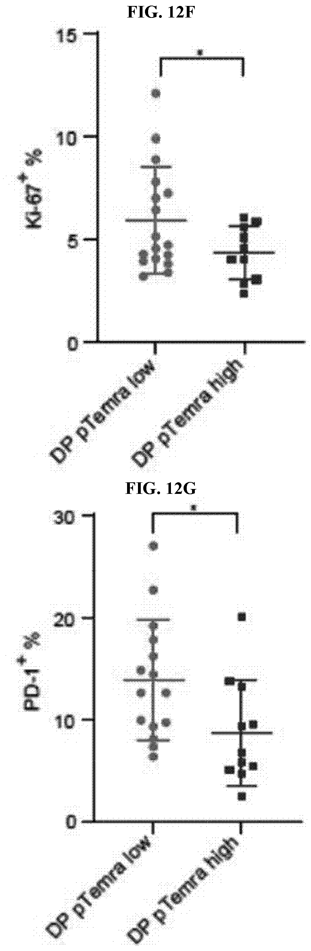

FIGS. 12F and 12G show the results of confirming Ki-67-expressing CD8$^+$ T cells (FIG. 12F) and PD-1-expressing CD8$^+$ T cells (FIG. 12G) in blood samples of NSCLC patients.

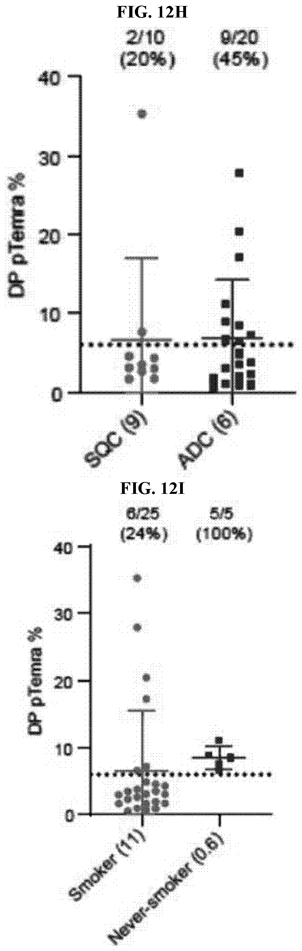

FIGS. 12H and 12I show the proportion of DP pTemra in SCC and non-SCC patients (FIG. 12H), and the proportion of DP pTemra in smoker and non-smoker patients (FIG. 12I). A dotted line represents a baseline when DP pTemra %=6%.

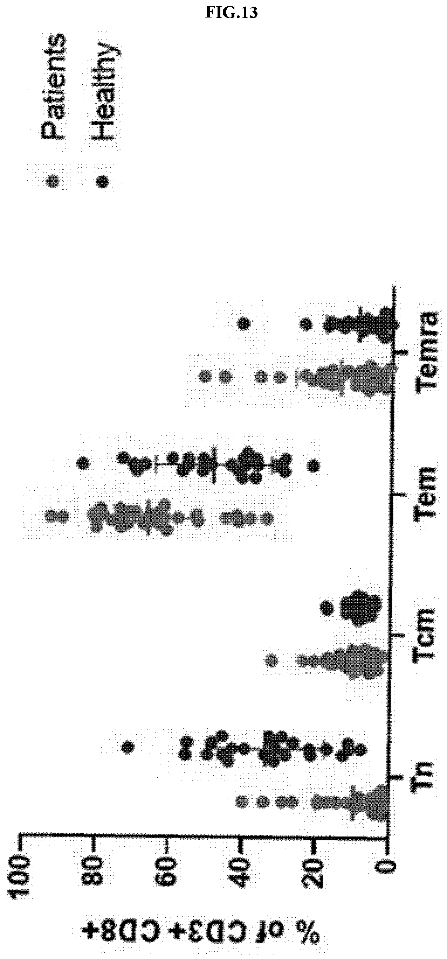

FIG. 13 shows the results of measuring subsets (Tn, Tcm, Tem, and Temra) in CD8 T cells of a healthy subject and an NSCLC cancer patient.

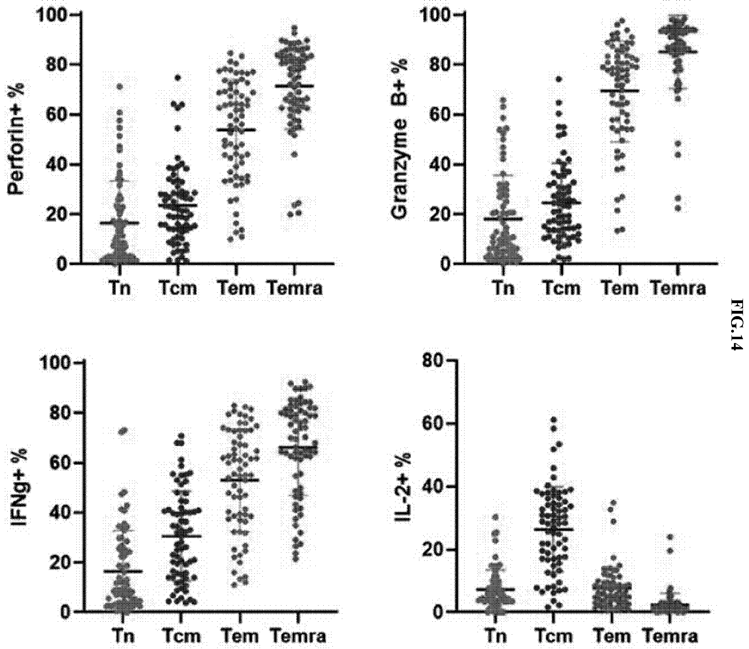

FIG. 14 shows the results of confirming the proportion of cells secreting perforin, granzyme B, IFN-$\gamma$, or IL-2 in each subset of CD8 T cells through flow cytometry after restimulating PBMCs of an NSCLC cancer patient with PMA/ionomycin.

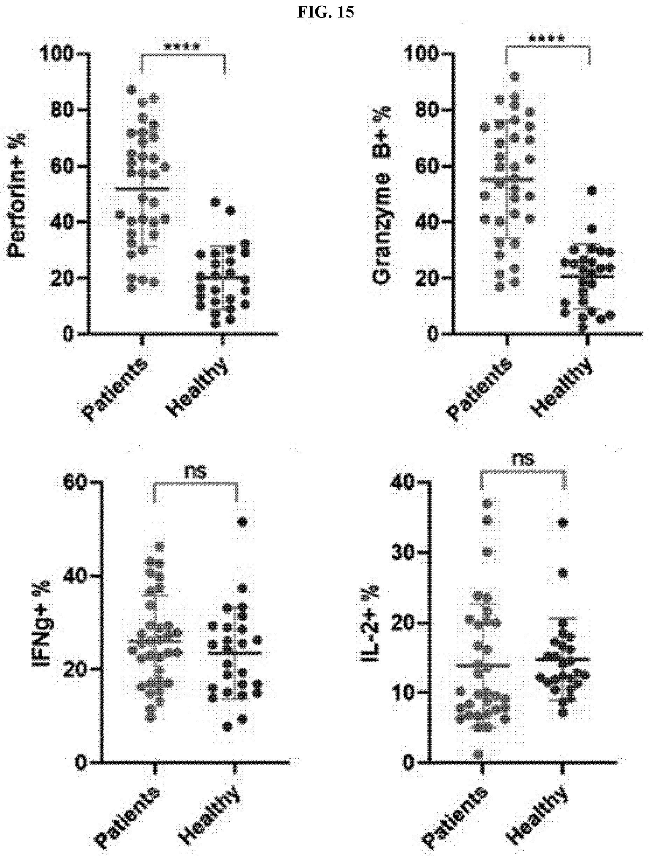

FIG. 15 shows the results of confirming the proportion of cells secreting perforin, granzyme B, IFN-$\gamma$, or IL-2 in Tem cells through flow cytometry after restimulating PBMCs of a healthy subject or a NSCLC cancer patient with PMA/ionomycin.

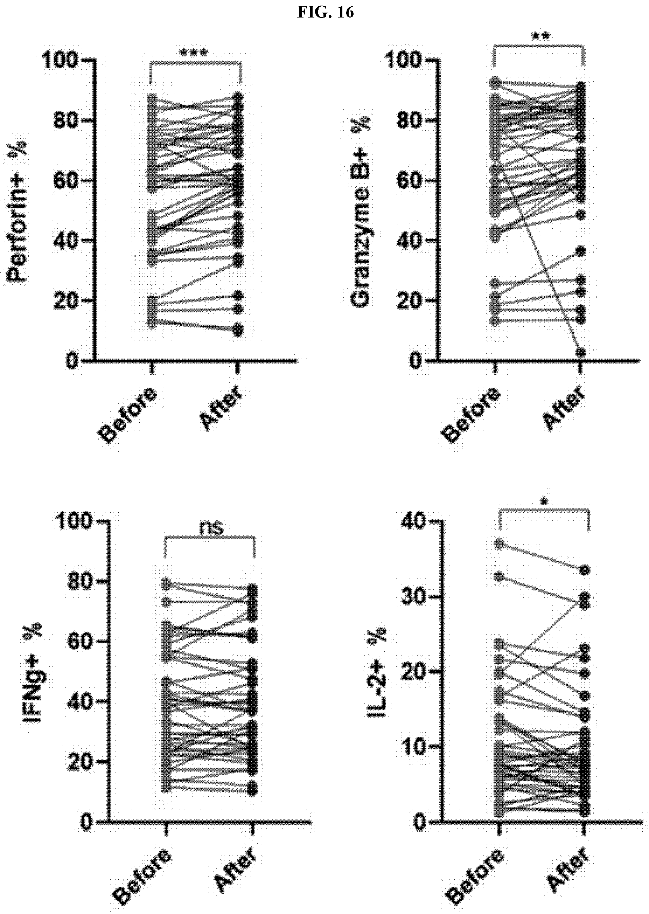

FIG. 16 shows the results of confirming the proportion of cells secreting perforin, granzyme B, IFN-γ, or IL-2 in Tem cells through flow cytometry after restimulating PBMCs of NSCLC cancer patients either before (Before) or after (After) cancer immunotherapy (PD-1 therapy or PD-L1 therapy, the same below) with PMA/ionomycin.

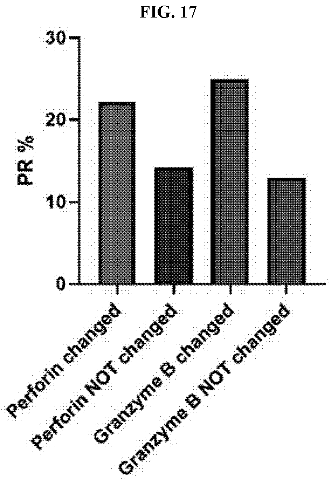

FIG. 17 shows the results of measuring the proportions of patients showing a partial response (PR) to cancer immunotherapy either in patient groups (Perform changed and Granzyme B changed) with increased perforin and granzyme B levels after cancer immunotherapy or unchanged patient groups (Perforin NOT changed and Granzyme B NOT changed).

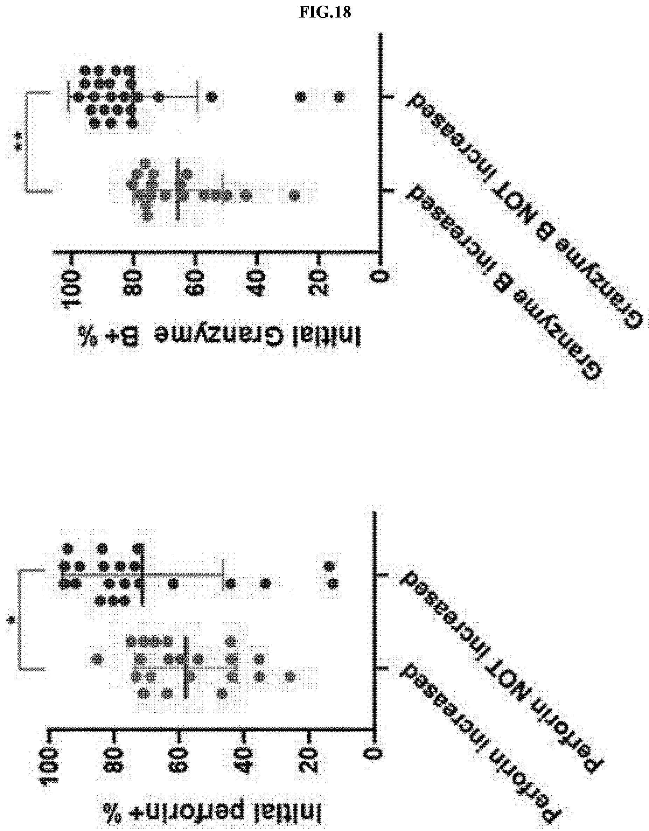

FIG. 18 shows the results of measuring a perforin level (Initial perform) and a granzyme B level (Initial Granzyme B) of blood before cancer immunotherapy either in patient groups with increased perforin and granzyme B levels (Perforin increased and Granzyme B increased) or unchanged patient groups (Perforin NOT increased and Granzyme B NOT increased) after cancer immunotherapy.

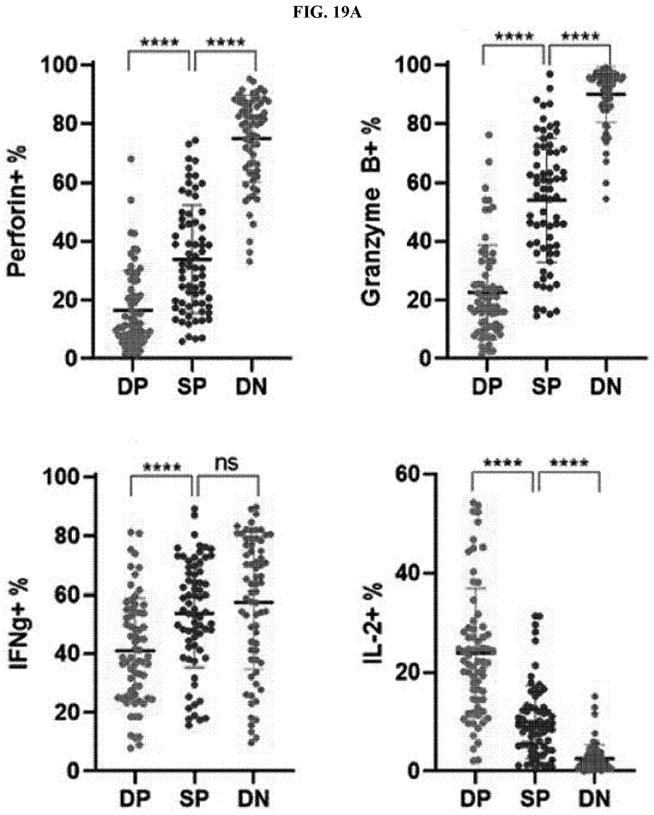

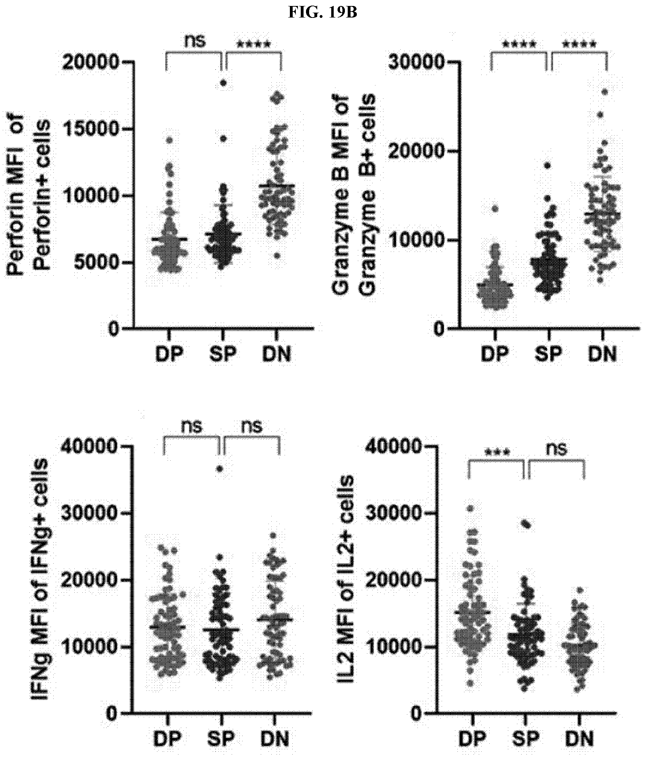

FIGS. 19A and 19B show the results of confirming the proportion of cells secreting perforin, granzyme B, IFN-γ, or IL-2 in each subset (DP, SP, or DN) of Tem cells of a cancer patient through flow cytometry (FIG. 19A), and the results of measuring the MFI of a molecule expressed in each type of cells (FIG. 19B).

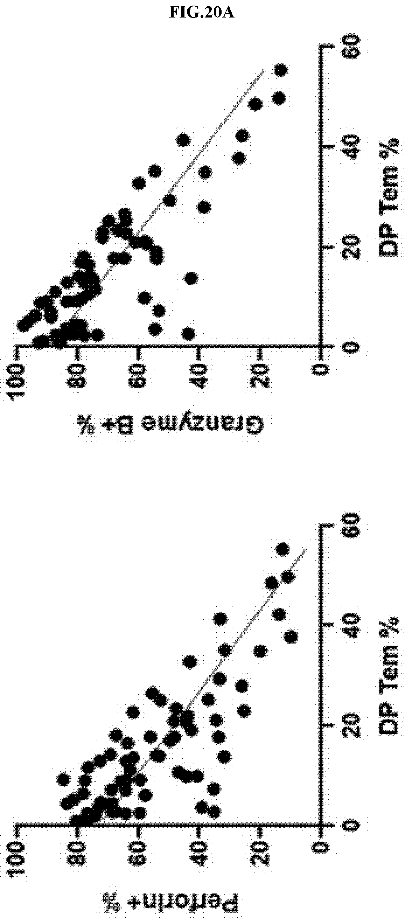

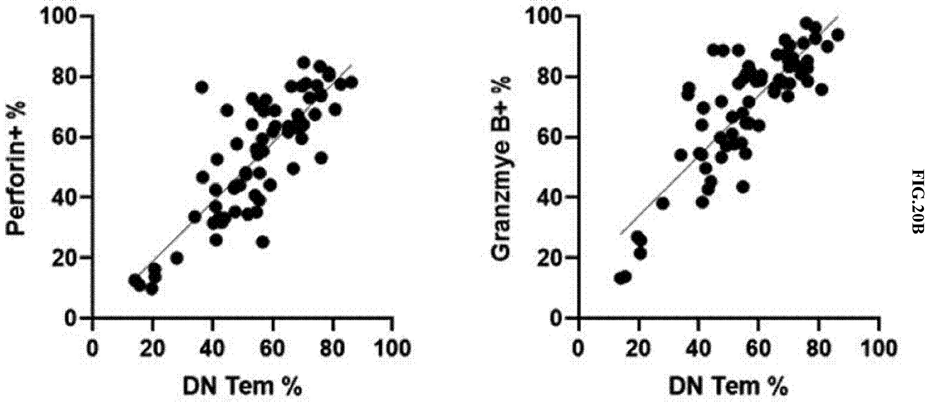

FIGS. 20A and 20B are graphs showing the correlation between the proportion of DP or DN subset in total Tem of a cancer patient and the expression levels of perforin and granzyme B. FIG. 20A is a graph showing the correlation between the proportion of DP Tem in total Tem; and perforin or granzyme B, and FIG. 20B is a graph showing the correlation between the proportion of DN Tem in total Tem; and perforin or granzyme B.

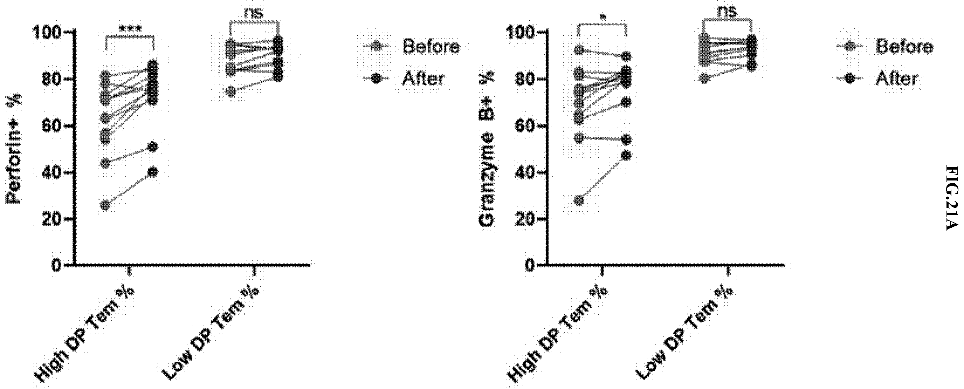

FIG. 21A shows the results of comparing the expression levels of perforin and granzyme B before and after cancer immunotherapy (PD-1 therapy) after dividing cancer patients into patients with a high DP Tem proportion (High DN Tem%) and patients with a low DP Tem proportion (Low DP Tem%) in total Tem.

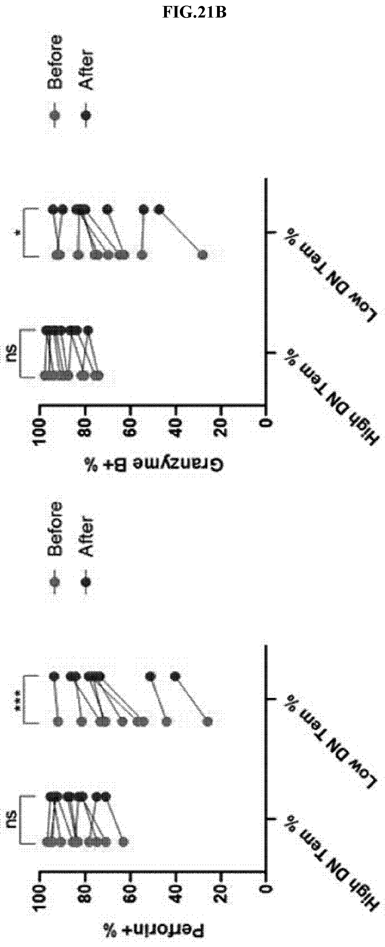

FIG. 21B shows the results of comparing the expression levels of perforin and granzyme B before and after cancer immunotherapy (PD-1 therapy) after dividing cancer patients into patients with a high DN Tem proportion (High DN Tem%) and patients with a low DN Tem proportion (Low DN Tem%) in total Tem.

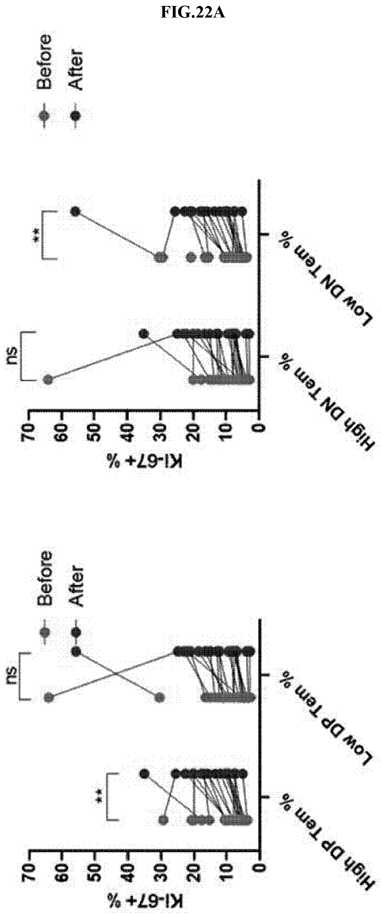

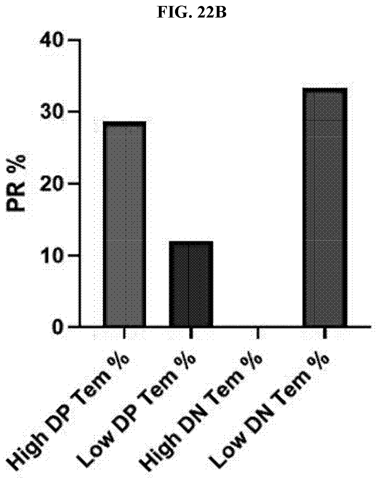

FIGS. 22A and 22B show the results of dividing cancer patients into four groups (High DP Tem%; Low DP Tem%; High DN Tem%; and Low DN Tem%) based on the proportions of DP Tem and DN Tem in total Tem, and comparing the proportions of patients showing Ki-67 expression level (FIG. 22A) before and after cancer immunotherapy (PD-1 therapy) and a partial response (PR) to cancer immunotherapy (FIG. 22B) in each group.

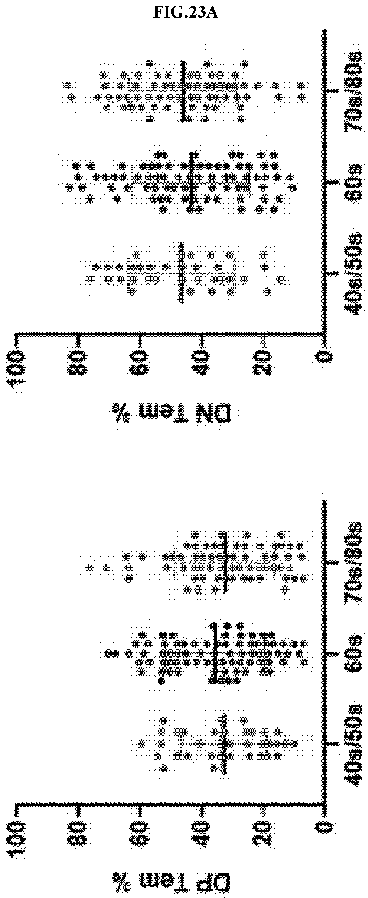

FIG. 23A shows the results of grouping patients with NSCLC stage 3 and stage 4 by age (40s/50s; 60s; and 70s/80s) and confirming the proportions of DP Tem and DN Tem in total Tem for each group.

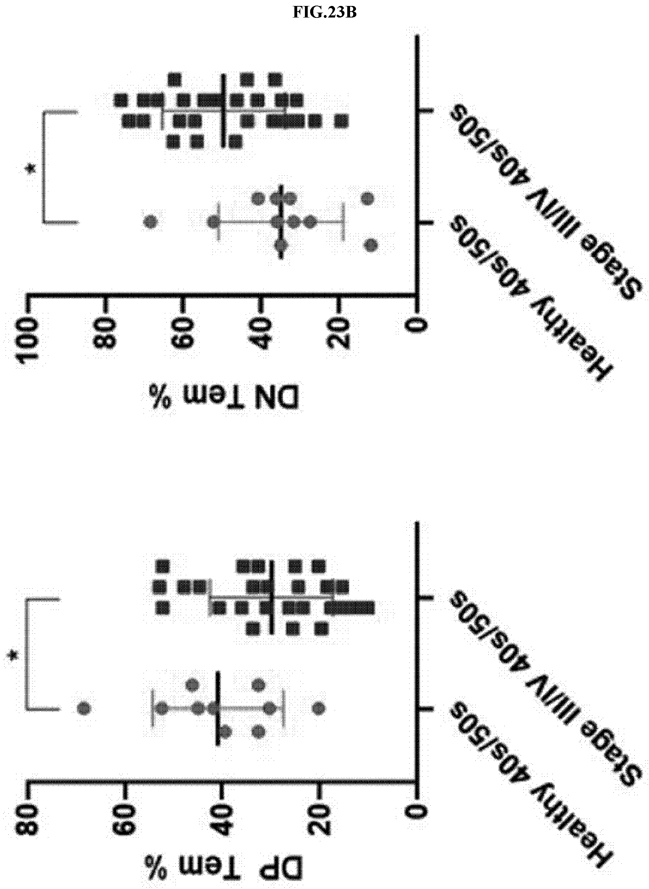

FIG. 23B shows the results of comparing the proportions of DP Tem and DN Tem in total Tem between a healthy group in their 40s/50s and an NSCLC stage III/IV patient group.

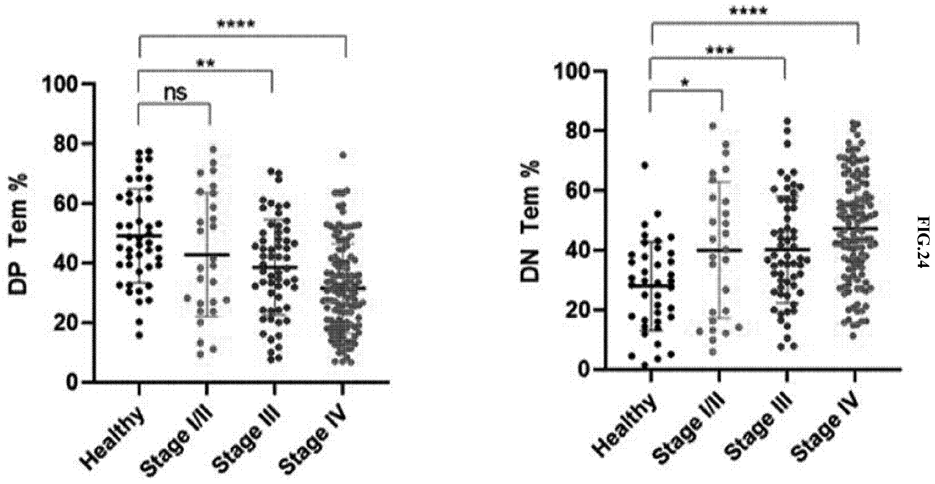

FIG. 24 shows the results of confirming the proportions of DP Tem and DN Tem in total Tem for each of healthy and NSCLC patient (Stage I/II, Stage III, and Stage IV) groups to confirm DP Tem and DN Tem distribution patterns according to the degree of tumor progression.

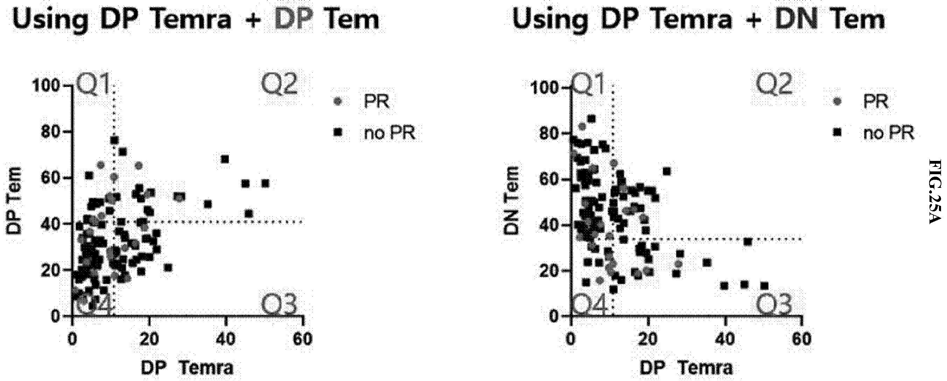

FIG. 25A shows a 2D graph of NSCLC patients, in which the x axis is set as DP Temra, and the y axis is set as DP Tem (left graph) or DN Tem (right graph), plotting patients (PR) showing responsiveness to cancer immunotherapy (P1 treatment) and patients having no responsiveness (no PR), and dividing into Q1 to Q4 groups based on DP Temra and DP/DN Tem levels.

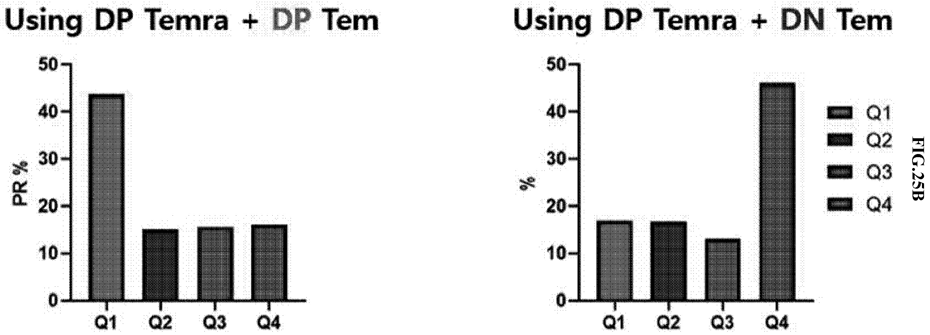

FIG. 25B shows the results of comparing the proportions of patients showing a partial response to cancer immunotherapy between Q1 to Q4 groups divided based on a dual marker of DP Temra; and DP Tem (left graph) or DN Tem (right graph).

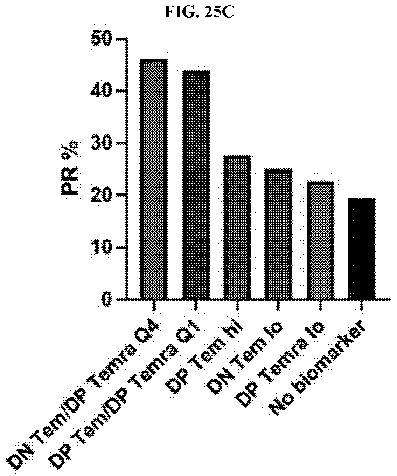

FIG. 25C shows the results of confirming the proportions of PR-patient for each marker to compare the capability to predict immunotherapeutic responsiveness of the dual marker of DP Temra and DP/DN Tem with the capability to predict a single marker.

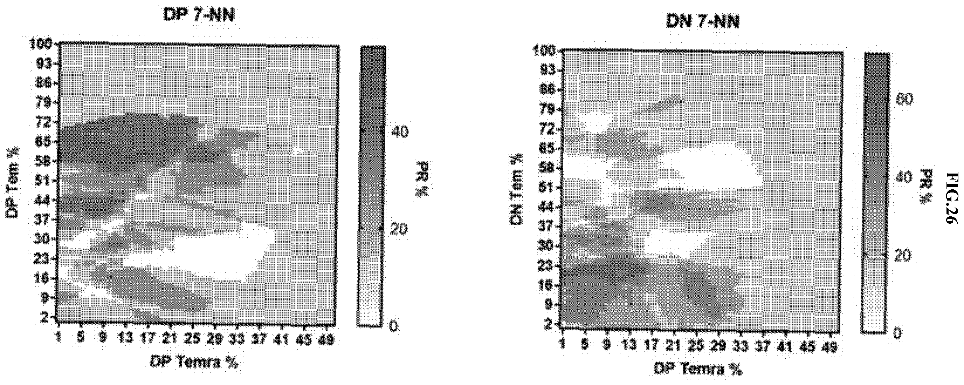

FIG. 26 shows pGRAPH showing PR frequency for each plot using K-nearest neighbor (K-NN) machine leaning (K=7) on DP Temra/DP Tem graph (left) and DP Temra/DN Tem graph (right). A higher PR frequency is represented red.

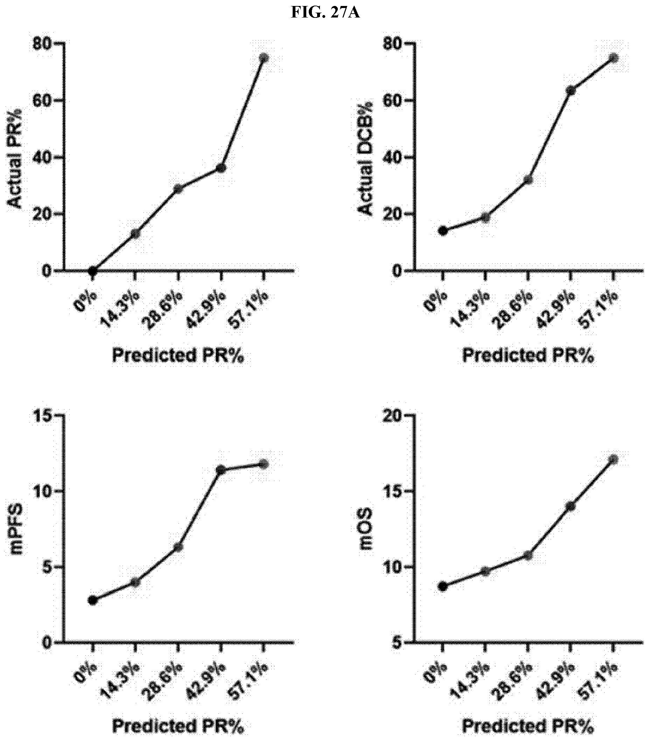

FIG. 27A shows the results of confirming whether predicted PR% is consistent with actual prognostic factors (actual PR%, actual DCB%, mPFS, and mOS) after obtaining predicted PR% values by applying DP Temra and DN Tem levels of 120 NSCLC patients to pGRAPH.

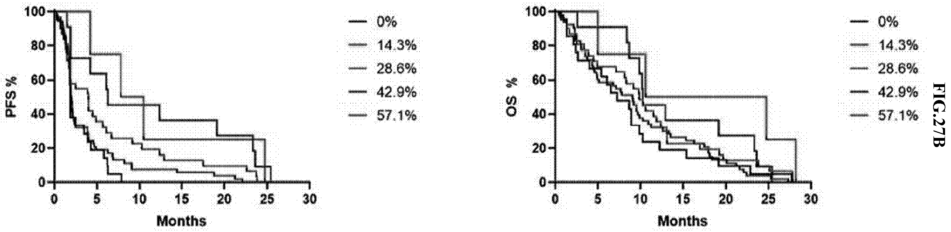

FIG. 27B is a graph confirming the progression-free survival period and overall survival period according to the predicted PR% of FIG. 27A.

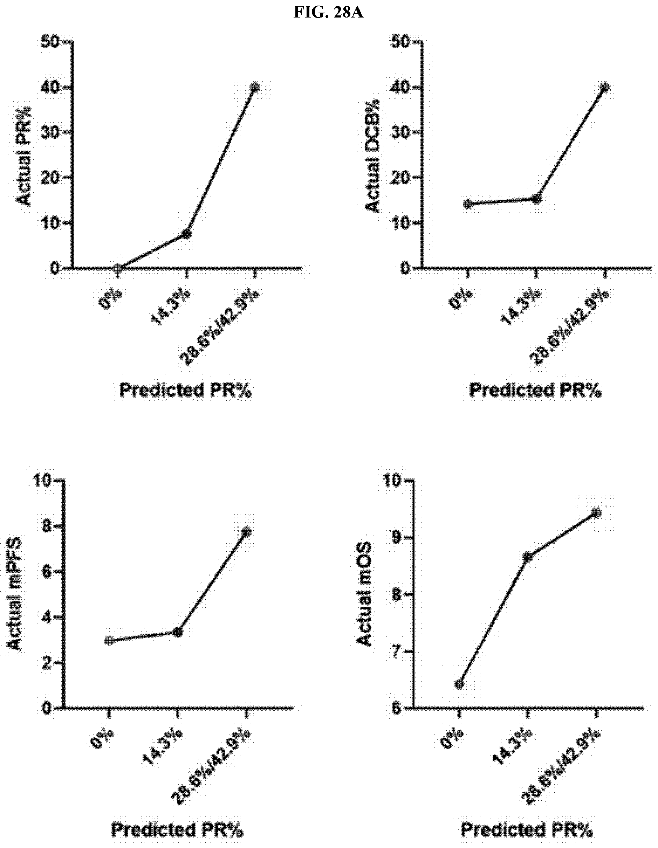

FIG. 28A shows the results of confirming whether predicted PR% is consistent with actual prognostic factors (actual PR%, actual DCB%, mPFS, and mOS) after obtaining predicted PR% of 30 other patients using pGRAPH constructed based on data of 72 cancer patients.

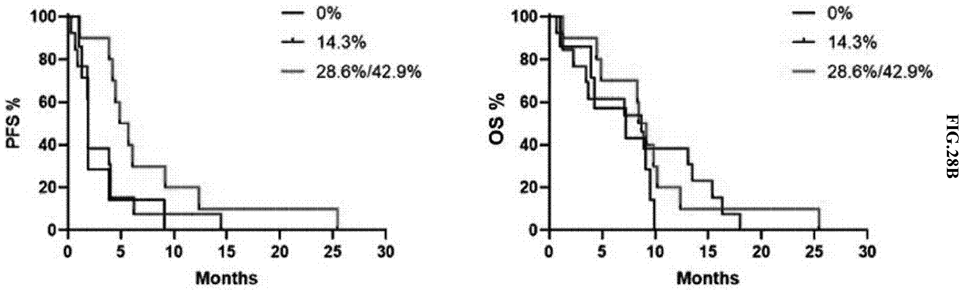

FIG. 28B is a graph confirming the progression-free survival period (left) and overall survival period (right) according to predicted PR% of FIG. 28A.

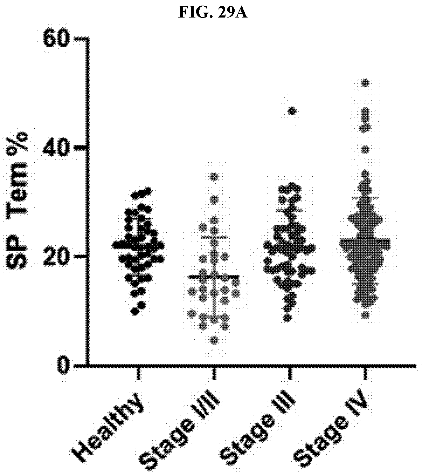

FIG. 29A is a graph confirming the proportion of SP Tem in total Tem for each of healthy and NSCLC patient (Stage I/II, Stage III, and Stage IV) groups.

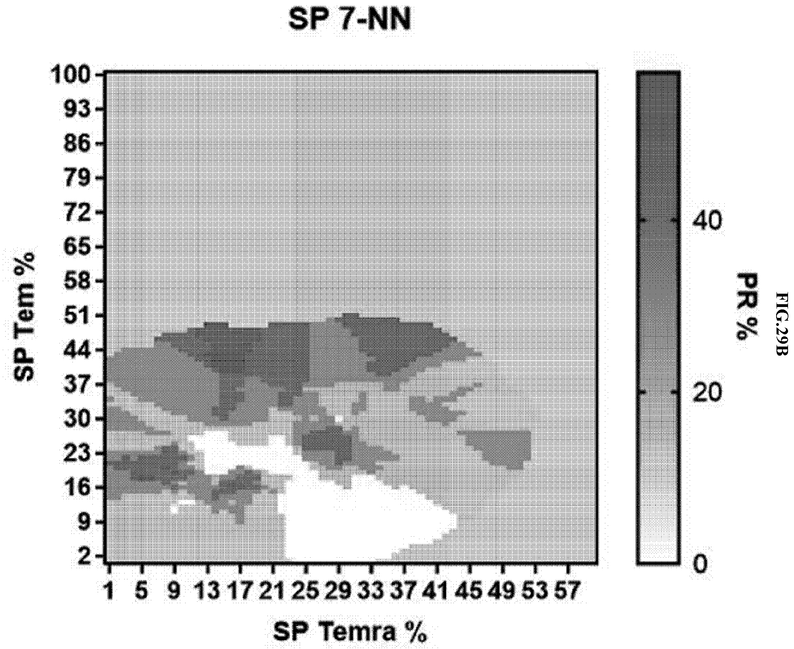

FIG. 29B is pGRAPH constructed based on SP Temra and SP Tem data of NSCLC patients.

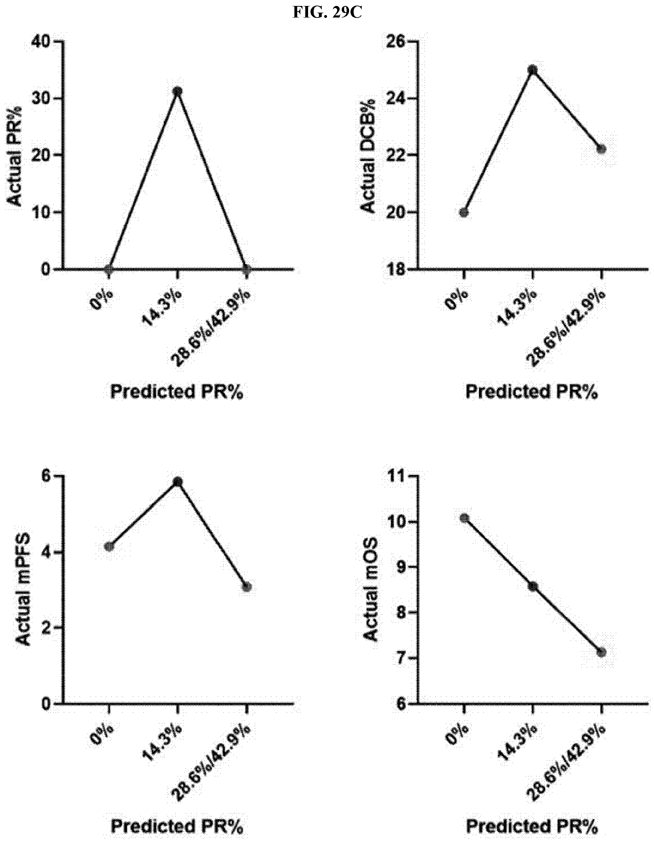

FIG. 29C shows the results of confirming whether predicted PR% is consistent with actual prognostic factors (actual PR%, actual DCB%, actual mPFS, and actual mOS) after obtaining predicted PR% of 30 other patients using pGRAPH constructed based on SP Temra and SP Tem data of 72 cancer patients.

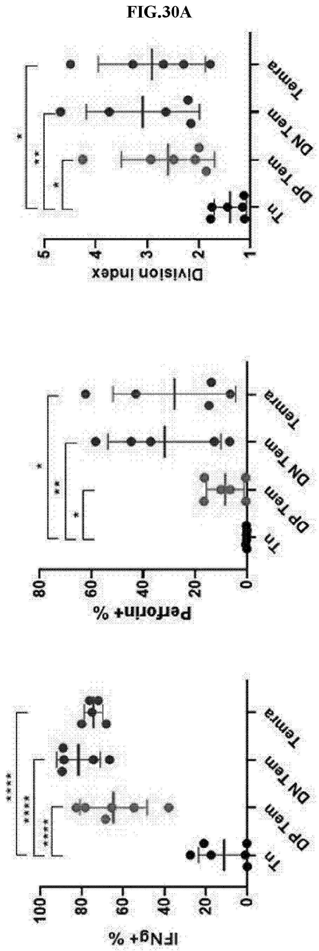

FIG. 30A shows the results of measuring the proportion of IFN-$\gamma$-expressing cells (left), the proportion of perforin-expressing cells (middle), and a division index based on CTV dilution (right) after stimulating Tn, DP Tem, DN Tem, and Temra cells sorted from PBMCs of healthy subjects with IL-2 for 7 days.

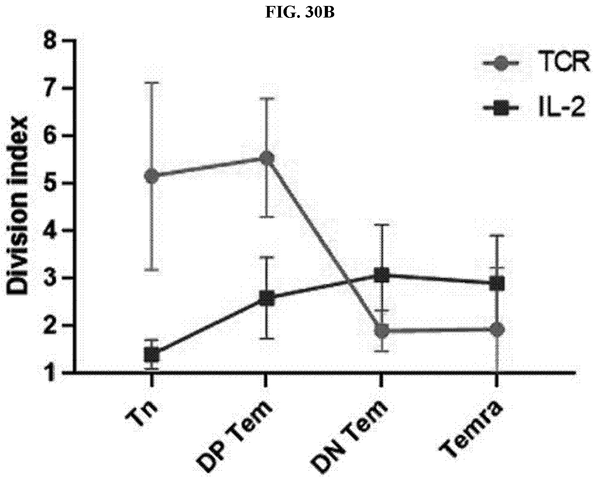

FIG. 30B shows the results of comparing degrees of cell division after stimulating Tn, DP Tem, DN Tem, and Temra cells sorted from PBMCs of healthy subjects with IL-2 or anti-CD3/CD28 for 7 days.

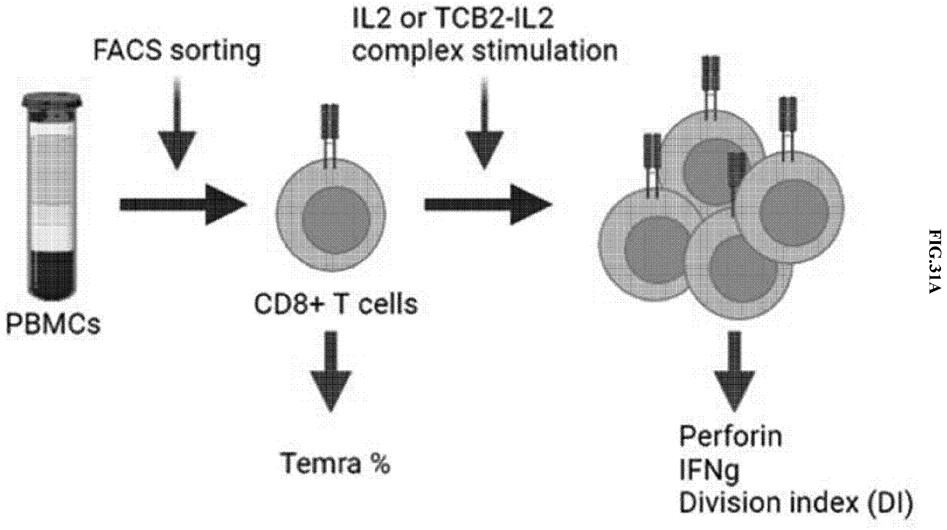

FIG. 31A shows an experimental flowchart to confirm the association between the initial Temra proportion and responsiveness to IL-2 treatment (IL-2 or TCB2-IL-2).

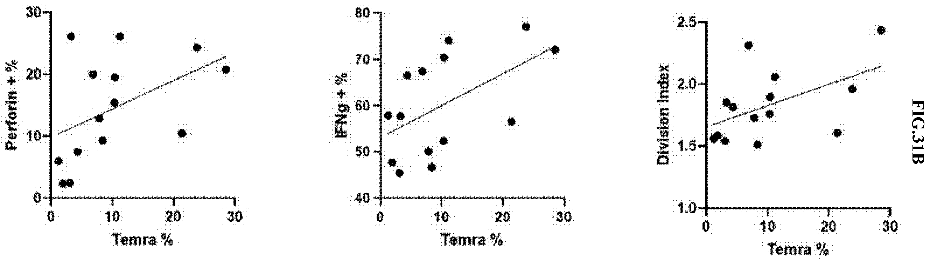

FIG. 31B shows the results of confirming a perforin level, an IFN-$\gamma$ level, and a division index after IL-2 stimulation according to the initial Temra proportion of healthy subjects.

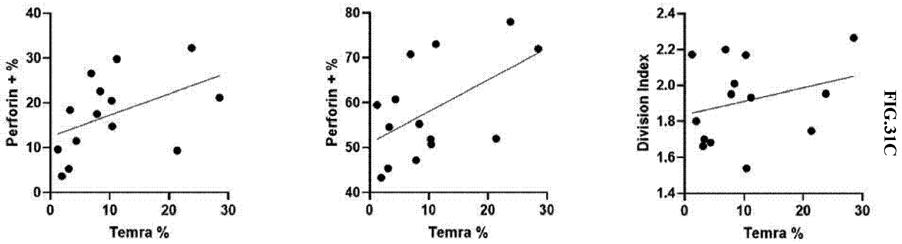

FIG. 31C shows the results of confirming a perforin level, an IFN-$\gamma$ level, and a division index after the stimulation with a TCB2-IL2 complex according to the initial Temra proportion of healthy subjects.

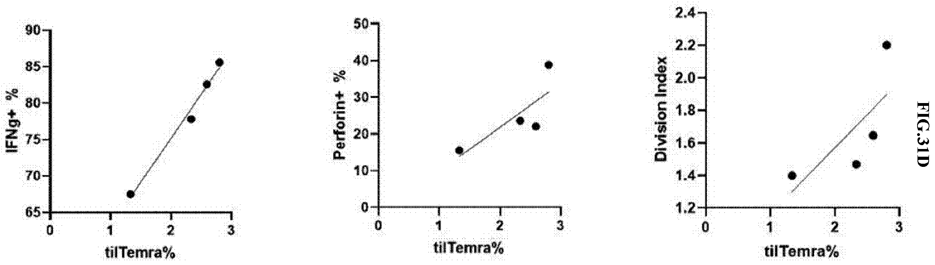

FIG. 31D shows the results of confirming an IFN-$\gamma$ level, a perforin level, and a division index after IL-2 stimulation according to the proportion of tilCD8$^+$ (tilTemra) cells.

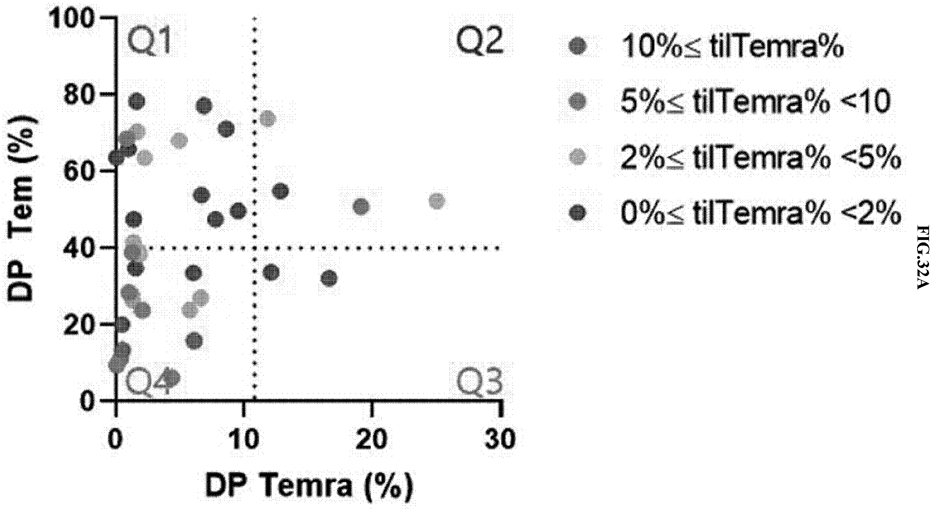

FIG. 32A is a view showing tilTemra % of each patient after plotting a 2D graph with DP Temra % and DP Tem % data of cancer patients and dividing the patients into Q1 to Q4 groups.

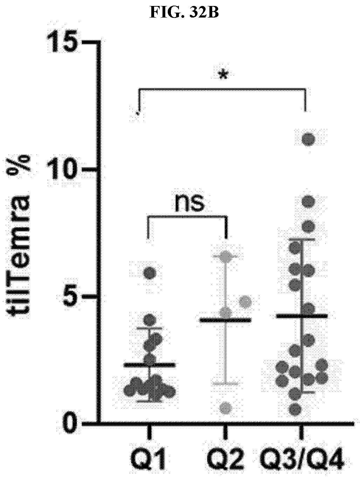

FIG. 32B shows the results of comparing tilTemra % of Q1, Q2, and Q3/Q4 groups of FIG. 32A.

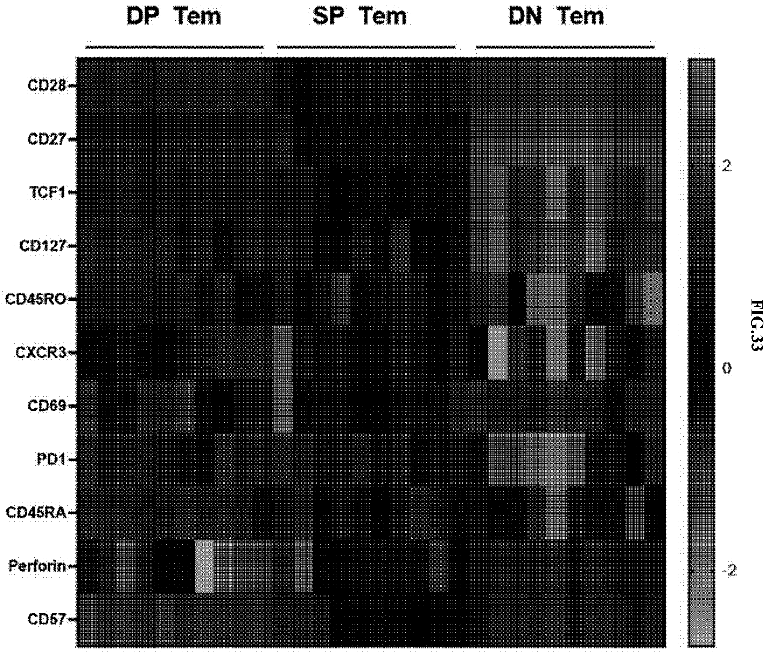

FIG. 33 is a heatmap comparing the expression levels of markers of DP Tem, SP Tem, and DN Tem to discover molecular markers capable of indirectly defining DP Tem and DN Tem.

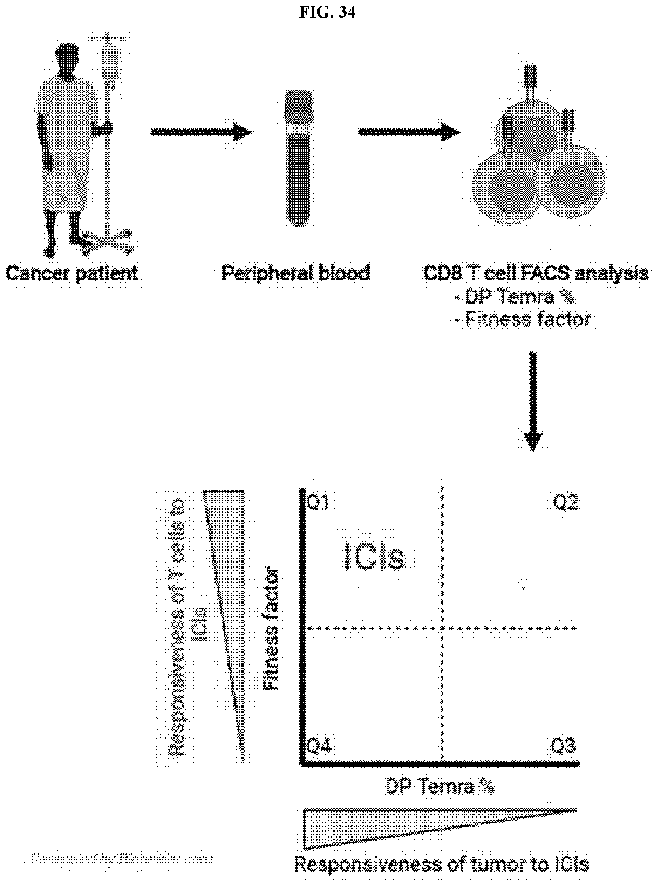

FIG. 34 is a diagram illustrating the overall principle of a method of predicting the prognosis or responsiveness to anticancer treatment of a cancer patient according to the present invention.

[Modes of the Invention]

The present invention relates to a method of predicting the prognosis of a cancer patient and/or responsiveness to anticancer treatment, and it is completed by confirming that the proportion of a specific CD8$^+$ T cell subset is associated with the prognosis of a cancer patient and responsiveness to anticancer treatment.

Specifically, in one embodiment of the present invention, it was confirmed that DP pTemra (CD27$^+$ CD28$^+$ CCR7$^-$ CD45RA$^+$CD8$^+$ T cells) has an inverse correlation with tumor mutation burden (TMB) and the number of tumor-infiltrating CD8 T cells, and thus can be used as a marker reflecting the immunogenicity (or antigenicity) of a patient and a marker for predicting a patient's responsiveness to cancer immunotherapy (Example I).

In another embodiment of the present invention, by discovering Fitness factor markers that can reflect the capability of CD8$^+$ cytotoxic T lymphocytes (CTLs) to induce and perform an anticancer immune response in a cancer patient (i.e., immunity: capability to respond to cancer immunotherapy), it was confirmed that as DP Tem% increased; DN Tem% decreased; perforin$^+$ Tem% increased after cancer immunotherapy; granzyme B$^+$ Tem% increased after cancer immunotherapy; and IL-2$^+$ Tem% decreased after cancer immunotherapy, the toxic T cell immunity of a cancer patient is high, so the markers are advantageous for anticancer treatment, and therefore, it was confirmed that the Fitness factors can be utilized as independent biomarkers for predicting the responsiveness of a patient to cancer immunotherapy (Example II).

In still another embodiment of the present invention, it was confirmed that the combination of DP Temra indicating cancer immunogenicity (and antigenicity) and Fitness factors indicating the toxic T cell immunity state of a patient exhibits a better prognosis predicting ability and a better ability to predict the responsiveness of a cancer patient to a cancer immunotherapeutic agent even when each marker is independently used, and through K-nearest neighbor (K-NN) algorithm-based machine learning based on DP Temra and Fitness factor data, prognosis prediction capability of a cancer patient and a prediction system (pGRAPH) for responsiveness to anticancer treatment were established (Example III).

Hereinafter, the present invention will be described in detail.

The present invention provides a method of predicting the prognosis or responsiveness to anticancer treatment of a cancer patient (or a method of providing information therefor), which includes measuring the proportion of a CD8$^+$ T cell subset in a biological sample isolated from a subject, in which the proportion of the CD8$^+$ T cell subset is one or more selected from the group consisting of the following:

(i) the proportion of CD27+ CD28+ CCR7- CD45RA+ CD8+ T cells (double positive (DP) Temra) among total CCR7- CD45RA+ CD8+ T cells (Temra);
(ii) the proportion of CCR7- CD45RA+ CD8+ T cells (Temra) among total CD8+ T cells;
(iii) the proportion of CD27+ CD28+ CCR7- CD45RA- CD8+ T cells (DP Tem) among total CCR7- CD45RA- CD8+ T cells (Tem);
(iv) the proportion of CD27- CD28- CCR7- CD45RA- CD8+ T cells (DN (double negative) Tem) among total CCR7- CD45RA- CD8+ T cells (Tem);
(v) the proportion of perforin+ CCR7- CD45RA- CD8+ T cells (perforin-expressing Tem) among total CCR7- CD45RA- CD8+ T cells (Tem);
(vi) the proportion of granzyme B+ CCR7- CD45RA- CD8+ T cells (granzyme B-expressing Tem) among total CCR7- CD45RA- CD8+ T cells (Tem); and
(vii) the proportion of IL-2+ CCR7- CD45RA- CD8+ T cells (IL-2-expressing Tem) among total CCR7- CD45RA- CD8+ T cells (Tem).

The DP and DN are based on the expression levels of CD27 and CD28. CD27 and CD28 are co-stimulatory molecules that can be expressed on the surface of T cells.

The "+" encompasses a case in which the corresponding protein is expressed and a case in which the expression level of the corresponding protein is higher than other subsets. That is, the "+" in the present invention includes "high (hi)" indicating high expression. Likewise, the "-" encompasses a case in which a corresponding protein is not expressed as well as a case in which the expression level of a corresponding protein is lower than those of other subsets, and a case in which the expression level is so low that detection of the corresponding protein is impossible. That is, in the present invention, "-" includes "low (lo)" indicating low expression.

In the present invention, "CD8+ T cells" are T cells characterized by expressing the CD8 glycoprotein on a cell surface, and cytotoxic T cells which have functions of recognizing external antigens expressed in pathogens such as viruses or bacteria and tumors, transplanted organs and removing cells expressing the corresponding antigens.

CD8+ T cells are divided into various subsets according to a differentiation state, and these subsets may be distinguished by cell surface markers such as C-C chemokine receptor type 7 (CCR7), CD45RA, CD45RO, and CD57. Particularly, CCR7 and CD45RA were widely used to distinguish the differentiation state of CD8+ T cells in both healthy subjects and patients. Fully mature CD8+ T cells are cells that exit the thymus as naive T cells (CCR7+ CD45RA+, Tn). Upon activation, the expression of CCR7 and CD45RA in Tn cells is gradually down-regulated, inducing differentiation into effector T cells (CCR7- CD45RA-; Teff). Teff cells further differentiate into effector memory T cells (CCR7- CD45RA-; Tem), or central memory T cells (CCR7+ CD45RA-; Tcm) with a gradually increase in CCR7 expression. However, some cells differentiate into Temra cells re-expressing CD45RA (CCR7- CD45RA+). Temra among the various subsets of CD8+ T cells is considered to be the most terminally differentiated subset, and actively produces perforin and granzyme B, and has low cell proliferation capacity and differentiation plasticity.

Each CD8+ T cell subset may also be subdivided based on expression patterns of costimulatory molecules, CD27 and CD28. Although Tn cells generally express both CD27 and CD28 at high levels, in the cases of Teff, Tem, and Temra cells, the expression patterns of CD27 and CD28 vary.

Based on the above information, the present inventors searched for the correlation between the levels of CD8+ T cell subsets and the prognosis of a cancer patient and/or responsiveness to an anticancer agent, and as a result, it was confirmed that the proportion of a specific CD8+ T cell subset among total CD8+ T cells determines the prognosis of a cancer patient and the responsiveness to an anticancer agent.

Meanwhile, "perforin" is a protein found in cytotoxic T cells and natural killer cells (NK cells), and a cytolytic protein that can form a pore in the cell membrane of a target cell (e.g., cancer cell). "Granzyme" is also a representative protein that is also expressed in cytotoxic T cells and NK cells and can induce the apoptosis of target cells. That is, cytotoxic T cells induce the death of target cells by perforating the membrane of the target cell using perforin and delivering granzyme (e.g., granzyme B). "Interleukin-2 (IL-2)" refers to a cytokine playing an essential role in the survival, expansion, and functional activation of immune cells. T cells express an IL-2 receptor on their surface, and cell division and activity increase when IL-2 interacts with the receptor.

That is, all of perforin, granzyme B, and IL-2 are proteins highly related to the function of T cells, and the present inventors found there are differences in the expression levels of the proteins in CD8+ T cell subsets, and it was found that cells exhibiting a specific expression pattern are associated with the prognosis of a cancer patient and the responsiveness to an anticancer agent.

Specifically, the present inventors confirmed that CD27+ CD28+ CCR7- CD45RA+ CD8+ T cells (DP Temra) have an inverse correlation with tumor mutation burden (TMB) and the number of tumor-infiltrating CD8 T cells. That is, it was confirmed that the DP Temra according to the present invention has a negative correlation with cancer antigen mutations or cancer antigen immunogenicity. Further, the present inventors confirmed that DP Temra can reflect the immunogenicity (and antigenicity) state of tumor antigens in a cancer patient, and patients with a low level of DP Temra

EP 4 321 625 A1

among total Temra exhibit a partial response (PR) after cancer immunotherapy.

That is, the prediction method according to the present invention may further include predicting that, when the proportion of DP Temra among total Temra is lower compared to the control, a subject has a better prognosis, or the responsiveness to anticancer treatment is higher.

Further, the prediction method according to the present invention may further include determining the responsiveness to anticancer treatment (preferably, anti-PD-1 or anti-PD-L1 treatment) to be higher when the number of Temra (CD45RA$^+$, CCR7$^-$) cells in which both CD27 and CD28 are positive is detected at a predetermined proportion or less relative to the total number of Temra (CD45RA$^+$, CCR7$^-$) cells present in the sample, and specifically, the predetermined proportion may be 1 to 10%, 1 to 9%, 1 to 8%, 1 to 7%, 1 to 6%, 2 to 10%, 3 to 10%, 4 to 8%, 5 to 7%, or 6%, but the present invention is not limited thereto. The information providing method according to one embodiment may determine the responsiveness to cancer immunotherapy to be higher when the number of Temra (CD45RA$^+$, CCR7$^-$) cells in which both of CD27 and CD28 are active is 10%, 9%, 8%, 7%, or 6% or less relative to the total number of Temra (CD45RA$^+$, CCR7$^-$) cells present in the sample.

In addition, the present inventors confirmed that the proportion of the CD8$^+$ T cell subsets can reflect toxic T-cell immunity (anticancer treatment, particularly, capability to respond to cancer immunotherapy) of a cancer patient, and these were collectively named "Fitness factor:"

(a) the proportion of CD27$^+$ CD28$^+$ CCR7$^-$ CD45RA$^-$ CD8$^+$ T cells (DP Tem) among total CCR7$^-$ CD45RA$^-$ CD8$^+$ T cells (Tem);

(b) the proportion of CD27$^-$ CD28$^-$ CCR7$^-$ CD45RA$^-$ CD8$^+$ T cells (DN Tem) among total CCR7$^-$ CD45RA$^-$ CD8$^+$ T cells (Tem);

(c) the proportion of perforin$^+$ CCR7$^-$ CD45RA$^-$ CD8$^+$ T cells (perforin-expressing Tem) among total CCR7$^-$ CD45RA$^-$ CD8$^+$ T cells (Tem);

(d) the proportion of Granzyme B$^+$ CCR7$^-$ CD45RA$^-$ CD8$^+$ T cells (Granzyme B-expressing Tem) among total CCR7$^-$ CD45RA$^-$ CD8$^+$ T cells (Tem); and

(e) the proportion of IL-2$^+$ CCR7$^-$ CD45RA$^-$ CD8$^+$ T cells (IL-2-expressing Tem) among total CCR7$^-$ CD45RA$^-$ CD8$^+$ T cells (Tem).

Specifically, the present inventors confirmed that patients with a higher DP Tem proportion among total Tem; and patients with a lower DN Tem proportion among total Tem exhibit a partial response (PR) after cancer immunotherapy (anti-PD-1/PD-L1 treatment).

Accordingly, the prediction method according to the present invention may further include determining that a subject has a better prognosis, or predicting that the responsiveness to anticancer treatment (preferably, anti-PD-1 or anti-PD-L1 treatment) is higher when the proportion of DP Tem among total Tem is higher compared to the control, and/or the proportion of DN Tem among total Tem is lower compared to the control.

In addition, the present inventors confirmed that patients exhibiting a partial response after cancer immunotherapy have an increased proportion of perforin$^+$ Tem and/or granzyme B$^+$ Tem among total Tem after cancer immunotherapy (anti-PD-1 or anti-PD-L1 treatment) compared to that prior to cancer immunotherapy, and confirmed that patients with this tendency to increase had a lower proportion of perforin$^+$ Tem and/or granzyme B$^+$ Tem among total Tem before cancer immunotherapy.

Accordingly, when the proportion of perforin$^+$ Tem among total Tem is lower, and/or the proportion of granzyme B$^+$ Tem among total Tem is lower, compared to the control, the prediction method according to the present invention may further include determining that a subject has a better prognosis, or predicting that responsiveness to anticancer treatment (preferably, anti-PD-1 or anti-PD-L1 treatment) is higher. Here, preferably, the subject may be a subject prior to anticancer treatment, and more preferably, a subject prior to cancer immunotherapy. Preferably, the prediction method according to the present invention may further include determining that a subject is a better prognosis, or the responsiveness to anticancer treatment is higher when the proportion of perforin$^+$ Tem among total Tem and/or the proportion of granzyme B$^+$ Tem among total Tem increase(s) after cancer immunotherapy (preferably, anti-PD-1 or anti-PD-L1 treatment), compared to that prior to cancer immunotherapy.

In addition, the present inventors confirmed that patients exhibiting a partial response after cancer immunotherapy have a decreased proportion of IL-2$^+$ Tem among total Tem after cancer immunotherapy (anti-PD-1/PD-L1 treatment) compared to that prior to cancer immunotherapy.

Accordingly, the prediction method according to the present invention may further include determining that a subject has a better prognosis, or predicting that the responsiveness to anticancer treatment (preferably, anti-PD-1 or anti-PD-L1 treatment) is higher when the proportion of IL-2$^+$ Tem among total Tem is higher than that of the control. Here, preferably, the subject may be a subject prior to anticancer treatment, and more preferably, a subject prior to cancer immunotherapy. Preferably, the prediction method according to the present invention may further include, when the proportion of IL-2$^+$ Tem among total Tem after cancer immunotherapy (preferably, anti-PD-1 or anti-PD-L1 treatment)

12

compared to that prior to the cancer immunotherapy, determining that a subject has a better prognosis, or predicting that responsiveness to anticancer treatment is higher.

The above-described method of predicting the responsiveness to anticancer treatment may be suitable for the prediction of the responsiveness to cancer immunotherapy, particularly, PD-L1 treatment or PD-1 treatment.

In fact, the present inventors confirmed that the higher the DP Tem proportion and the lower the DP Temra proportion, the higher the responsiveness of a cancer patient to PD-1 treatment; however, in the case of concurrent chemoradiation therapy (CCRT), patients with a high DP Tem proportion and a low DP Temra proportion exhibited the worst responsiveness. According to this, it is predicted that, for patients with a high DP Tem proportion and a low DP Temra proportion, anti-PD-1 or anti-PD-L1 treatment is suitable for cancer treatment, and for other patients, it is considered that CCRT treatment is appropriate.

In addition, as a result of confirming the correlation between a level of the CD8$^+$ T cell subset and the responsiveness to IL-2 treatment (anticancer treatment with IL-2 alone or in combination with IL-2), the present inventors confirmed that (i) as the initial Temra proportion among total CD8$^+$ T cells before IL-2 treatment is higher, the responsiveness of a cancer patient to IL-2 treatment is higher, and (ii) as the DP Tem proportion among total Tem is lower, the tilTemra proportion is higher.

Accordingly, the prediction method according to the present invention is to predict responsiveness to IL-2 treatment, and the subject is a subject prior to anticancer treatment (anticancer treatment with IL-2 alone or in combination with IL-2), and may include

measuring the proportion of CCR7$^-$ CD45RA$^+$ CD8$^+$ T cells (Temra) among total CD8$^+$ T cells; and determining that a subject has a better prognosis, or predicting that the responsiveness to anticancer treatment of a subject is higher than that of the control when the proportion of Temra among total CD8$^+$ T cells is higher than that of the control. Here, in a patient determined to have a better prognosis, or a patient predicted to have a higher responsiveness to anticancer treatment, the proportion of DP Temra among total Temra may be higher, equivalent, or lower than that of the control. Preferably, the proportion of DP Temra among total Temra may be lower than that of the control, but the present invention is not limited thereto.

Alternatively, the prediction method according to the present invention is to predict the responsiveness to IL-2 treatment, and the subject is a subject prior to anticancer treatment (anticancer treatment with IL-2 alone or in combination with IL-2), and may include

measuring the proportion of CD27$^+$ CD28$^+$ CCR7$^-$ CD45RA$^-$ CD8$^+$ T cells (DP Tem) among total CCR7$^-$ CD45RA$^-$ CD8$^+$ T cells (Tem); and predicting that, when the proportion of DP Tem among total Tem is lower than that of the control, the responsiveness of a subject to anticancer treatment is higher than that of the control.

Here, the IL-2 treatment may be performed by a complex of IL-2 and an IL-2-specific antibody (preferably, TCB2). Most preferably, the IL-2 treatment may be performed by a complex in which anti-hEL-2 antibody or an antigen-binding fragment thereof is coupled with hIL-2 (TCB2-IL-2), as disclosed in Korean Patent No. 10-2099593.

The prediction method according to the present invention may be performed before, during or after anticancer treatment of a cancer patient. Since it can be seen in advance whether the patient has high responsiveness to anticancer treatment even before anticancer treatment by the method of the present invention, the method of the present invention may be conducted before anticancer treatment.

In the present invention, "cancer" is characterized by uncontrolled cell growth, and refers to a cell mass called a tumor, formed by abnormal cell growth and infiltrating into peripheral tissue, and if severe, possibly spreading to other organs of the body. The cancer may be solid cancer or blood cancer, and non-limiting examples thereof include, but not limited to, melanoma, small cell lung cancer, non-small cell lung cancer, gliomas, liver cancer, thyroid tumors, stomach cancer, prostate cancer, ovarian cancer, bladder cancer, lung cancer, colorectal cancer, breast cancer, Hodgkin's lymphoma, urothelial cell carcinoma, glioblastoma, endometrial cancer, cervical cancer, kidney cancer, colon cancer, pancreatic cancer, esophageal carcinoma, head and neck cancer, mesothelioma, sarcomas, cholangiocarcinoma, adenocarcinoma of the small intestine, childhood malignancies, epidermal cancer, testicular cancer, blood cancer, and brain cancer, and may include any type of cancer that can be treated by an immune checkpoint inhibitor or immune cell activator without limitation. Preferably, the cancer according to the present invention may be non-small cell lung cancer or melanoma.

In the present invention, "diagnosis" includes determining the susceptibility of a subject to a specific disease or disorder, determining whether a subject currently has a specific disease or disorder, determining the prognosis of a subject with a specific disease or disorder, or performing therametrics (e.g., monitoring a subject condition to provide information on therapeutic efficacy).

In the present invention, "prognosis" means the prediction of disease occurrence, progression, treatment success, recovery, relapse, and drug resistance, and refers to a forecast or preliminary evaluation. That is, the prognosis refers to the prediction of medical outcomes (e.g., long-term survival possibility, disease-free survival, etc.), a positive prognosis, or a negative prognosis, the negative prognosis includes the progression or mortality of a disease, such as relapse, worsening of symptoms (in the case of cancer, tumor growth, metastasis, etc.), drug resistance, etc., and the positive prognosis includes the remission of a disease (the reduction or disappearance of a disease), or the alleviation or stabilization of a disease. Preferably, in the present invention, "prognosis" refers to the determination by comprehensively considering all of the occurrence of cancer or a tumor, severity, progression, relapse, disease-free survival, progression-free survival (PFS), overall survival, and complications.

In the present invention, "responsiveness" refers to the degree of response to medical treatment or a drug, and responsiveness prediction means prediction of the degree of a therapeutic effect when medical treatment or a drug is administered to a target patient. Specifically, when the responsiveness to the corresponding treatment or drug is high, an excellent therapeutic effect can be expected, whereas when responsiveness is low, a poor therapeutic effect is expected. In the present invention, predicting that "responsiveness is high" includes predicting that a patient has a high probability of exhibiting a partial response or complete response after anticancer treatment.

The term "prediction" used herein refers to guessing in advance about medical consequences, and it means guessing in advance about the course (the progression, alleviation, relapse, aggravation, drug resistance, etc.) of a disease of a patient diagnosed with cancer for the purpose of the present invention.

In the present invention, "anticancer treatment" may be one or more selected from the group consisting of cancer immunotherapy, chemotherapy, radiation therapy, and chemotherapy-radiation therapy combination therapy. In one embodiment of the present invention, the cancer immunotherapy may be performed using an immune checkpoint inhibitor or immune cell activator. The immune checkpoint inhibitor is preferably, an immune checkpoint-targeting antibody. In another embodiment of the present invention, the conventional cancer immunotherapy may be selected from the group consisting of anti-PD-L1 treatment, anti-PD-1 treatment, anti-CTLA-4 treatment, anti-LAG3 treatment, anti-TIM3 treatment, anti-BTLA treatment, anti-4-1BB treatment, anti-OX40 treatment, cytokine treatment, IL-2 treatment, cytokine receptor treatment, CAR-T cell treatment, and autologous $CD8^+$ T immune cell treatment. Preferably, the anticancer treatment according to the present invention may be treatment with a drug that activates or proliferates $CD8^+$ T cells in cancer tissue, but the present invention is not limited thereto. More preferably, the anticancer treatment according to the present invention may be anti-PD-1/PD-L1 treatment, and may be performed with, more preferably, Tecentriq, Keytruda, or Opdivo. In addition, the anticancer treatment according to the present invention may be a complex in which anti-hEL-2 antibody or an antigen-binding fragment thereof is coupled with hIL-2 (TCB2-IL-2), disclosed in Korean Patent No. 10-2099593.

Accordingly, a "subject" according to the present invention may be a patient who is receiving or will receive treatment with an immune checkpoint inhibitor or an immune cell activator, and specifically, a patient who is receiving or will receive anti-PD-L1 treatment, anti-PD-1 treatment, anti-CTLA-4 treatment, anti-LAG3 treatment, anti-TIM3 treatment, anti-BTLA treatment, anti-4-1BB treatment, anti-OX40 treatment, cytokine treatment (e.g., IL-2 treatment, IL-4 treatment, IL-7 treatment, or IL-15 treatment), cytokine-antibody complex treatment, cytokine receptor treatment (e.g., IL-2 receptor treatment, IL-4 receptor treatment, IL-7 receptor treatment, IL-15 receptor treatment), CAR-T cell treatment, and autologous $CD8^+$ T immune cell treatment, and more specifically, the cancer patient may be a patient who is receiving or will receive anti-PD-1/PD-L1 treatment (more specifically, Tecentriq, Keytruda, or Opdivo).

Preferably, the cytokine-antibody complex treatment may be performed by a conjugate of an IL-2 cytokine; and an anti-IL-2 antibody or a fragment thereof. More preferably, the cytokine-antibody complex treatment may be a complex in which antihIL-2 antibody or an antigen-binding fragment thereof is coupled with hIL-2, disclosed in Korean Patent No. 10-2099593.

When the anticancer treatment according to the present invention is chemotherapy, the anticancer treatment may be performed with one or more selected from the group consisting of pemetrexed (Alimta), oxaliplatin, cisplatin, gemcitabine, carboplatin, 5-FU, cyclophosphamide, paclitaxel, vincristine, etoposide, and doxorubicin, but the present invention is not limited thereto.

When the anticancer treatment according to the present invention is radiation therapy, the anticancer treatment may be performed by applying high-energy radiation, including, but not limited to, x-rays, gamma rays, and neutrons, to a patient. Such types of therapy may include, but not limited to, external light therapy, internal radiation therapy, implant radiation, brachytherapy, and body radiotherapy.

In the present invention, "control" may be a biological sample isolated from a healthy subject or a cancer patient showing a complete response or partial response after anticancer treatment (i.e., a patient with responsiveness to anticancer treatment), but the present invention is not limited thereto. That is, the prediction method according to the present invention may predict a relative prognosis and/or relative responsiveness to anticancer treatment between cancer patients by measuring the proportions of $CD8^+$ T cell subsets of cancer patients and then comparing the proportions.

In the present invention, a "biological sample" may include something that is collected from a subject whose cancer prognosis is predicted or a subject whose responsiveness to anticancer treatment is predicted without limitation. For example, the biological sample may be selected from blood, whole blood, plasma, urine, saliva, tissue, cells, an organ, bone marrow, a microneedle aspiration specimen, a core needle biopsy specimen, and a vacuum aspiration biopsy specimen.

Preferably, the biological sample according to the present invention is a non-invasive sample such as blood, whole blood, and plasma. More preferably, the blood is peripheral blood. That is, by the prediction method according to the present invention, it is possible to accurately predict a prognosis and responsiveness to anticancer treatment by detecting a specific cell type in a blood sample rather than tissue. When the sample is a blood sample, information for predicting the responsiveness of a cancer patient to a cancer immunotherapeutic agent without involving tissue analysis of a cancer lesion in a cancer patient may be provided. In this case, the possibility of generating variables such as the diversity of cancer cells and cancer tissue, and the diversity of primary cancer and metastatic cancer tissue in cancer lesion tissue-based analysis may be reduced. In addition, information for predicting the responsiveness of a cancer patient to cancer immunotherapy under various conditions such as carcinoma, the progression of cancer, and anticancer drug treatment may be provided, enabling rapid and accurate prediction. Accordingly, the CD8$^+$ cell subset according to the present invention may be a CD8$^+$ cell subset in peripheral blood.

The biological sample may be pretreated prior to the use for detection or diagnosis. For example, examples of pretreatment methods may include homogenization, filtration, distillation, extraction, concentration, inactivation of interfering components, and the addition of reagents. The sample may be prepared to increase the detection sensitivity of a protein marker, and for example, a sample obtained from a patient may be pretreated using anion exchange chromatography, affinity chromatography, size exclusion chromatography, liquid chromatography, sequential extraction, or gel electrophoresis.

The term "measurement" used herein refers to measuring and detecting the presence (expression) of target materials (in the present invention, total CD8$^+$ T cells and CD8$^+$ T cell subsets; and the following molecular markers for distinguishing them: CD27, CD28, granzyme B, perform, IL-2, CCR7, CD45RA, CD8, etc.), or measuring and detecting the change in levels (expression levels) of present target materials. That is, measuring the expression level of a specific gene (i.e., detecting the presence or absence of expression), or measuring the degree of qualitative and quantitative changes in mRNA and/or protein expressed from the gene. The measurement may be performed by both qualitative and quantitative methods (analyses) without limitation. Types of qualitative and quantitative methods for measuring an mRNA or protein level are well known in the art, and include experimental methods described herein. A method of comparing the specific level of mRNA or a protein for each method is well known in the art. Accordingly, target mRNA and/or protein detection includes detection of the presence or absence of a CD8$^+$ T cell subset, or confirming an increase (upregulation) or decrease (down-regulation) in level (or proportion) of the CD8$^+$ T cell subset according to the present invention.

The term "analysis" used herein preferably means "measurement," and the qualitative analysis may mean measuring or detecting the presence or absence of a target material, and the quantitative analysis may mean measuring and detecting the change in level (expression level) or amount of a target material. The analysis or detection in the present invention may be performed by both a qualitative method and a quantitative method without limitation, and preferably, the quantitative measurement may be performed.

Herein, "the level is increased" means that something that has not been detected is detected, or a detection level is relatively larger than a normal level. The meaning of the opposite term can be understood as having the opposite meaning to the above definition by those of ordinary skill in the art.

For example, the fact that the level is "increased" means that the level of an experimental group is at least 1%, 2%, 3%, 4%, 5%, 10% or higher, e.g., 5%, 10%, 20%, 30%, 40%, 50%, 60%, 70%, 80%, 90% or higher, and/or 0.5 times, 1.1 times, 1.2 times, 1.4 times, 1.6 times, 1.8 times or more higher than that of a control. Specifically, it means that the level of an experimental group is increased 1 to 1.5 times, 1.5 to 2 times, 2 to 2.5 times, 2.5 to 3 times, 3 to 3.5 times, 3.5 to 4 times, 4 to 4.5 times, 4.5 to 5 times, 5 to 5.5 times, 5.5 to 6 times, 6 to 6.5 times, 6.5 to 7 times, 7 to 7.5 times, 7.5 to 8 times, 8 to 8.5 times, 8.5 to 9 times, 9 to 9.5 times, 9.5 to 10 times, or 10 times or more that of the control, but the present invention is not limited thereto.

In the present invention, the protein level may be measured by a method such as western blotting, ELISA, radioimmunoassay (RIA), radioimmunodiffusion, Ouchterlony immunodiffusion, Rocket immunoelectrophoresis, tissue immunostaining, immunoprecipitation assay, complement fixation assay, mass spectrometry, FACS, and/or a protein chip. Preferably, the proportion of a CD8$^+$ T cell subset among total CD8$^+$ T cells according to the present invention may be measured through ELISA or flow cytometry.

In the present invention, the mRNA level may be measured by a method such as PCR, RNase protection assay, northern blotting, southern blotting, *in situ* hybridization, a DNA chip, and/or an RNA chip.

In addition, the present invention provides a composition for predicting the prognosis or responsiveness to anticancer treatment of a cancer patient, including an agent for detecting a CD8$^+$ T cell subset as an active ingredient, and the CD8$^+$ T cell subset includes one or more selected from the group consisting of the following:

CCR7$^-$ CD45RA$^+$CD8$^+$ T cells (Temra);
CD27$^+$ CD28$^+$ CCR7$^-$ CD45RA$^+$CD8$^+$ T cells (DP Temra);
CCR7$^-$ CD45RA$^-$ CD8$^+$ T cells (Tem);

CD27$^+$ CD28$^+$ CCR7$^-$ CD45RA$^-$ CD8$^+$ T cells (DP Tem);
CD27$^-$ CD28$^-$ CCR7$^-$ CD45RA$^-$ CD8$^+$ T cells (DN Tem);
perforin$^+$ CCR7$^-$ CD45RA$^-$ CD8$^+$ T cells (perforin-expressing Tern);
granzyme B$^+$ CCR7$^-$ CD45RA$^-$ CD8$^+$ T cells (granzyme B-expressing Tern); and
IL-2$^+$ CCR7$^-$ CD45RA$^-$ CD8$^+$ T cells (IL-2-expressing Tern).

When the detection of the CD8$^+$ T cell subset is performed by measurement of an mRNA level of the molecular marker of each cell subset, an agent that measures each mRNA level may be a primer set or probe specifically binding to mRNA. The composition of the present invention including a primer set or probe specific to the mRNA of the markers may further include an agent required for a known method of detecting RNA. The known method of detecting RNA using the composition of the present invention may be used without limitation to measure an mRNA level of biomarkers according to the present invention in a subject.

The "primer" or "probe" may be chemically synthesized using a method of synthesizing a solid support such as phosphoramidite or other well-known methods. In addition, the primer or probe may be modified in various ways according to methods known in the art to the extent that hybridization with mRNA is not hindered. Examples of such modifications are methylation, capping, substitution of natural nucleotides with one or more homologs, and modifications between nucleotides, for example, uncharged linkers (e.g., methyl phosphonate, phosphotriester, phosphoramidate, carbamate, etc.) or charged linkers (e.g., phosphorothioate, phosphorodithioate, etc.), and binding of a fluorescent or enzymatic labeling material.

When the detection of a CD8$^+$ T cell subset is performed by measuring a protein level of the molecular marker of each cell subset, the agent that measures an expression level of each protein may be an antibody or aptamer specifically binding to the corresponding protein. Preferably, the antibody or aptamer may be an antibody or aptamer particularly suitable for ELISA or flow cytometry.

The term "antibody" used herein refers to a specific protein molecule directed against an antigenic site. For the purpose of the present invention, the antibody refers to an antibody specifically binding to a marker protein, and includes all of polyclonal antibodies, monoclonal antibodies, and recombinant antibodies. In addition, as long as it has an antigen-antibody binding property, a part of the entire antibody is also included in the antibody of the present invention, and all kinds of immunoglobulin antibodies specifically binding to the proteins of the present invention are included. For example, the antibody used herein includes a complete antibody form with two full-length light chains and two full-length light chains as well as functional fragments of the antibody, that is, Fab, F(ab'), F(ab')$_2$, and Fv, each having an antigen-binding function. Furthermore, the antibody of the present invention includes special antibodies such as humanized antibodies and chimeric antibodies and recombinant antibodies as long as they can specifically bind to the protein.

The term "aptamer" used herein is a material capable of specifically binding to an analyte to be detected in a sample, and refers to a single-stranded nucleic acid (DNA, RNA, or modified nucleic acid) with a stable tertiary structure by itself, and it can specifically confirm the presence of a target protein in the sample. The aptamer may be prepared by synthesizing an oligonucleotide by determining a sequence thereof with selective and high binding affinity to a target protein to be confirmed, and modifying the 5' end or the 3' end of the oligonucleotide with -SH, -COOH, -OH, or NH$_2$ to bind to a functional group of an aptamer chip according to a general method of preparing an aptamer, but the present invention is not limited thereto.

The composition according to the present invention may further include an agent required for a known method of detecting a protein, and the expression level of a protein in a subject (a biological sample acquired from the subject of the present invention) may be measured using the known method of detecting a protein using the composition without limitation.

In addition, the present invention provides a kit for predicting the prognosis or responsiveness to anticancer treatment of a cancer patient, which includes the composition according to the present invention.

In the present invention, a "kit" means, particularly, an apparatus for examination for predicting the prognosis of cancer or predicting responsiveness to anticancer treatment by measuring the amounts of mRNA or protein of total CD8$^+$ T cells and CD8$^+$ T cell subsets themselves in a biological sample, or molecular markers (CD27, CD28, granzyme B, perforin, IL-2, CCR7, CD45RA, CD8, etc.) for detecting them, and includes any form that can measure the expression levels of the markers from a biological sample isolated from a patient, and further, a form that can detect CD8$^+$ T cell subsets. Accordingly, the kit of the present invention may include not only an antibody recognizing a target protein as a marker or a primer set or probe recognizing mRNA as a marker but also one or more types of component compositions, solutions or devices suitable for an analysis method . For example, the kit according to the present invention may include a plate coated with the biomarker peptide according to the present invention, an enzyme and/or a substrate for a luminescent reaction to detect an antibody-peptide binding, a washing solution, and a control sample, but the present invention is not limited thereto. Further, the kit may further include protocols or instructions describing the prediction method according to the present invention.

The type of kit may be all kits known in the art, and may include, for example, an immunochromatography strip kit, a Luminex assay kit, a protein microarray kit, an ELISA kit, a flow cytometry kit, or an immunological dot kit. In addition, the kit may be a kit for implementing western blotting, immunoprecipitation assay, complement fixation assay, flow cytometry, or a protein chip, and an additional component suitable for each analysis method may be further included.

In addition, the present invention provides a method of treating cancer, including the following steps:

(S 1) measuring the proportion of $CD27^+$ $CD28^+$ $CCR7^-$ $CD45RA^+CD8^+$ T cells (DP Temra) among total $CCR7^-$ $CD45RA^+CD8^+$ T cells (Temra) in a biological sample isolated from a subject;
(S2) predicting the prognosis or responsiveness to anticancer treatment of a cancer patient by comparing the proportion of DP Temra among total Temra measured in (S 1) with a control; and
(S3) performing anticancer treatment on a subject predicted to have a better prognosis or responsiveness to the anticancer treatment.

In addition, the present invention provides a method of treating cancer, including the following steps:

(S 1) measuring the proportion of $CCR7^-$ $CD45RA^+CD8^+$ T cells (Temra) among total $CD8^+$ T cells in a biological sample isolated from a subject;
(S2) measuring the prognosis or responsiveness to anticancer treatment of a cancer patient by comparing the proportion of Temra among total $CD8^+$ T cells measured in (S 1); and
(S3) performing anticancer treatment (preferably, IL-2 or TCB2-IL-2) on a subject predicted to have a better prognosis or responsiveness to the anticancer treatment.

In addition, the present invention provides a method of treating cancer, including the following steps:

(S 1) measuring the proportion of $CD27^+$ $CD28^+$ $CCR7^-$ $CD45RA^-$ $CD8^+$ T cells (DP Tem) among total $CCR7^-$ $CD45RA^-$ $CD8^+$ T cells (Tern) in a biological sample isolated from a subject;
(S2) predicting the prognosis or responsiveness to anticancer treatment of a cancer patient by comparing the proportion of DP Tem among total Tem measured in (S1); and
(S3) performing anticancer treatment (preferably, IL-2 or TCB2-IL-2) on a subject predicted to have a better prognosis or responsiveness to the anticancer treatment.

The treatment method may further include measuring the above-described other Fitness factors and comparing them with the control.

"Treatment" in the present invention means all actions involved in alleviating or beneficially changing symptoms of a target disease and symptoms of related metabolic abnormalities by administration of the pharmaceutical composition according to the present invention, and "alleviation" means all actions that reduce a parameter associated with a target disease by administration of the composition according to the present invention, for example, the degree of a symptom.

In the present invention, the anticancer treatment may use a method such as chemotherapy, immunotherapy, radiotherapy, surgery, biological therapy, or antibiotic administration.

The surgery includes all therapeutic or diagnostic procedures involving the methodical action of hands or hands together with a device with respect to the body of an individual to achieve a curative, therapeutic or diagnostic effect.

The biological therapy means a therapeutic method that directly/indirectly uses the immune system of the human body by using a biological agent containing a material derived from an organism or an organism, and the biological agent includes vaccines whose potency and safety cannot be evaluated only by a physical or chemical test, allergens, antigens, hormones, cytokines, enzymes, blood and plasma, immune sera, monoclonal antibodies, fermentation products, antitoxins, and laboratory diagnostics. The biological agent may be, for example, one or more selected from the group consisting of, for example, adalimumab, alemtuzumab, bevacizumab, cetuximab, daratumumab, panitumumab, rituximab, trastuzumab, pertuzumab, ipilimumab, nivolumab, pembrolizumab, atezolizumab, durvalumab, avelumab, tocilizumab, sarilumab, satralizumab, and siltuximab, but the present invention is not limited thereto.

However, preferably, the anticancer treatment is performed, as described above, by one or more selected from the group consisting of cancer immunotherapy, chemotherapy, radiation therapy, and a combination of chemotherapy-radiation therapy, and more preferably, by cancer immunotherapy, and most preferably, anti-PD-1 therapy, anti-PD-L1 therapy, and/or IL-2 therapy.

Meanwhile, the prediction method according to the present invention and the information providing method therefor may be performed using a prediction model based on machine learning. Preferably, the machine learning may be machine learning based on the K-nearest neighbor (K_NN) algorithm.

That is, the present invention provides a system for predicting the prognosis or responsiveness to anticancer treatment of a cancer patient, including the following components:

(S1) a measurement unit for extracting data on the proportion of (i) CD27$^+$ CD28$^+$ CCR7$^-$ CD45RA$^+$CD8$^+$ T cells (DP Temra) among total CCR7$^-$ CD45RA$^+$CD8$^+$ T cells (Temra), and (ii) the proportion of CD27$^+$ CD28$^+$ CCR7$^-$ CD45RA$^-$ CD8$^+$ T cells (DP Tem) or CD27$^-$ CD28$^-$ CCR7$^-$ CD45RA$^-$ CD8$^+$ T cells (DN Tem) among total CCR7$^-$ CD45RA$^-$ CD8$^+$ T cells (Tem);

(S2) a distance calculation unit that calculates a distance between the extracted data and sample data; and

(S2) an analysis unit that calculates the probability of a cancer patient having a good prognosis or responsiveness to anticancer treatment using sample data at the closest distance within a predetermined range based on the extracted data.

In one embodiment of the present invention, the distance calculation unit may use a K-NN algorithm method.

In another embodiment of the present invention, the distance calculation may be performed using a Euclidean distance calculation method or a Manhattan distance calculation method. More preferably, the Euclidean distance calculation method may calculate the distance through the following formula:

$$d(\mathrm{x},y) = d(y,\mathrm{x}) = \sqrt{(y1 - x1)^2 + (y2 - x2)^2}$$

In this formula, x is the proportion of DP Temra among total Temra, and y is the proportion of DP Tem or DN Tem among total Tem, and vice versa.

In still another embodiment of the present invention, the distance calculation unit may use a K-NN algorithm method, the predetermined range means that K may be 1 to 10, 1 to 9, 1 to 8, 1 to 7, 1 to 6, 1 to 5, 1 to 4, 1 to 3, 1 to 2, 2 to 10, 3 to 10, 4 to 10, 5 to 10, 6 to 10, 7 to 10, 8 to 10, 6 to 9, 6 to 8, or 7.

In yet another embodiment of the present invention, the analysis unit may calculate the probability of a cancer patient having a good prognosis or responsiveness to anticancer treatment by calculating the proportion of data of a patient showing a complete response or partial response after anticancer treatment in the entire sample data at the closest distance in a predetermined range based on the extracted data.

In addition, the present invention provides a method of predicting the prognosis or responsiveness to anticancer treatment of a cancer patient using the system for predicting the prognosis or responsiveness to anticancer treatment of a cancer patient:

(S 1) extracting data on the proportion of (i) CD27$^+$ CD28$^+$ CCR7$^-$ CD45RA$^+$CD8$^+$ T cells (DP Temra) among total CCR7$^-$ CD45RA$^+$CD8$^+$ T cells (Temra), and (ii) the proportion of CD27$^+$ CD28$^+$ CCR7$^-$ CD45RA$^-$ CD8$^+$ T cells (DP Tem) or CD27$^-$ CD28$^-$ CCR7$^-$ CD45RA$^-$ CD8$^+$ T cells (DN Tem) among total CCR7$^-$ CD45RA$^-$ CD8$^+$ T cells (Tem);

(S2) calculating the distance between the extracted data and sample data; and

(S3) calculating the probability of a cancer patient having a good prognosis or responsiveness to anticancer treatment using sample data located at the closest distance in a predetermined range based on the extracted data.

Hereinafter, preferred examples are presented to better understand the present invention. However, the following examples are provided to more easily understand the present invention, and the content of the present invention is not limited by the following examples.

In the following examples, the following abbreviations have the following meanings.

- CD8 T cells (also represented by 'CD8$^+$ cells'): CD8$^+$ CD3$^+$ T cells
- Tn: CCR7$^+$ CD45RA$^+$ CD8 T cells among CD8 T cells
- Tern: CCR7$^+$ CD45RA$^-$ CD8 T cells among CD8 T cells
- Tern: CCR7$^-$ CD45RA$^-$ CD8 T cells among CD8 T cells
- Temra: CCR7$^-$ CD45RA$^+$CD8 T cells among CD8 T cells
- DP: CD27$^+$ and CD28$^+$
- DN: CD27$^-$ and CD28$^-$

**[Experimental methods]**

**1. Human sample**

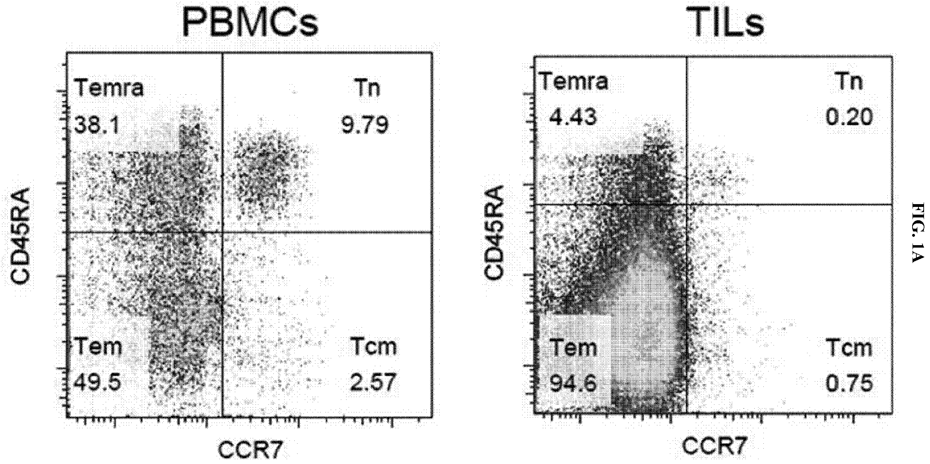

For the following examples, tumor tissue and blood samples of patients with non-small-cell lung cancer (NSCLC) (n-26) were harvested at Chonnam National University Hwasun Hospital. In some examples, another set of blood samples of NSCLC cancer patients (n=30) was used. The clinical characteristics of the patients are summarized in Tables 1 and 2. Table 1 shows the tumor and blood characteristics of 26 NSCLC patients related to the examples of FIGS. 1A- 6I. Table 2 shows 30 NSCLC patients related to the examples of FIGS. 6F- 6I, and FIG. 12B-12E and the blood characteristics thereof. The experiments of the examples were approved by the Institutional Review Boards of Chonnam National University Hwasun Hospital.

[Table 1]

| Age | | 67(40~78) |
|---|---|---|
| Sex | Male | 17 (65%) |
| | Female | 9 (35%) |
| Histology | ADC | 13 (50%) |
| | SQC | 5 (15%) |
| | Unknown | 8 (35%) |
| P Stage | IA1 | 1 (4%) |
| | IA2 | 1 (4%) |
| | IA3 | 5 (19%) |
| | Ib | 6 (23%) |
| | IIa | 1 (4%) |
| | IIb | 2 (8%) |
| | IIIa | 2 (8%) |
| | Unknown | 8 (31%) |
| N Stage | N0 | 8 (31%) |
| | N1 | 2 (8%) |
| | N2 | 2 (8%) |
| | Nx | 6 (23%) |
| | Unknown | 8 (31%) |
| M Stage | M0 | 18 (69%) |
| | Unknown | 8 (31%) |

[Table 2]

| Age | | 67 (44~82) |
|---|---|---|
| Sex | Male | 23 (77%) |
| | Female | 7 (23%) |
| Smoking | Current/Former | 25 (83%) |
| | Never | 5 (17%) |
| Histology | ADC | 18 (60%) |
| | SQC | 10 (33%) |
| | NSCLC | 2 (7%) |
| Stage | 3A | 1 (3%) |
| | 3B | 2 (7%) |
| | 4A | 14 (47%) |
| | 4B | 13 (43%) |
| EGFR Mutation | Wild | 21 (70%) |
| | E19del | 2 (7%) |
| | L858R | 2 (7%) |
| | Unknown | 5 (16%) |
| PD-L1 expression on Tumor | >50% | 16 (53%) |
| | <50% | 13 (43%) |
| | Unknown | 1 (3%) |
| ICIs | Tecentriq | 15 (50%) |
| | Keytruda | 15 (50%) |

## 2. Sample Preparation

Tumor tissue was received directly from the operating room and processed immediately. The tissue was chopped into small pieces, and then digested with 0.5 mg/mL collagenase IV (Gibco) and 200 U DNase I (Roche) in a 37 °C magnetic stirrer. Tumor-infiltrating lymphocytes (TILs) were purified from the digested tissue using Percoll (Cytiva). Blood samples were harvested in BD Vacutainer (BD Biosciences). Peripheral blood mononuclear cells (PBMCs) were purified using Lymphoprep (Alere Technologies).

## 3. Flow cytometry

Cell suspensions of TILs or PBMCs were prepared and stained with the following antibodies (purchased from BioLegend, eBioscience and BD Biosciences) for fluorescence activated cell sorter (FACS) analysis: CD45RA (HI100), CD45RO (UCHL1), CD27 (LG.7F9), CD28 (CD28.2), CD57 (QA17A4), CD5 (L17F12), CD3 (HIT3a), CCR7 (G043H7), CD8 (SK1), CD279 (MIH4), perforin (BD48), granzyme B (GB11), IFNg (B27), IL2 (MQ1-17H12), Ki67 (Ki-67).

Flow cytometry samples were run using FACSCantoII (BD Biosciences) or CytoFLEX LX (Beckman Coulter) and analyzed by FlowJo software (Tree star).

#### 4. Intracellular staining

Cells were plated in a 96-well cell culture plate ($1\times10^6$ cells/well) and cultured with the eBioscience™ Cell Stimulation Cocktail (including a protein transport inhibitor) (Invitrogen) for 4 hours in a 37 °C $CO_2$ incubator. The cells were stained with a surface marker, fixed with BD Cytofix/Cytoperm buffer (BD Biosciences), and permeabilized. Then, the cells were stained for an indicated intracellular molecule and analyzed by flow cytometry. For Ki-67 staining, the eBioscience Foxp3/Transcription Factor Staining Buffer Set (eBioscience) was used with *ex vivo* samples.

#### 5. Relative telomere length

A relative telomere length was measured using a relative telomere-to-single copy ratio. Briefly, $1\times10^5$ cells were purified and used for DNA extraction using a DNA extraction kit (Bioneer). Quantitative PCR was performed by SYBR Green qPCR Master Mix (Maxima) using the following primers:

tel1 (SEQ. ID NO: 1):
GGTTTTTGAGGGTGAGGGTGAGGGTGAGGGTGAGGGT
tel2 (SEQ ID NO: 2):
TCCCGACTATCCCTATCCCTATCCCTATCCCTATCCCTA
36B4u (SEQ ID NO: 3):
CAGCAAGTGGGAAGGTGTAATCC
36B4d (SEQ ID NO: 4):
CCCATTCTATCATCAACGGGTACAA

#### 6. *Ex vivo* activation

Indicated cell types were purified using FACSAria™ III (BD Biosciences) and plated in an anti-CD3 and anti-CD28 coated Nunc MaxiSorp™ Flat-Bottom Plate (Invitrogen) or 96-well cell culture plate (SPL) ($5\times10^3$ cells/well). For some experiments, cells were labeled with 2.5 $\mu$M Cell Trace™ Violet (Invitrogen) (CTV) prior to stimulation. 0.5 ng/mL of IL-7 was applied to all wells to promote survival. Cells were stimulated with indicated stimulants for 7 days, and then stained and analyzed by flow cytometry. The degree of cell division was observed by measuring a CTV dilution index. A higher index value indicates that more cell division occurred.

#### 7. Bioinformatics

For RNA sequencing, $3\times10^5$ cells were purified and used for RNA extraction using TriZOL (Invitrogen). A library was constructed using the TruSeq RNA Sample Prep Kit (Illumina) and sequenced with an Illumina HiSeq2500 sequencer (Illumina) according to the manufacturer's protocols. PCA and hierarchical clustering were performed using the Factoextra R Package. tSNE analysis was performed using FlowJo Software (Tree star). FLOW-MAP was performed using the FLOWMAPR R package according to the developer's protocol. Briefly, CD3$^+$ CD8$^+$ cells were gated and exported using FlowJo. The exported fcs file was used for FLOW-MAP. Forcedirected layout (Force Atlas) was carried out with Gephi software. Gene set enrichment analysis (GSEA) was performed using a GSEA tool (Broad Institute).

#### 8. Statistics

To test statistical significance using Prism (GraphPad Software) shown in each drawing, a paired or un-paired two-tailed Student's t-test was carried out. Values of *$p<0.05$, **$p<0.01$, ***$p<0.001$, and ****$p<0.0001$ were considered significant.

[Examples]

#### <Example I> DP Temra (CD27$^+$ CD28$^+$ Temra) levels of cancer patients reflect an immunogenic or antigenic state of cancer patients against tumor antigens and responsiveness to cancer immunotherapy.

The responsiveness of cancer patients to cancer immunotherapy is dependent on the immunogenic or antigenic state of a tumor antigen and a patient's immunity state (particularly, toxic T cell response capacity). Therefore, a factor reflecting the tumor immunogenicity state of a cancer patient may be used as a biomarker for predicting the responsiveness to cancer immunotherapy and prognosis of a patient. Meanwhile, immune cells such as T cells are closely related to the degree of tumor progression, and the immunity and prognosis of a patient. Therefore, the present inventors specifically analyzed T cells to investigate a biomarker capable of being used as a marker for reflecting the tumor state of a cancer patient and a factor for predicting responsiveness to cancer immunotherapy.

**Example I-1. Temra with more differentiated characteristics than Tern is included in CD8+ TILs.**

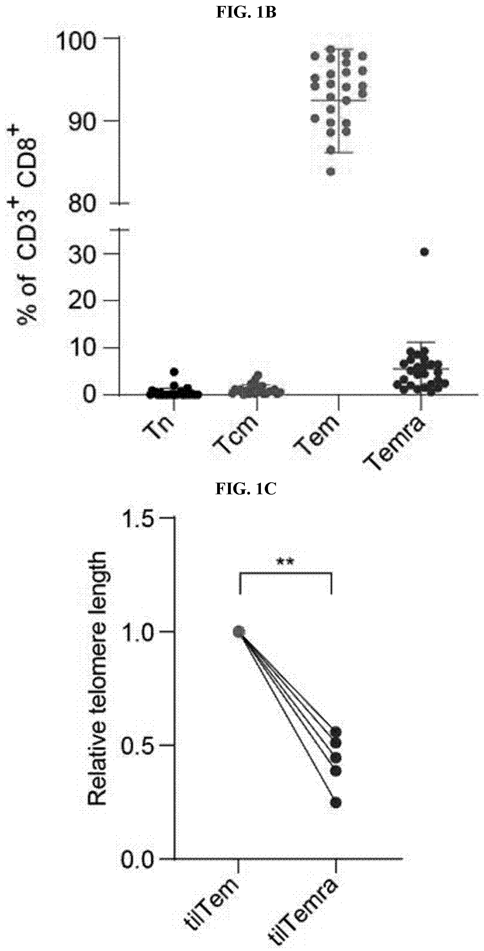

To examine heterogenous CD8+ TILs, PBMCs and TILs of 26 NSCLC patients were analyzed using a flow cytometer. The summary of the clinical information of the patients is shown in Table 1. CD8+ T cells were largely separated into four different subsets based on CCR7 and CD45RA: CCR7+CD45RA+ Tn, CCR7+CD45RA- Tern, CCR7-CD45RA- Teff/Tem (referred to as Tern), and CCR7-CD45RA+ Temra (FIGS. 1A and 7A). Unlike PBMCs, Tn and Tcm were not able to be detected in most TILs (average <1%). Instead, Tem accounted for the majority of TILs, ranging from 68.2 to 98.7% (average 92.29%) (FIGS. 1B and 7B). Temra was consistently found in TILs, accounting for 0.68 to 30.4 % (average 5.72%).

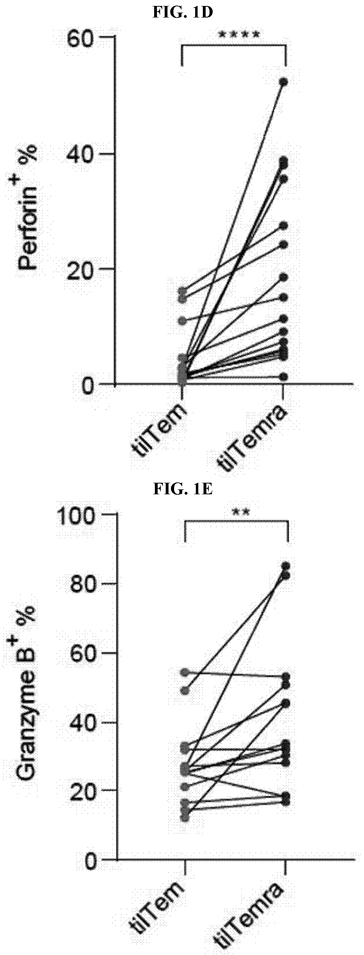

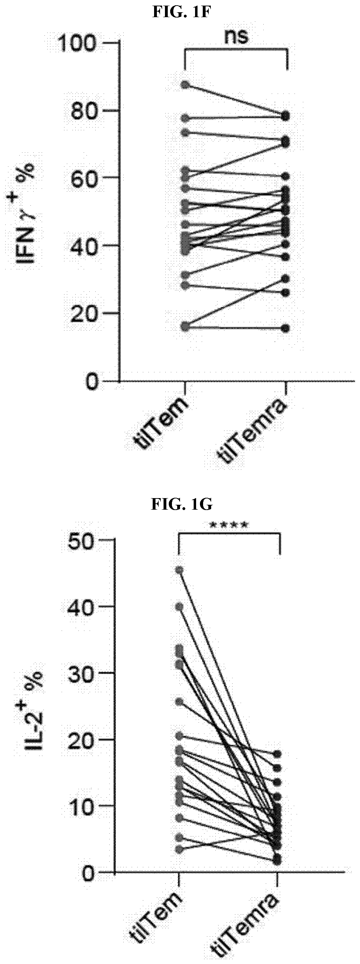

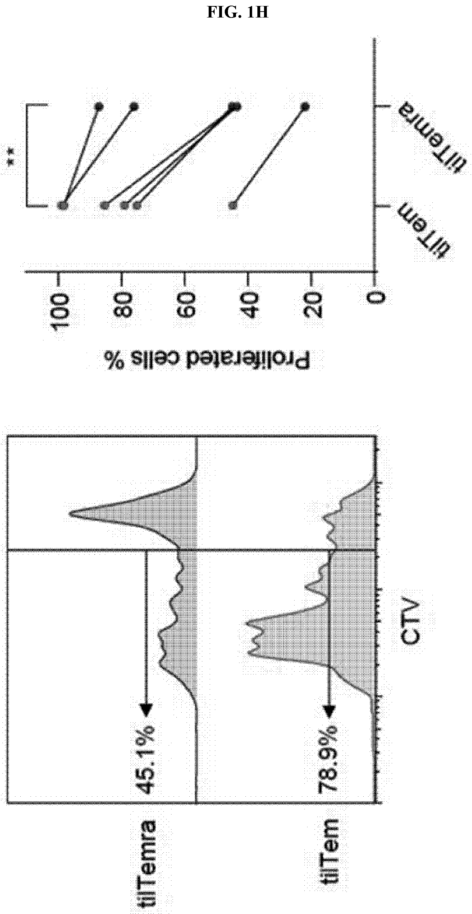

As demonstrated by a short telomere length and increased expression of perforin and granzyme B, Temra is considered the most differentiated subset of human CD8+ T cells. Since most characterizations of Temra were established with peripheral blood Temra (pTemra), the present inventors investigated whether tumor infiltrating Temra (tilTemra) exhibits the standard characterization of pTemra. Actually, tilTemra has a shorter telomere length than tilTem (FIG. 1C). In addition, tilTemra showed increased expression levels of perforin and granzyme B (FIGS. 1D and 1E), but interferon gamma (IFN-γ) production is similar (FIG. 1F). Unlike a cytolytic function, proliferation capacity decreases with T cell differentiation. Accordingly, tilTemra showed decreased interleukin-2 (IL-2) production compared with tilTem (FIG. 1G). A similar pattern was observed in their proliferation capacities upon stimulation with anti-CD3/CD28 and IL-2 (FIG. 1H). The above data suggests that tilTemra exhibits broadly similar characteristics to Temra defined in PBMCs (FIGS. 7C-7F).

**Example I-2. tilTemra has a unique transcriptome that is different from pTemra.**

Despite their overall similarity, the present inventors investigated whether tilTemra and pTemra are actually the same population distributed at spatially different locations. To this end, the gene expression profiles of FACS-purified tilTemra and pTemra of NSCLC patients (tilTemra_1 to 3, pTemra_1 to 3) were compared with pTemra of healthy donors (pTemra_4 to 6) through RNA sequencing (RNA-seq) analysis. Principal component analysis (PCA) showed that tilTemra was clearly separated from pTemra by the first principal component (PC1) (FIG. 2A). The difference in the second principal component (PC2) was significant in tilTemra, but not in pTemra. This seems to indicate that PC2 represents the heterogeneity of tilTemra by the differential contexts of each tumor microenvironment (TME). The present inventors confirmed the observation with hierarchical clustering, which groups tilTemra and pTemra into different clusters (FIG. 2B). The distance between samples represented as "height" was lower in pTemra and higher in tilTemra. This demonstrates that the heterogenicity of tilTemra is higher.

Next, differentially expressed genes (DEGs) between pTemra and tilTemra were analyzed. Among the analyzed 15,299 genes, 1,708 genes (11.16%) were differentially expressed (FIG. 2C). 910 genes (5.95%) were highly expressed in tilTemra (red dots), whereas 798 genes (5.22%) were highly expressed in pTemra (blue dots). Signature genes of differentiated cells such as TBX21, EOMES, PRF1, GZMB, and GZMH were upregulated in pTemra (FIG. 8A), whereas costimulatory molecules ICOS and CD28, which decreased in differentiated CD8+ T cells, were higher in tilTemra (FIG. 8B). It is shown that B3GAT1 and TOX, which are markers related to T cell senescence or exhaustion, were upregulated in pTemra (FIG.8C), and MYC and TCF7, which are markers indicating stem cell-like self-renewal capacity, were upregulated in tilTemra (FIG. 8D).

To further understand the functional differences between tilTemra and pTemra, pathway analysis was performed using gene set enrichment analysis (GSEA). The present inventors obtained several gene sets involved in differentiation, cytotoxicity, and proliferation from open resources (Gene Ontology and Broad Institute). The gene sets involved in differentiation showed that tilTemra was closer to a naive-like phenotype than pTemra (top of FIG. 2D, FIG. 8E). In addition, pTemra has enriched gene sets involved in cytotoxicity (middle of FIG. 2D, FIG. 8F), whereas tilTemra has enriched gene sets involved in proliferation (bottom of FIG. 2D, FIG. 8G). The above data shows that tilTemra has a unique transcriptome clearly distinguished from terminally differentiated pTemra, and has less exhausted (TBX21$^{lo}$TOX$^{lo}$PDCD1$^{lo}$), naive (and stem)-like (TCF7$^{hi}$MYC$^{hi}$) gene expression profiles.

**Example I-3. The phenotype and function of tilTemra are heterogeneous in terms of tumor progression.**

Since tilTemra was transcriptionally different from pTemra, the present inventors confirmed differences in functional characteristics. In agreement with previous findings, pTemra showed higher perforin, granzyme B and IFN-γ production, whereas tilTemra showed higher capacity in IL-2 production and proliferation (FIGS. 3A-3E; FIG. 9A). Similar patterns were observed between tilTem and pTem, with pTem showing higher perforin and granzyme B production (FIGS. 9B-9E).

According to the above functional data, tilTemra was confirmed to be functionally less differentiated than pTemra. The differentiation state of each subset may be further defined by the expression of costimulatory receptors, CD27 and CD28. While less differentiated cells show a double-positive (CD27⁺CD28⁺; DP) phenotype, more differentiated cells show a single-positive (CD27⁺CD28⁻ or CD27⁻CD28⁺; SP) or double-negative (CD27⁻CD28⁻; DN) phenotype. tilTemra has a higher proportion of the DP phenotype than pTemra (FIGS. 3F and 3G). Interestingly, the proportion of DP phenotype in tilTemra was always higher than that in pTemra. As shown from the equation "tilDP % - pDP %," the DP phenotype proportion in tilTemra was higher than 0 in every patient without exception (FIG. 3H). This indicates tumor-specific accumulation of DP Temra. Unlike Temra, Tern does not show such consistency (FIG. 3H, FIG. 9F), supporting the unique characteristics of DP Temra as well as the close relationship between tumor progression and DP Temra.

The present inventors further examined whether the functional differences between tilTemra and pTemra is caused by the differentiated proportion of DP cells in these two subsets. As expected, CD27⁺Temra showed lower perforin production and higher IL-2 production than those of CD27⁻ counterparts derived from the same origin (TILs or PBMCs) (FIGS. 3I and 3J). Together with transcriptional analyses in FIGS. 2A-2D, the above data suggests that tilTemra and pTemra are populations with heterogeneous phenotypes and functions, and tilTemra (particularly, DP) shows much less differentiated functional characteristics.

**Example I-4. CD8⁺ T cells show bifurcated trajectory in differentiation into Temra.**

To further understand the differentiation and lineage relationship between DN and DP Temra, by applying a t-distributed stochastic neighbor embedding (tSNE) method, the clustering of phenotypically similar cells in flow cytometry data was visualized. Interestingly, tilTemra clustered into two different populations: (1) CD27loCD28loCD57hi and (2) CD27hiCD28hiCD57lo (FIG. 4A; FIG. 10A). Such bifurcation of tilTemra was consistently observed in different NSCLC patients (FIG. 4B; FIG. 10B), which suggests that there is a complex interaction between Temra differentiation and tumor progression.

Next, the present inventors attempted to define the differentiation trajectory into DP Temra utilizing the recently published FLOW MAP. FLOW-MAP clusters phenotypically similar cells into 'nodes' and connects similar nodes with 'edges,' generating a trajectory of nodes (left, FIG. 4C). The present inventors labeled each node based on the expression of CD45RA, CD45RO, CD27, and CD28 (right, FIG. 4C; FIG. 10C). The present inventors observed a transition from the CD45RO⁺ Temra nodes (left) to CD45RA⁺ nodes (right), but the transition followed a bifurcated trajectory (FIG. 4D): (1) DP Tem → SP Tem → DN Tem → DN Temra (blue arrow) and (2) DP Tem → DP Temra (red arrow). These results strongly suggest that DP tilTemra is generated directly from DP tilTem without the transition state of SP or DN tilTem. In line with this idea, a high correlation between the proportions of DP tilTem and DP tilTemra was observed (FIG. 4E).

**Example I-5. The strength of TCR engagement determines Temra differentiation.**

Next, the present inventors confirmed the basic mechanism of divergent differentiation into the above-described DN or DP tilTemra. The key requirement for this bifurcation is either timely upregulation or downregulation of CD27 and CD28 while re-expressing CD45RA, which is a hallmark of Temra, during tumor progression. Thus, the present inventors confirmed the role of antigenic stimulation by TCR engagement and the role of cytokines produced by various cell types in the tumor microenvironment (TME). To this end, the present inventors purified tilTem by FACS and cultured tilTem with various doses of anti-CD3/CD28 or various cytokines. Unlike Tn (FIG. 11A), tilTem considerably downregulated CD28 upon anti-CD3/CD28 stimulation (left, FIG. 5A). However, CD27 expression in tilTem was moderately reduced only at a high concentration of anti-CD3, but not significantly changed (left, FIG. 5B).

Contrast to the TCR results, the present inventors found that, among various cytokines tested *in vitro,* IL-2 induced substantial downregulation of CD27 (FIG. 5B, FIG. 11B), but did not induce CD28 reduction (right, FIG. 5A, FIG. 11C). In line with this finding, the proportion of CD27-expressing (CD27⁺) tilTem showed a significant inverse correlation with the proportion of total IL-2-producing (IL-2⁺) CD3⁺ T cells (FIG. 5C). Since IL-2 is mainly produced by activated CD3⁺ T cells (CD4⁺ and CD8⁺) after TCR stimulation (FIG. 11D), it seems that TCR stimulation modulates CD27 and CD28 expression directly or indirectly (via IL-2). Therefore, the above data suggests that strong TCR engagement distinctly promotes the downregulation of CD27 and CD28 and thus induces DN Temra differentiation but inhibits DP Temra differentiation.

Considering the role of TCR in the regulation of CD27 and CD28 expression, the present inventors investigated the influence of TCR on CD45RA expression, which is upregulated during Temra differentiation. The expression level of CD45RA was very low in tilTem but more clearly downregulated after treatment with anti-CD3/CD28 stimulation (left, FIG. 5D). Similar downregulation of CD45RA was also observed in Temra (FIG. 11E), suggesting that strong antigenic stimulation has to be avoided for upregulation of CD45RA during the transition of tilTem to tilTemra. Unlike TCR engagement, IL-2 treatment induced the upregulation of CD45RA expression. However, for upregulation, a large amount of IL-2 was needed (right, FIG. 5D). This result suggests that IL-2 is involved in regulating the differentiation of Temra cells.

Due to long-term repeated proliferation, Temra has a short telomere length (FIG. 1C). Accordingly, the present inventors examined whether tilTem cells can actually proliferate consistently in response to TCR stimulation. Interestingly, tilTem did not properly proliferate upon anti-CD3/CD28 engagement (left, FIG. 5E, FIG. 11F) but significantly proliferated upon IL-2 exposure (right, FIG. 5E, FIG. 11G). Even by treatment with IL-2 alone, prominent proliferation was achieved, and there was little effect by additional TCR engagement (FIG. 5F). Together with the previous reports showing low responsiveness of Tem cells to TCR stimulation, these results suggest that TCR signaling is unnecessary during tilTemra differentiation and is able to be timely suppressed (by an active mechanism such as TCR desensitization).

Supporting this idea, the re-expression of CD45RA in tilTem was much more pronounced in CD27$^-$ and CD28$^-$ cells (that is, cells lacking costimulatory molecules for TCR signaling). Similarly, the expression level of CD45RA significantly increased as tilTem cells differentiated from DP to a SP or DN state (FIG. 5H). Similar results were also observed in pTem (FIGS. 11H and11I). The above data strongly suggests that a negligible or low level of TCR engagement (in conjunction with the loss of costimulatory molecules) promotes the re-expression of CD45RA in Tem (or Teff) cells and the transition to DN or DP Temra.

**Example 1-6. The proportion of DP Temra is inversely correlated with the number of CD8$^+$ TILs and tumor mutation burden (TMB).**

The main implication of the above experimental results is that strong and repeated antigen stimulation through TCR binding with a tumor antigen has a negative effect on Temra (particularly, DP Temra) differentiation. Such result is consistent with previous reports confirming that CD27$^-$CD28$^-$DN Temra is more widely prevalent in chronically-infected viral antigen-specific CD8$^+$ T cells. Accordingly, the question of how antigen-specific CD8$^+$ Tem (or Teff) can differentiate into DP Temra rather than DN Temra arises. Therefore, the present inventors presumed that, provided that high doses of anti-CD3/CD28 treatment downregulated CD27 and CD28 (FIGS. 5A and 5B), lowering TCR stimulation below a level that does not trigger a DP-to-DN transition would be crucial for maintaining constantly high levels of CD27 and CD28 costimulatory molecules throughout Temra differentiation during tumor progression.

Tumor antigens such as tumor-associated antigens (TAAs) and neo-antigens (associated with tumor mutation burden (TMB)) vary in their stimulatory capacities for antigen-specific CD8$^+$ Tn cells and CD8$^+$ TILs, just as TCR engagement can induce T cell expansion to various extents according to its strength (FIG. 12). Accordingly, the present inventors speculated that TAAs and neo-antigens may have different path choices between DN and DP Temra differentiation. Particularly, the present inventors noted that there is an inverse correlation between the number of CD8$^+$ TILs in NSCLC patients and the proportions of DP tilTem (FIG. 6A) and DP tilTemra (FIG. 6B). Therefore, the above result strongly supports the idea of the present inventors that low immunogenic stimulation using a tumor antigen (e.g., TAAs) induces continuous expression of CD27 and CD28 in tumor-specific CD8$^+$ T cells and promotes the differentiation pathway into DP Temra rather than DN Temra.

Interestingly, the present inventors also observed a strong correlation between the proportions of DP pTemra and DP tilTemra (FIG. 6C), suggesting that these two subsets can share some extent of specificity for a given tumor antigen and circulate between a tumor and peripheral blood. In addition, it was confirmed that the proportion of DP pTemra is inversely correlated with the number of CD8$^+$ TILs (FIG. 6D). Accordingly, patients with a relatively low proportion of DP pTemra (<6%) showed twice as many CD8$^+$ TILs as patients with a high proportion of DP pTemra (>6%), thus the number of CD8$^+$ TILs can be easily estimated by analyzing DP Temra in the blood of NSCLC patients.

To investigate the relationship more specifically between DP tilTemra and DP pTemra, TCR $\alpha/\beta$ usages in CD8$^+$ T cell subsets (CD27$^+$/CD27-Tem and CD27$^+$/CD27-Temra) in PBMCs and TILs of six NSCLC patients were examined. Among the subsets, CD27$^+$ pTemra and CD27$^+$ tilTemra cells appeared to share the most similar degrees of TCR V$\alpha$7.2, Y$\beta$8, V$\beta$12 chain usages (red box, FIGS. 12B-D). Particularly, CD27$^+$ tilTemra and CD27$^-$ tilTemra cells appeared to have a distinct difference in degrees of TCR V$\alpha$7.2, V$\beta$8, V$\beta$12 chain usages (blue box, FIGS. 12B to 12D), suggesting almost no relationship between the subsets. For further verification, TCR $\alpha/\beta$ usages (Va2, V$\alpha$7.2, V$\alpha$12.1, V$\beta$3, V$\beta$5b, V$\beta$8, V$\beta$12, and V$\beta$13.1) of PBMC- or TIL-derived CD27$^+$/CD27$^-$ Tem and CD27$^+$/CD27$^-$ Temra cells were analyzed, the similarity between the 8 TCR $\alpha/\beta$ usages was calculated using a sample distance matrix (DESeq2). As a result, it was confirmed that CD27$^+$ tilTemra and CD27$^+$ pTemra cells were the closest (red line, FIG. 12E), while CD27$^-$ tilTemra cells were the farthest (blue line).

To further confirm the strong inverse correlation between the proportion of DP pTemra and the number of CD8$^+$ TILs together with the degree of tumor immunogenicity (TMB load), PBMCs were newly collected from 30 NSCLC patients (Table 2) and divided into two groups according to the proportion of DP pTemra. The population with a low proportion of DP pTemra (DP pTemra % < 6%) showed increased levels of Ki-67$^+$ CD8$^+$ T cells (FIG. 12F) and PD-1$^+$ (FIG. 12G) CD8$^+$ T cells, compared with the population with a high proportion of DP pTemra (DP pTemra % > 6%), indicating that a stronger immune response is underway.

Next, to confirm whether the observed difference between the two groups is actually related with the degree of tumor immunogenicity, the clinical information of the patients was sorted based on a well-known TMB load. First, it is known that squamous cell carcinoma (SCC) has a higher TMB than non-SCC, and consistent with this, 20% of SCC patients (TMB: 9) were classified as a high-DP pTemra group, and 45% of non-SCC patients (TMB: 6) were classified as a high-DP pTemra group (FIG. 6F; FIG. 12H). Likewise, smoking history is also known to be a critical factor for NSCLC TMB, and while only 24% of smokers (TMB: 11) were classified as a high-pTemra group, all non-smokers (TMB: 0.6) were included in a high-DP pTemra (FIG. 6G; FIG. 12I).

The number of CD8+ TILs and TMB are most widely used biomarkers for cancer immunotherapy. Since patients with a high CD8+ TIL or TMB level are known to have high responsiveness to immune checkpoint inhibitors (ICIs; e.g., anti-PD-1 or anti-PD-L1), the present inventors speculated that patients with a lower proportion of DP pTemra cells exhibited higher responsiveness to ICIs. For verification, the patients were treated with an ICI (ani-PD-1/PD-L1) after blood analysis (FIGS. 6F and 6G), the responsiveness of the patients to the ICI was monitored, and the clinical result was compared with the proportion of DP pTemra obtained before treatment. As a result, while only 20% of the patients with partial response (PR) were classified as a high-DP pTemra group (high proportion of DP pTemra cells), each of 36.4% and 42.9% of the patients with steady stable disease (SD) and progressive disease (PD) correspond to the high-DP pTemra group (FIG. 6H).

These results demonstrate that the proportion of DP pTemra is inversely correlated with the degree of immunogenicity of tumor antigens (i.e., TMB) and the number of CD8+TILs, and can be utilized as a marker for predicting the responsiveness of cancer patients to immunotherapy.

**<Example II> Fitness factors (DP/DN Tem%, perforin+ Tem%, granzyme B+ Tem%) of cancer patients reflect the immunity and the responsiveness of the patients to cancer immunotherapy.**

Through Example I, it was confirmed that DP pTemra has an inverse correlation with tumor mutation burden (TMB) and the number of tumor-infiltrating CD8 T cells, and thus can be used as a marker reflecting the immunogenic and antigenic states of patients to tumor antigens, and a marker for predicting the responsiveness of patients to cancer immunotherapy.

In the following examples, to discover biomarkers (Fitness factors) that can exhibit a patient's immunity (i.e., the ability to respond to immunotherapy such as PD-1 treatment), which gradually weakens with tumor progression, the characteristics of immune cells according to tumor progression were analyzed, and factors correlated with responsiveness to immunotherapy were explored.

**Example II-1. Tumors promote the differentiation of CD8 T cells.**

To discover biomarkers reflecting the immunity of a patient and resulting responsiveness to cancer immunotherapy, the immunological differences between NSCLC cancer patients and healthy subjects were first identified. For this, PBMCs were obtained from 33 NSCLC cancer patients and 25 healthy subjects, and the proportion of each subset (Tn, Tcm, Tern, and Temra) in CD8 T cells (CD8+CD3+ T cells) was measured through flow cytometry.

As a result, as shown in FIG. 13, the cancer patients showed a lower proportion of Tn cells and higher proportions of Tem and Temra cells compared to those of the healthy subjects. Tn cells are relatively less differentiated cells, whereas Tem and Temra are relatively more differentiated cells (i.e., divide in the order of Tn → Tcm → Tem → Temra). Accordingly, this result shows that CD8 T cell subsets of the cancer patients are overall shifted to more differentiated cells, indicating that, as confirmed in the previous example, tumors promote the differentiation of CD8 T cells.

**Example II-2. As the differentiation of CD8 T cells progresses, cytotoxicity increases but division capacity decreases.**

Subsequently, functions (division capacity and cytotoxicity) of each subset of CD8 T cells in cancer patients were observed. Specifically, PBMCs obtained from 69 cancer patients were restimulated with PMA/ionomycin for 4 hours (GolgiStop was also added) to induce the secretion of cytokines. Subsequently, the proportions of cells secreting perforin, granzyme B, IFN-γ, and IL-2 in each CD8 T cell subset were examined through flow cytometry.

The results are shown in FIG. 14. As can be confirmed in the drawing, it is shown that perforin, granzyme B, and IFN-γ are secreted in more differentiated cells at a higher level, whereas IL-2 is more highly secreted in less differentiated cells.

Perforin and granzyme B are cytotoxic molecules that are used when CD8 T cells attack cancer cells, and IFN-γ is a cytokine that activates immune cells to create an environment in which cancer cell attacks are more effective. IL-2 is a cytokine promoting T cell division. Thus, the above result suggests that, as CD8 T cells differentiate, cytotoxicity increases but division capacity decreases.

**Example II-3. The increase in perforin and granzyme B in Tem cells is a cancer patient-specific immunological characteristic.**

Based on the finding that, as the differentiation of CD8 T cells progresses, cytotoxicity increases but division capacity decreases, it was confirmed whether there were differences in function between T cells of cancer patients and healthy subjects.

Accordingly, PBMCs obtained from either 33 NSCLC patients or 25 healthy subjects were restimulated with PMA/ionomycin for 4 hours (GolgiStop was also added) to induce the secretion of cytokines, and the proportions of cells secreting perforin, granzyme B, IFN-γ, and IL-2 in Tem cell populations among subsets of CD8 T cells were confirmed through flow cytometry.

As result, as shown in FIG. 15, there was no significant difference in proportions of IFN-γ secreting cells and IL-2 secreting cells between Tem cells of healthy subjects and cancer patients, but the proportions of perforin secreting cells and granzyme B secreting cells are higher in Tem cells of cancer patients. As seen from the previous example, perforin and granzyme B are more highly expressed in more differentiated cells. Accordingly, the above result that the proportion of perforin or granzyme B secreting cells increases in cancer patients suggests that Tem of cancer patients is more differentiated than Tem of healthy subjects. That is, it can be seen that the increase in perforin and granzyme B in Tem cells is a cancer patient-specific immunological characteristic.

**Example II-4. Cancer immunotherapy increases perforin- or granzyme B-expressing Tern in cancer patients.**

Since the cancer patient-specific immunological characteristic (the increase in perforin- or granzyme B-expressing Tem cells) was confirmed, in the next step, the immunological change that occurred when cancer patients received cancer immunotherapy (PD-1 therapy) was observed.

Specifically, blood (Before) before a cancer patient was subjected to PD-1 therapy (Keytruda or Tecentriq) and blood (After) 7 to 14 days after a cancer patient was subjected to PD-1 therapy were obtained (total 42 sets), PBMCs obtained from each blood were restimulated with PMA/ionomycin for 4 hours (GolgiStop was also added), and then the proportions of cells secreting perforin, granzyme B, IFN-γ, and IL-2 in a Tem cell population among the subsets of CD8 T cells were confirmed through flow cytometry.

As a result, as shown in FIG. 16, it was found that the perforin and granzyme B expression levels in Tem of the cancer patients further increase after receiving the PD-1 therapy compared with before receiving the PD-1 therapy. In the case of INF-γ secreting cells, there was no significant difference before and after receiving the PD-1 therapy, and in the case of IL-2 secreting cells, the proportion was somewhat decreased after receiving the PD-1 therapy.

Taking the above examples together, there was a difference in expression levels of perforin and granzyme B in Tem between cancer patients and healthy subjects (Example II-3), and there was a difference in production of perforin and granzyme B in Tem before and after cancer immunotherapy in cancer patients (Example II-4). These results demonstrate that there is a significant correlation between tumor progression and the expression level(s) of perforin and/or granzyme B. However, after cancer immunotherapy, although it was confirmed that perforin and granzyme B overall increased, specifically, since there was a mixture of patients with increased levels of perforin and granzyme B and patients without an increase after cancer immunotherapy, additional analysis was conducted.

**Example II-5. The increase in perforin and granzyme B production in Tern is correlated with the responsiveness of a cancer patient to cancer immunotherapy.**

As described above, since the increase in perforin and granzyme B after cancer immunotherapy was confirmed in several cancer patients, but patients with decreased levels of perforin and granzyme B after cancer immunotherapy were also found, it was confirmed whether there was a difference in responsiveness to PD-1 therapy between patients with increased perforin/granzyme B and patients with no increase.

Specifically, the patients with significant increased levels of perforin and granzyme B or no increase were classified into separate groups, and the proportion of patients with a partial response (PR) to cancer immunotherapy in each group was measured.

As a result, as shown in FIG. 17, it was confirmed that a patient with increased perforin or granzyme B expression has higher responsiveness to PD-1 therapy. This result demonstrates that the increase in perforin and granzyme B production in Tern is correlated with a cancer patient's responsiveness to cancer immunotherapy.

**Example II-6. The initial levels of perforin and granzyme B before anticancer treatment determine responsiveness to cancer immunotherapy.**

Since it was confirmed through the previous example that the increase in perforin and granzyme B is correlated with a cancer patient's responsiveness to cancer immunotherapy, the present inventors identified which patients had the characteristic of increased levels of perforin and granzyme B.

Specifically, patients with significantly increased levels of perforin and granzyme B (i.e., patients with higher responsiveness to PD-1 therapy) and patients with no increase (i.e., patients with lower responsiveness to PD-1 therapy) were divided into separate groups, and the perforin and granzyme B levels in blood samples before cancer immunotherapy (Before) for each group were confirmed.

As a result, as shown in FIG. 18, it was confirmed that the groups of patients with increased levels of perforin and granzyme B after cancer immunotherapy (Perforin increased and Granzyme B increased) exhibited lower levels of perforin and granzyme B than the groups of patients with unchanged levels of perforin/granzyme B (Perforin NOT increased and Granzyme B NOT increased) prior to cancer immunotherapy.

Looking at the above results, it seemed that the patients with low initial levels of perforin and granzyme B before anticancer treatment may have increased levels of perforin and granzyme B in response to cancer immunotherapy, whereas the patients with higher initial levels of perforin and granzyme B before anticancer treatment cannot have further increased levels of perforin and granzyme B even after cancer immunotherapy. In addition, such differences appear to eventually lead to responsiveness to cancer immunotherapy.

**Example II-7. The expression levels of perforin and granzyme B are increased in more differentiated Tern.**

Since the above example demonstrated that the levels of perforin and granzyme B initially expressed in Tem determine the responsiveness of cancer patients to cancer immunotherapy, the present inventors attempted to determine the cause of the difference in expression of perforin and granzyme B by Tem among cancer patients.

Specifically, after restimulating PBMCs derived from 69 cancer patients with PMA/ionomycin for four hours (GolgiStop was also added), the proportion of cells secreting perforin, granzyme B, INF-$\gamma$, or IL-2 was confirmed in each subset of Tem through flow cytometry. In addition, for qualitative analysis, the MFI of each molecule was measured in cells expressing the molecule. The subsets of Tem are classified depending on expression patterns of CD27 and CD28 as follows: DP Tem (CD27 and CD28 double-positive; CD27$^+$ CD28$^+$ CCR7$^-$ CD45RA$^-$), SP Tem (CD27 or CD28 single positive; CD27$^+$ CD28$^-$ CCR7$^-$ CD45RA$^-$ or CD27$^-$ CD28$^+$ CCR7$^-$ CD45RA$^-$), and DN Tem (CD27 and CD28 double-negative; CD27$^-$ CD28$^-$ CCR7$^-$ CD45RA$^-$).

The results are shown in FIGS. 19A and 19B. When Tem was divided into the three subgroups as above, the proportion of perforin or granzyme B-expressing cells increased in the order of DP, SP, and DN (i.e., DN>SP>DP Tern). In addition, the perforin MFI value and granzyme B MFI value also increased in the same order as above (DN>SP>DP Tern). However, the proportion of IL-2 expressing cells decreased in the order of DP, SP, and DN (i.e., DP>SP>DN Tern). There was no significant difference in IFN-$\gamma$ among subgroups.

Tem differentiation occurs in the order of DP, SP, and DN. That is, the above result suggests that, in the Tem group, as T cell differentiation progresses further, the expression of perforin and granzyme B is increased, and the expression of IL-2 is decreased, such a pattern is consistent with the previous tendency observed with CD8 T cells. Accordingly, depending on the distribution of DP, SP, and DN Tem in the Tem population, the expression levels of perforin and granzyme B are changed in total Tern, and it is believed that this eventually determines responsiveness to cancer immunotherapy.

**Example II-8. The initial levels of perforin and granzyme B of cancer patients are determined according to the distribution pattern of Tem subsets (DP and DN).**

Through the above example, it was confirmed that DP Tern, which is the least differentiated among Tem subsets, expresses the least perforin/granzyme B, and DN Tern, which is the most differentiated among Tem subsets, expresses the most perforin/granzyme B. Therefore, it was confirmed whether the differences in expression levels of perforin and granzyme B among cancer patients were due to differences in distribution of DP, SP, and DN Tem subsets in Tern.

For this, it was confirmed that perforin and granzyme B expression levels in total Tem derived from PBMCs of 69 NSCLC patients are consistent with the proportions of DP Tem and DN Temm among total Tem cells.

As a result, as shown in FIGS. 20A and 20B, among total Tem cells, as the proportion of DP Tem increases (FIG. 20A) and the proportion of DN Tem decreases (FIG. 20B), the expression levels of perforin and granzyme B increase.

The above result suggests that the difference in expression level of perforin and granzyme B among cancer patients is due to the difference in distribution of DP and DN Tem among total Tem cells.

**Example II-9. The tendency for perforin and granzyme B to increase by cancer immunotherapy is determined according to the distribution pattern of Tem subsets (DP and DN).**

Through a previous example, it was confirmed that patients with low initial perforin and granzyme B levels before cancer immunotherapy showed a tendency for perforin and granzyme B to increase after cancer immunotherapy and higher responsiveness to cancer immunotherapy (Example II-5). In addition, through a previous example, it was confirmed that the initial levels of perforin and granzyme B were determined by the distribution of DP/DN Tem among total Tem cells (Example II-8). Therefore, it was confirmed whether the distribution of DP/DN Tem among total Tem cells affects the tendency for perforin or granzyme B to increase by cancer immunotherapy.

Specifically, cancer patients were divided into the following four groups according to the proportions of DP Tem and DN Tern, and perforin or granzyme B levels before and after cancer immunotherapy (PD-1 therapy) were confirmed for each group: a group with a high DP Tem proportion (high-DP Tem %); a group with a low DP Tem proportion (low-DP Tem %); a group with a high DN Tem proportion (high-DN Tem %); and a group with a low DN Tem proportion (low-DN Tem %).

As a result, referring to FIGS. 21A and 12B, it was confirmed that the patients with a higher DP Tem proportion, or the patients with a lower DN Tem proportion have increased levels of perforin and granzyme B after cancer immunotherapy.

This result suggests that the distribution of DP/DN Tem among total Tem determines whether perforin and granzyme B are increased by cancer immunotherapy. In addition, such a result demonstrates that the distribution of DP/DN Tem among the total Tem of cancer patients determines responsiveness to cancer immunotherapy.

**Example II-10. The distribution patterns of Tem subsets (DP and DN) determine the responsiveness to cancer immunotherapy and prognosis of cancer patients.**

Through the previous example, it was confirmed that the increase in perforin/granzyme B by cancer immunotherapy is associated with the responsiveness of cancer patients to cancer immunotherapy, and the distribution of DP/DN Tem among all Tem also determines whether perforin/granzyme B is increased by cancer immunotherapy. Therefore, the present inventors confirmed whether the responsiveness of cancer patients to cancer immunotherapy is actually changed according to the DP and DN Tem distribution.

Specifically, the patients were divided into four groups according to the proportions of DP Tem and DN Tem among all Tern: a group with a high DP Tem proportion (high-DP Tem %); a group with a low DP Tem proportion (low-DP Tem %); a group with a high DN Tem proportion (high-DN Tem %); and a group with a low DN Tem proportion (low-DN Tem %). The Ki-67 expression levels in Tem before (Before) and after (After) cancer immunotherapy (PD-1 therapy) were compared for each group, and the proportion of patients with a partial response (PR) to cancer immunotherapy was also confirmed for each group.

As a result, it was confirmed that the patients with a higher DP Tem proportion or a lower DN Tem proportion have increased Ki-67 expression after cancer immunotherapy (FIG. 22A). Ki-67 is a molecule expressed in dividing cells, and it is known that, among the cancer patients receiving PD-1 therapy, patients in which increased Ki-67 expression is observed have a better prognosis. Therefore, the above result show that the patients with a higher DP Tem proportion or a lower DN Tem proportion have higher responsiveness to cancer immunotherapy (more cell division occurs) and a better prognosis. In fact, as a result of confirming the proportion of patients showing a partial response to cancer immunotherapy, it was confirmed that the proportion of patients showing a partial response to cancer immunotherapy was higher in patients with a higher DP Tem proportion or a lower DN Tem proportion (FIG. 22B). Overall, the above results demonstrate that the distribution of the distribution of DP and DN Tem among total Tem cells in cancer patients determines the patients' responsiveness to cancer immunotherapy and prognosis.

**Example II-11. The distribution of Tern subsets (DP and DN) was determined by the presence or absence of tumors, not age.**

Through the above example, it was confirmed that the proportion of DP/DN Tem in Tem of cancer patients determines the responsiveness to cancer immunotherapy and prognosis. Therefore, the present inventors attempted to examine the factors that change the distribution of DP/DN Tem in cancer patients. Particularly, Tem is differentiated in the order of DP, SP, and DN, and as a person gets older, the person receives more immune stimulation, so the proportion of differentiated cells is higher. Therefore, it was confirmed whether the distribution of DP/DN Tem is changed depending on the age of a patient.

First, NSCLC stage III and IV patients (Stage III, IV) were grouped into a 40s/50s group; a 60s group; and a 70/80s group according to age, and the proportions of DP Tem and DN Tem among all Tem for each group were confirmed by flow cytometry. In addition, the DP Tem and DN Tem proportions of subjects in their 40s/50s among healthy people and subjects in their 40s/50s among the NSCLC stage III and IV patients were compared.

First, as a result of confirming the DP/DN Tem distribution by age group, it was confirmed that there is no significant difference in DP/DN Tem distribution according to age in the NSCLC stage III and IV patients (FIG. 23A). However, as a result of comparing the DP/DN Tem distribution between healthy subjects and cancer patients in the same age group (40s/50s), it was confirmed that, in cancer patients, the DP Tem proportion is lower and the DN Tem proportion is higher than those of healthy subjects (FIG. 23B).

The above results show that the distribution of DP/DN Tem among total Tem cells is affected by the presence or absence of tumors, not by age.

**Example II-12. The proportion of DP Tem decreases with tumor progression.**

Through the above example, since it was confirmed that the distribution of DP/DN Tem was determined by the presence or absence of tumors, it was confirmed whether the distribution of DP/DN Tem is changed according to the degree of tumor progression.

Specifically, the proportions of DP Tem and DN Temn among total Tem in healthy subjects and NSCLC patients (Stage I/II, Stage III, and Stage IV) were confirmed by flow cytometry.

As a result, it was confirmed that, in the overall NSCLC patient group, the mean DP Tem proportion was lower than that of the healthy group, and the group in which a tumor further progressed, i.e., the group with a higher stage among the NSCLC patient groups had a lower DP Tem proportion (FIG. 24). In addition, it was confirmed that, in the overall NSCLC patient group, the mean DN Tem proportion was higher than that of the healthy group, and the group in which a tumor further progressed, i.e., the group with a higher stage had an increased DN Tem proportion. The results suggest that as the tumor progresses, the distribution of DP and DN Tem is changed, and as the stage increases, the DP Tem proportion increases and the DN Tem proportion decreases.

Through a previous example, the present inventors confirmed that the higher the DP Tem proportion and the lower the DN Tem proportion, the higher the responsiveness to cancer immunotherapy (Example II-10). This indicates that the more of the less-differentiated cells (DP Tern) there are, the more likely the tumor is to be attacked in response to cancer immunotherapy, whereas the more differentiated cells (DN Tern) there are, the lower the percentage of cells that can respond to cancer immunotherapy, which means the likelihood of the tumor being attacked is reduced. Here, the DN Tem proportion increases as tumors progress suggests that T cells are further differentiated in continuous response to tumors as the interaction between cancer cells and T cells increases. That is, as the interaction between cancer cells and T cells continues, the DP Tem proportion decreases and thus the responsiveness to cancer immunotherapy decreases, so it can be determined that it is important to receive cancer immunotherapy when the immunity remains (i.e., when the DP Tem proportion is high).

**<Example III> Prediction of cancer immunotherapy responsiveness in cancer patient using dual markers (DP Temra and Fitness factors)**

Through Example 1, it was confirmed that DP pTemra has an inverse correlation with tumor mutation burden (TMB) and the number of tumor-infiltrating CD8 T cells. Therefore, it is a marker that reflects the immunogenicity and antigenicity of tumor antigens of patients and can be used as an independent biomarker for predicting the responsiveness of patients to cancer immunotherapy.

In addition, through Example II, it was confirmed that as DP Tem% is higher; DN Tem% is lower; perforin[+] Tem% increases after cancer immunotherapy; granzyme B[+] Tem% increases after cancer immunotherapy; and IL-2[+] Tem% decreases after cancer immunotherapy (overall, expressed that 'Fitness is high'), the immunity of cancer patients (capability to respond to cancer immunotherapy) is advantageous for anticancer treatment, and therefore, it was confirmed that the Fitness factor can be used as an independent biomarker for predicting the responsiveness of patients to cancer immunotherapy.

In order for cancer patients to show high responsiveness to cancer immunotherapy, both the cancer immunogenicity and a patient's anticancer immunocompetency have to be good. Therefore, in the following example, the present inventors attempted to establish a model that classifies patients in a 2D graph using DP Temra representing a cancer immunogenic state and a Fitness factor representing a patient's anticancer immunity, and estimates the probability of a partial response (PR) of a cancer patient to cancer immunotherapy by K-NN algorithm-based machine learning. The advantage of K-NN machine learning is that there is no missing patient at the threshold boundary, and it is possible to provide patient-customized treatment by giving different probability values, from a low probability to a high probability, for each patient.

**Example III-1. The capacity of a dual marker of DP Temra and a Fitness factor (DP Tern or DN Tern) to predict the responsiveness to cancer immunotherapy is superior to that of a single marker.**

First, the present inventors attempted to increase the capacity to predict the responsiveness of a cancer patient to immunotherapy (an immune checkpoint inhibitor) using both DP Temra and a Fitness factor (DP Tem% or DN Tem%). As confirmed in the previous example, for a cancer patient to show high responsiveness to cancer immunotherapy, a DP Temra level is low (i.e., a cancer state is a state in which TMB is high and the number of tilCD8 is large), the patient's immunity (i.e., Fitness) has to be good (DP Tem% is high and DN Tem% is low). Accordingly, when a 2D graph was made with DP Temra and DP Tern as the x axis and the y axis, it was expected that patients with low DP Temra and high DP Tem show high responsiveness to immunotherapy (an immune checkpoint inhibitor); and in the 2D graph created with DN Temra and DN Tem as the x axis and the y axis, it was expected that patients with low DP Temra and low DN Tem show high responsiveness to an immunotherapy.

Specifically, DP Temra%, DP Tem%, and DN Tem% in PBMCs of 120 NSCLC Stage III/IV patients were confirmed by flow cytometry. Afterward, first, a graph was drawn using DP Temra on the x axis and DP Tem on the y axis, and patients were classified into Q1, Q2, Q3, and Q4 groups based on the two values. In the same manner, a graph was drawn using DP Temra on the x axis and DN Tem on the y axis, and patients were classified into Q1, Q2, Q3, and Q4 groups based on the two values. Finally, the proportion of patients with a partial response (PR-patient proportion) to cancer immunotherapy (PD-1 therapy) was confirmed for each of the Q1 to Q4 groups.

The 2D graph using DP Temra and DP/DN Tem is shown in FIG. 25A, and the proportion of patients with a partial response for each of the Q1, Q2, Q3, and Q4 groups is shown in FIG. 25B. As can be seen in FIG. 25A, when the patients were grouped using DP Temra and DP Tern, the proportion of PR-patients in the Q1 group was significantly higher than that of the other groups, and when the patients were grouped using DP Temra and DN Tern, the proportion of PR-patients in the Q4 group was much higher than that of the other groups. That is, the results are consistent with the prediction that a patient with lower DP Temra and higher Fitness will have better responsiveness to cancer immunotherapy (an immune checkpoint inhibitor).

Further, the present inventors confirmed that the PR-patient proportions in the Q4 group based on DP Temra and DP Tern; and the Q1 group based on DP Temra and DN Tem are considerably higher than a PR-patient proportion confirmed using each marker alone (FIG. 25C). This means that the capacity of a combination of two markers, such as DP Temra and DP/DN Tern, to predict the responsiveness to cancer immunotherapy is superior to that of a single marker (DP Temra, DP Tern, or DN Tern) alone.

**Example III-2. A graph (pGRAPH) for predicting the responsiveness to cancer immunotherapy using K-NN machine learning is established.**

The present inventors attempted to improve the graph established in the previous Example by eliminating the threshold. Accordingly, the frequency of PR patients among the closest 7 patients (7-NN) in every plot on two graphs (Temra/DP Tem graph; and DP Temra/DN Tem graph) was confirmed using K-NN machine learning, and as the frequency of the PR patients is higher, it is marked in dark red. A plot without 7 patients in the vicinity were marked in gray. The graph established through the K-NN machine learning was named probability graph (pGRAPH).

As a result, on the Temra/DP Tern-based pGRAPH, a plot with lower DP Temra and higher DP Tem showed strong red, and on the Temra/DN Tern-based pGRAPH, a plot with lower DP Temra and lower DN Tem showed strong red (FIG. 26). That is, this is consistent with the tendency confirmed in the previous example (the lower the DP Temra and the higher the Fitness, the higher the responsiveness to immunotherapy).

The pGRAPH established in this example allows the percentage (%) of probability of PR to be predicted based on the information of nearby patients for each plot of the DP TemralFitness graph (PR%, for example, when PR patient=0, PR%=0%; when PR patient=1, PR%=14.3% (1/7); when PR patient=2, PR%=28.6% (2/7)). That is, PR% of each plot indicates the probability that the corresponding patient will show a partial response after cancer immunotherapy.

**Example III-3. Verification of capacity of pGRAPH to predict responsiveness to cancer immunotherapy**

To verify whether the pGRAPH established in the previous example can actually predict the responsiveness of a cancer patient to cancer immunotherapy, the responsiveness of an NSCLC patient to cancer immunotherapy was predicted using pGRAPH.

Specifically, after measuring DP Temra and DN Tem levels for each patient, these values were plotted on pGRAPH to obtain a PR% value (predicted PR%) corresponding to the plot. In addition, it was verified that the confirmed PR% value was consistent with the actual clinical prognosis of each patient. As clinical prognostic factors, actual PR%, actual durable clinical benefit (DCB: partial response (PR)+stable disease (SD) for 6 months)%, median progression-free survival (mPFS), and median overall survival (mOS) were used.

As a result, it is shown that the higher the predicted PR% of a patient, the higher the actual PR%, the actual DCB %, the mPFS, and the mOS (FIG. 27A). Moreover, as a result of confirming the mPFS and mOS according to the predicted PR% of a patient, the higher the predicted PR%, the longer the survival period (FIG. 27B). The above results demonstrate that the pGRAPH according to the present invention can be used as a diagnostic model for predicting a clinical prognosis including the responsiveness of a patient to cancer immunotherapy.

**Example III-4. pGRAPH has a better function of predicting prognosis (including responsiveness to cancer immunotherapy) than conventional biomarkers.**

Next, to confirm how well the pGRAPH according to the present invention functions as a biomarker for the responsiveness of a cancer patient to cancer immunotherapy, it was compared with the function of the conventional marker for predicting the responsiveness to immunotherapy. Since the pGRAPH is based on PBMCs, that is, blood biomarkers (DP Temra, DP/DN Tern, etc.), the pGRAPH of the present invention was compared with blood biomarkers such as a neutrophil-to-lymphocyte ratio (NLR) and a platelet-to-lymphocyte ratio (PLR) for accurate comparison. In addition, the pGRAPH of the present invention was also compared with an EGFR mutation level and a PD-L1 expression level, which are biomarkers that can only be confirmed by tumor surgery.

Specifically, analysis was conducted on 120 NSCLC stage III/IV patients subjected to cancer immunotherapy (PD-1/PD-L1 therapy; Keytruda or Tecentriq) (18 patients subjected to Keytruda and 102 patients subjected to Tecentriq). The threshold of each biomarker was set according to the following criteria: (1) setting the proportion of patients who pass the threshold to 30% (20% for patients receiving Tecentriq); and (2) setting a point where the objective response rate (ORR) is higher when the proportion is more than 30%, compared to when the proportion is 30%, as a threshold.

The results are shown in Tables 3 and 4 below. Table 3 shows the analysis results for all patients, and Table 4 shows the analysis results for only patients receiving Tecentriq.

[Table 3]

| Total (120 patients) | Biomarker source | ORR | DCB | mPFS | mOS | AUC | Proportion |
|---|---|---|---|---|---|---|---|
| No biomarker | | 19.2 | 27.5 | 5.3 | 10.5 | 0.500 | 100.0 |
| NLR | PBMC | 20.5 | 33.3 | 5.9 | 13.6 | 0.496 | 32.5 |
| PLR | PBMC | 25.5 | 39.2 | 6.9 | 12.3 | 0.548 | 42.5 |
| EGFR mutation | Tumor | 25.0 | 34.7 | 5.9 | 11.0 | 0.620 | 60.0 |
| PD-L1 | Tumor | 28.6 | 45.7 | 7.4 | 12.4 | 0.629 | 30.7 |
| pGRAPH | PBMC | 38.9 | 47.2 | 7.8 | 11.0 | 0.710 | 30.0 |

[Table 4]

| Tecentriq (102 patients) | Biomarker source | ORR | DCB | mPFS | mOS | AUC | Proportion |
|---|---|---|---|---|---|---|---|
| No biomarker | | 15.7 | 20.6 | 4.6 | 9.9 | 0.500 | 100.0 |
| NLR | PBMC | 15.9 | 25.4 | 5.1 | 11.3 | 0.464 | 61.2 |
| PLR | PBMC | 22.2 | 33.3 | 6.0 | 11.3 | 0.569 | 43.7 |
| EGFR mutation | Tumor | 20.0 | 25.0 | 4.9 | 9.9 | 0.613 | 81.1 |
| PD-L1 | Tumor | 20.8 | 29.2 | 5.7 | 11.0 | 0.532 | 24.7 |
| pGRAPH | PBMC | 39.1 | 39.1 | 7.2 | 10.3 | 0.767 | 22.5 |

As shown in the two tables, the biomarker, pGRAPH, according to the present invention was found to be superior to conventional prognosis-predicting biomarkers in terms of cancer immunotherapy responsiveness- and prognosis-prediction function. Particularly, a PD-L1 expression level in tumor tissue is used as the most representative biomarker for predicting responsiveness to PD-1 therapy, and despite being a blood-based biomarker, pGRAPH showed a much higher ORR compared to PD-L1 expression (38.9% vs. 28.6%). In addition, in the case of Tecentriq therapy, it is known that the PD-L1 expression level does not function as an accurate biomarker, and in fact, in the above experiment, the analysis result for patients receiving only Tecentriq therapy showed a lower prediction function of PD-L1 than the analysis results of all patients. However, in the case of pGRAPH of the present invention, a high prediction function was maintained even in the analysis result for the patients receiving only Tecentriq therapy.

**Example III-5. pGRAPH can accurately predict a prognosis even for a patient not used for machine learning.**

To verify the usefulness of pGRAPH as a prognosis prediction model, it was confirmed that an excellent prognosis prediction function was maintained even for patients who were not used for pGRAPH establishment (machine learning).

To this end, from 120 NSCLC patients, 102 patients not receiving Tecentriq therapy were selected and were further divided into groups of 72 patients and 30 patients according to the experimental set. Subsequently, machine learning was performed with a group of 72 patients, pGRAPH was constructed, and the constructed pGRAPH predicted the prognosis of the remaining 30 patients.

After confirming the predicted PR% of the 30 other patients with pGRAPH produced in the group of 72 patient group and comparing it with the actual prognostic factors (actual PR%, actual DCB%, mPFS, and mOS), consistent with the previous results, it was confirmed that the higher the predicted PR%, the better the prognosis (the actual PR%, the actual DCB%, high mPFS, and mOS) (FIG. 28A), and the progression-free survival and overall survival were also longer (FIG. 28B).

The above results support that the pGRAPH according to the present invention exhibits an excellent prognosis prediction function even for patients not used for machine learning.

**Example III-6. pGRAPH implemented with factors other than DP Temra and the Fitness factor has low prognosis prediction capacity.**

Since it was confirmed that pGRAPH based on DP Temra and the Fitness factor had an excellent capacity to predict the prognosis of cancer patients through the previous example, it was confirmed whether pGRAPH could be created using factors other than DP Temra and the Fitness factor.

Specifically, unlike DP Tem% and DN Tem% showing a constant change depending on the degree of tumor progression, focusing on maintaining a constant level of SP Tem% regardless of the degree of tumor progression (FIG. 29A), pGRAPH was constructed based on SP Temra and SP Tern. As in Example III-5, pGRAPH was constructed with SP Temra and SP Tem data of 72 cancer patients, and the prognosis of 30 other cancer patients was predicted with the pGRAPH.

The pGRAPH constructed based on SP Temra and SP Tem is shown in FIG. 29B, and here, the data of 30 cancer patients was entered and the predicted PR% was compared with the actual prognosis (actual PR%, actual DCB%, mPFS, and mOS). As a result, different from that the pGRAPH constructed with DP Temra and Fitness, which tends to match actual prognosis, pGRAPH constructed based on SP Temra and SP Tem showed a sporadic pattern, rather than a consistent pattern (FIG. 29C). That is, unlike the DP Temra and Fitness-based pGRAPH, it was confirmed that the SP Temra and SP Tern-based pGRAPH had a very low capacity to predict the prognosis of a new patient.

The above results ultimately demonstrate the excellent prognosis prediction function of the DP Temra and Fitness-based pGRAPH according to the present invention.

**Example III-7. Confirmation of responsiveness of CD8+ T subsets to IL-2**

To further verify the present invention, the responsiveness of cancer patients to IL-2 therapy was predicted using DP Temra and the Fitness factor. First of all, the responsiveness of CD8+ T cell subsets (Tn, DP Tern, DN Tern, and Temra) to IL-2 was confirmed.

Specifically, healthy donor PBMCs were classified into Tn, DP Tern, DN Tern, and Temra cells, and labeled with CTV to measure the degree of division of the classified cells. The CD8+ T cell subsets were treated with IL-2 (50 ng/ml) and cultured for 7 days, but some of them were stimulated by treatment with anti-CD8/CD28 (i.e., TCR stimulation) for comparison. After completing the 7-day culture and then treating with PMA/ionomycin for 5 hours, the proportions of the IFN-γ-expressing CD8+ T cell subset and the IL-2-expressing CD8+ T cell subset were measured by flow cytometry. In addition, the degree of CTV dilution was observed to measure a division index.

The results are shown in FIGS. 30A-30D. In the case of IL-2 stimulation, it was confirmed that, in the order of Tn<DP Tem<DN Tem<Temra, the proportions of IFN-γ-expressing cells and perforin-expressing cells were high, and cell proliferation further occurred (FIG. 30A). Particularly, the division index had the opposite pattern for TCR-stimulated cells (FIG. 30B).

Anti-PD-1/PD-L1 therapy achieves an anticancer effect by inhibiting the inhibitory effect of PD-1 on TCR stimulation. However, cancer patients with a high proportion of highly divided cells (DN Tem and Temra) are likely to have little responsiveness to TCR stimulation. Cancer patients with a high DN Tem proportion ultimately have low Fitness according to the present invention, and are those corresponding to Q3 or Q4 on the 2D graph based on DP Temra and DP Tem (patients with the lowest responsiveness to anti-PD-L1). That is, it is expected that these patients have higher responsiveness to IL-2 therapy than to anti-PD-1 or anti-PD-L1 therapy.

**Example III-8. Confirmation of responsiveness to IL-2 therapy according to initial Temra proportion**

Through the previous example, it was confirmed that even cancer patients with a high proportion of more differentiated cells can exhibit high responsiveness to IL-2 treatment. Therefore, it was confirmed whether cancer patients with a large amount of differentiated cells might actually show better responsiveness to IL-2 treatment.

Specifically, CD8$^+$ T cells were isolated from PBMCs of a healthy subject through FACS sorting, and here, at the time of isolation, the proportion of the most differentiated cell subset Temra was measured. The isolated CD8$^+$ T cells were treated with IL-2 (50 ng/ml) or a TCB2-IL-2 complex (50 ng/ml; Selecxine) and cultured for 7 days. After 7 days, the cells were treated with PMA/ionomycin, an IFN-$\gamma$ expressing level and a perforin-expressing level were measured, and a division index was then obtained by CTV dilution. Further, the same experiment as described above was performed on tilCD8$^+$ T cells to examine whether it could also be applied to tumor-specific CD8$^+$ T cells.

The result for PBMCs stimulated with IL-2 is shown in FIG. 31B, the result for PBMCs stimulated with the TCB2-IL-2 complex is shown in FIG. 31C, and the result for tilCD8$^+$ T cells stimulated with IL-2 is shown in FIG. 31D. In all three cases, patients who initially show a high proportion of Temra had high perforin and IFN-$\gamma$ expressing levels, and a high division index. These results suggest that before treatment, patients with a higher Temra proportion show higher responsiveness to IL-2 therapy.

Example III-9. Prediction of responsiveness to IL-2 therapy using **DP** Temra and Fitness factor

Finally, the responsiveness to IL-2 therapy was predicted using DP Temra and the Fitness factor.

Specifically, a 2D graph was created based on DP Temra and Fitness factor data measured from patients' blood, and a Temra proportion (tilTemra %) was obtained in CD8$^+$ T cells obtained from a patient's tumor-infiltrating lymphocytes. Subsequently, the 2D graph was divided into quadrants (Q1 to Q4), and the tilTemra % of patients included in each quadrant was then compared.

As a result, it was confirmed that the tilTemra % was the lowest in Q1, and tilTemra % was the highest in Q3/Q4 (FIGS. 32A and 32B). Since the tilTemra % is proportional to the responsiveness of tilCD8$^+$ T cells to IL-2 therapy, it is expected that patients belonging to Q3 or Q4 will have the highest responsiveness to IL-2 therapy. That is, this supports that (i) the higher the proportion of Temra among total CD8$^+$ T cells, the higher the responsiveness of cancer patients to IL-2 therapy (IL-2 alone or co-treatment), and (ii) the lower the proportion of DP Tern among total Tern, the higher the responsiveness of cancer patients to IL-2 therapy.

**Example III-10. Additional exploration of cell subsets that can be utilized as Fitness factor**

As the Fitness factor according to the present invention is a marker representing a cancer patient's immunity, that is, the capacity to respond to immunotherapy, any cell subset that is changed in proportion to the tumor stage, such as DP Tem/DN Tem can be used as a Fitness factor. Particularly, it is also possible to identify DP Tem/DN Tem with other molecular markers other than CD27 and CD28. Particularly, DP Tem and DN Tem may be indirectly identified with any marker that is overall changed in the order of DP Tem→SP Tem→DN Tern. Therefore, it was confirmed by flow cytometry which marker exhibits a change pattern in this order.

As a result, it was confirmed that CD28, CD27, TCF1, CD127, CD45RO, CXCR3, CD69, and PD1 were expressed in the order of DP Tem>SP Tem>DN Tern; and CD45RA, perforin, and CD57 are expressed in the order of DN Tem>SP Tem>DP Te (FIG. 33).

That is, even if not necessarily by CD27 or CD28, DP Tem and DN Tem may be indirectly defined using the above markers, and thus they can be used as a Fitness factor. Furthermore, even if it is not the above markers, any molecular marker that shows a specific expression change pattern according to a CD8$^+$ cell subset as above can be utilized as a Fitness factor.

In conclusion, it was proved that the DP Temra and the Fitness factor according to the present invention are biomarkers representing the tumor state and immunity of a cancer patient, respectively, and may be utilized as independent biomarkers for the responsiveness of a cancer patient to cancer immunotherapy, and the combination of the two biomarkers can serve as a potent biomarker for predicting the prognosis of a cancer patient (FIG. 34). Therefore, it is expected that it is possible to perform more effective and efficient patient-specific treatment by accurately predicting the responsiveness of a cancer patient to cancer immunotherapy using a biomarker according to the present invention. Furthermore, the present invention provides a DP Temra and Fitness factor-based model for predicting the prognosis of a cancer patient through machine learning, and thus is expected to be useful in the field of cancer treatment.

It should be understood by those of ordinary skill in the art that the above descriptions of the present invention are exemplary, and the example embodiments disclosed herein can be easily modified into other specific forms without changing the technical spirit or essential features of the present invention. Therefore, it should be interpreted that the example embodiments described above are exemplary in all aspects, and are not limitative.

[Industrial Applicability]

The present invention relates to a method of predicting the prognosis or responsiveness to anticancer treatment of a cancer patient, and thus prognosis and the responsiveness to anticancer treatment can be accurately predicted by measuring the proportion of a specific CD8$^+$ T cell subset. Particularly, the present invention provides a system capable of rapidly and simply calculating the probability that a patient will show a response to anticancer treatment by applying machine learning to the prediction method, so the responsiveness of a cancer patient to anticancer treatment can be more efficiently predicted. Particularly, since the prediction method according to the present invention uses a non-invasive sample, rapid and accurate prognosis can be predicted without cancer tissue analysis or the like.

**Claims**

1. An information providing method for predicting the prognosis or responsiveness of a cancer patient to anticancer treatment, comprising:

   measuring the proportion of a CD8$^+$ T cell subset in a biological sample isolated from a subject,
   wherein the proportion of the CD8$^+$ T cell subset is the proportion of CD27$^+$ CD28$^+$ CCR7$^-$ CD8$^+$ T cells among total CCR7$^-$ CD8$^+$ T cells.

2. The information providing method of claim 1, further comprising:

   measuring a Fitness factor,
   wherein the Fitness factor includes one or more selected from the group consisting of the following:

   (a) the proportion of CD27$^+$ CD28$^+$ CCR7$^-$ CD45RA$^-$ CD8$^+$ T cells among total CCR7$^-$ CD45RA$^-$ CD8$^+$ T cells;
   (b) the proportion of CD27$^-$ CD28$^-$ CCR7$^-$ CD45RA$^-$ CD8$^+$ T cells among total CCR7$^-$ CD45RA$^-$ CD8$^+$ T cells;
   (c) the proportion of perforin$^+$ CCR7$^-$ CD45RA$^-$ CD8$^+$ T cells among total CCR7$^-$ CD45RA$^-$ CD8$^+$ T cells;
   (d) the proportion of granzyme B$^+$ CCR7$^-$ CD45RA$^-$ CD8$^+$ T cells among total CCR7$^-$ CD45RA$^-$ CD8$^+$ T cells; and
   (e) the proportion of IL-2$^+$ CCR7$^-$ CD45RA$^-$ CD8$^+$ T cells among total CCR7$^-$ CD45RA$^-$ CD8$^+$ T cells.

3. The information providing method of claim 1 or 2, wherein the anticancer treatment includes one or more selected from the group consisting of cancer immunotherapy, chemotherapy, radiation therapy, and a combination of chemotherapy-radiation therapy.

4. The information providing method of claim 3, wherein the cancer immunotherapy is treatment with an immune checkpoint inhibitor or an immune cell activator.

5. The information providing method of claim 3, wherein the cancer immunotherapy includes one or more selected from the group consisting of anti-PD-L1 treatment, anti-PD-1 treatment, anti-CTLA-4 treatment, anti-LAG3 treatment, anti-TIM3 treatment, anti-BTLA treatment, anti-4-1BB treatment, anti-OX40 treatment, a cytokine treatment, IL-2 treatment, and cytokine receptor treatment.

6. The information providing method of claim 1, wherein the anticancer treatment is treatment with an immune checkpoint inhibitor, and the information providing method further comprises, when the proportion of CD27$^+$ CD28$^+$ CCR7$^-$ CD8$^+$ T cells among total CCR7$^-$ CD8$^+$ T cells is lower than that of the control, determining that a subject has a better prognosis or predicting that the responsiveness to the anticancer treatment is higher.

7. The information providing method of claim 2, wherein the anticancer treatment is treatment with an immune checkpoint inhibitor, and the information providing method further comprises, when one or more of the following characteristics are satisfied, determining that a subject has a better prognosis, or predicting that the responsiveness to the anticancer treatment is higher:

   (a) the proportion of CD27$^+$ CD28$^+$ CCR7$^-$ CD45RA$^-$ CD8$^+$ T cells among total CCR7$^-$ CD45RA$^-$ CD8$^+$ T cells is higher than that of the control; and
   (b) the proportion of CD27' CD28$^-$ CCR7$^-$ CD45RA$^-$ CD8$^+$ T cells among total CCR7$^-$ CD45RA$^-$ CD8$^+$ T cells is lower than that of the control.

8. The information providing method of claim 2, wherein the anticancer treatment is treatment with an immune checkpoint inhibitor, and the subject is a subject before receiving the anticancer treatment, and the information providing method further comprises, when one or more of the following characteristics are satisfied, determining that a subject has a better prognosis, or predicting that the responsiveness to the anticancer treatment is higher:

   (c) the proportion of perforin$^+$ CCR7$^-$ CD45RA$^-$ CD8$^+$ T cells among total CCR7$^-$ CD45RA$^-$ CD8$^+$ T cells is lower than that of the control;
   (d) the proportion of granzyme B$^+$ CCR7$^-$ CD45RA$^-$ CD8$^+$ T cells among total CCR7$^-$ CD45RA$^-$ CD8$^+$ T cells is lower than that of the control; and
   (e) the proportion of IL-2$^+$ CCR7$^-$ CD45RA$^-$ CD8$^+$ T cells among total CCR7$^-$ CD45RA$^-$ CD8$^+$ T cells is higher than that of the control.

9. The information providing method of claim 1, wherein the anticancer treatment is IL-2 treatment and the subject is a subject before receiving the anticancer treatment, and the information providing method further comprises, when the proportion of CCR7$^-$ CD45RA$^+$CD8$^+$ T cells among total CD8$^+$ T cells is higher than that of the control, determining that a subject has a better prognosis, or predicting that the responsiveness to the anticancer treatment is higher.

10. The information providing method of claim 2, wherein the anticancer treatment is IL-2 treatment, and the information providing method further comprises, when the proportion of CD27$^+$ CD28$^+$ CCR7$^-$ CD45RA$^-$ CD8$^+$ T cells among total CCR7$^-$ CD45RA$^-$ CD8$^+$ T cells is lower than that of the control, determining that a subject has a better prognosis, or predicting that the responsiveness to the anticancer treatment is higher.

11. The information providing method of claim 9 or 10, wherein the IL-2 treatment is treatment with a conjugate of IL-2; and an anti-IL-2 antibody or a fragment thereof.

12. The information providing method of any one of claims 6 to 10, wherein the control is a biological sample isolated from a healthy subject or a cancer patient showing a complete response or partial response after anticancer treatment.

13. The information providing method of claim 1, wherein the sample is one or more selected from the group consisting of blood, peripheral blood, whole blood, plasma and cell.

14. The information providing method of claim 1, wherein the CD8$^+$ T cell subset is a CD8$^+$ T cell subset in peripheral blood.

15. The information providing method of claim 1, wherein the cancer is any one or more selected from the group consisting of melanoma, small cell lung cancer, non-small cell lung cancer, gliomas, liver cancer, thyroid tumors, stomach cancer, prostate cancer, ovarian cancer, bladder cancer, lung cancer, colorectal cancer, breast cancer, Hodgkin's lymphoma, urothelial carcinoma, glioblastoma, endometrial cancer, cervical cancer, kidney cancer, colon cancer, pancreatic cancer, esophageal carcinoma, head and neck cancer, mesothelioma, sarcoma, cholangiocarcinoma, small intestine adenocarcinoma, childhood malignancies, epidermal cancer, testicular cancer, blood cancer, and brain cancer.

16. A composition for predicting the prognosis or responsiveness to anticancer treatment of a cancer patient, comprising an agent for detecting CCR7$^-$ CD8$^+$ T cells and CD27$^+$ CD28$^+$ CCR7$^-$ CD8$^+$ T cells.

17. The composition of claim 16, further comprising:
   an agent for detecting one or more CD8$^+$ T cell subsets selected from the group consisting of the following:

   CCR7$^-$ CD45RA$^-$ CD8$^+$ T cells;
   CD27$^+$ CD28$^+$ CCR7$^-$ CD45RA$^-$ CD8$^+$ T cells;
   CD27$^-$ CD28$^-$ CCR7$^-$ CD45RA$^-$ CD8$^+$ T cells;
   perforin$^+$ CCR7$^-$ CD45RA$^-$ CD8$^+$ T cells;
   granzyme B$^+$ CCR7$^-$ CD45RA$^-$ CD8$^+$ T cells; and
   IL-2$^+$ CCR7$^-$ CD45RA$^-$ CD8$^+$ T cells.

18. The composition of claim 16, wherein the agent is an antibody or an aptamer.

19. A kit for predicting the prognosis of responsiveness to anticancer treatment of a cancer patient, comprising the composition of claim 16.

**20.** The kit of claim 19, further comprising:
an agent for detecting total CD8$^+$ T cells.

**21.** The kit of claim 19, further comprising:
instructions that describe the information providing method of claim 1 or 2.

**22.** A method of predicting the prognosis or responsiveness to anticancer treatment of a cancer patient, comprising:
measuring the proportion of a CD8$^+$ T cell subset in a biological sample isolated from a subject, wherein the proportion of the CD8$^+$ T cell subset is the proportion of CD27$^+$ CD28$^+$ CCR7$^-$ CD8$^+$ T cells among total CCR7$^-$ CD8$^+$ T cells.

**23.** A use of an agent for detecting CCR7$^-$ CD8$^+$ T cells and CD27$^+$ CD28$^+$ CCR7$^-$ CD8$^+$ T cells to prepare an agent for predicting the prognosis or responsiveness to anticancer treatment of a cancer patient.

**24.** A use of an agent for detecting CCR7$^-$ CD8$^+$ T cells and CD27$^+$ CD28$^+$ CCR7$^-$ CD8$^+$ T cells to predict the prognosis or responsiveness to anticancer treatment of a cancer patient.

**FIG. 1A**

**FIG. 1B**

**FIG. 1C**

**FIG. 1D**

**FIG. 1E**

**FIG. 1F**

**FIG. 1G**

**FIG. 1H**

**FIG. 2A**

**FIG. 2B**

EP 4 321 625 A1

**FIG. 2C**

44

**FIG. 2D**

**FIG. 3A**

**FIG. 3B**

**FIG. 3C**

**FIG. 3D**

**FIG. 3E**

**FIG. 3F**

**FIG. 3G**

**FIG. 3H**

**FIG. 3I**

FIG. 3J

# FIG. 4A

## FIG. 4B

**FIG. 4C**

**FIG. 4D**

**FIG. 4E**

**FIG. 5A**

**FIG. 5B**

**FIG. 5C**

**FIG. 5D**

**FIG. 5E**

**FIG. 5F**

**FIG.5G**

CD28

CD27

CD45RA

**FIG. 5H**

**FIG. 6A**

**FIG. 6B**

**FIG. 6C**

**FIG. 6D**

**FIG. 6E**

SCC | Non-SCC

TMB: 9 | 6

DP pTemra high: 20% | 45%

⬤ DP pTemra low
⬤ DP pTemra high

FIG.6F

**FIG.6G**

**FIG.6H**

## FIG.6I

## FIG.7A

**FIG. 7B**

**FIG. 7C**

**FIG. 7D**

**FIG. 7E**

**FIG. 7F**

FIG.8A

**FIG.8B**

**FIG.8C**

**FIG.8D**

**FIG.8E**

**FIG. 8F**

T Cell Mediated Cytotoxicity
(GO:0001913)

**FIG. 8G**

Positive Regulation of
Alpha Beta T Cell Proliferation
(GO:0046641)

**FIG.9A**

**FIG. 9B**

**FIG. 9C**

**FIG. 9D**

**FIG. 9E**

**FIG.9F**

**FIG. 9G**

**FIG. 9H**

**FIG. 9I**

**FIG.10A**

**FIG.10B**

**FIG.10C**

EP 4 321 625 A1

FIG.11A

**FIG. 11B**

**FIG. 11C**

**FIG. 11D**

FIG.11E

**FIG. 11F**

anti-CD3/28

**FIG. 11G**

IL-2

**FIG.11H**

**FIG. 11I**

**FIG. 12A**

## FIG.12B

**FIG.12C**

**FIG.12D**

# FIG. 12E

**FIG. 12F**

**FIG. 12G**

**FIG. 12H**

**FIG. 12I**

**FIG.13**

**FIG.14**

**FIG. 15**

**FIG. 16**

**FIG. 17**

FIG.18

EP 4 321 625 A1

**FIG. 19A**

**FIG. 19B**

**FIG.20A**

**FIG.20B**

**FIG.21A**

**FIG.21B**

**FIG.22A**

FIG. 22B

**FIG.23A**

**FIG.24**

**FIG.25A**

Using DP Temra + DP Tem

Using DP Temra + DN Tem

FIG.25B

**FIG. 25C**

**FIG.26**

**FIG. 27A**

## FIG.27B

**FIG. 28A**

**FIG.28B**

**FIG. 29A**

**FIG.29B**

**FIG. 29C**

## FIG.30A

**FIG. 30B**

**FIG.31A**

**FIG.31B**

**FIG.31C**

**FIG.31D**

**FIG.32A**

FIG. 32B

**FIG.33**

**FIG. 34**

## INTERNATIONAL SEARCH REPORT

| International application No. |
| --- |
| **PCT/KR2022/004909** |

**A. CLASSIFICATION OF SUBJECT MATTER**

**C12Q 1/02**(2006.01)i; **G01N 33/574**(2006.01)i; **C12Q 1/6886**(2018.01)i; **C12Q 1/6869**(2018.01)i; **A61P 35/00**(2006.01)i

According to International Patent Classification (IPC) or to both national classification and IPC

**B. FIELDS SEARCHED**

Minimum documentation searched (classification system followed by classification symbols)

C12Q 1/02(2006.01); C12Q 1/6886(2018.01); G01N 33/50(2006.01); G01N 33/574(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: CD8+ T 세포(CD8+ T cell), 아형(subset), TEMRA, CD45RA, CCR7, CD27, CD28, 암(cancer), 예후(prognosis)

**C. DOCUMENTS CONSIDERED TO BE RELEVANT**

| Category* | Citation of document, with indication, where appropriate, of the relevant passages | Relevant to claim No. |
| --- | --- | --- |
| A | READING, James L. et al. The function and dysfunction of memory CD8+ T cells in tumor immunity. Immunological Reviews. May 2018, vol. 283, no. 1, pp. 194-212.<br>　　　See pages 196, 202-203 and 206. | 1-24 |
| A | WISTUBA-HAMPRECHT, Kilian et al. Peripheral CD8 effector-memory type 1 T-cells correlate with outcome in ipilimumab-treated stage IV melanoma patients. European Journal of Cancer. 04 February 2017 (online publication date), vol. 73, pp. 61-70.<br>　　　See pages 63-64 and 68. | 1-24 |
| A | US 2019-0178892 A1 (INSTITUT NATIONAL DE LA SANTE ET DE LA RECHERCHE MEDICALE (INSERM) et al.) 13 June 2019 (2019-06-13)<br>　　　See entire document. | 1-24 |
| A | WO 2012-038068 A2 (GRABE, Niels) 29 March 2012 (2012-03-29)<br>　　　See entire document. | 1-24 |

☑ Further documents are listed in the continuation of Box C. ☑ See patent family annex.

| | |
| --- | --- |
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **21 July 2022** | **22 July 2022** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2022/004909** |

### C.     DOCUMENTS CONSIDERED TO BE RELEVANT

| Category* | Citation of document, with indication, where appropriate, of the relevant passages | Relevant to claim No. |
| --- | --- | --- |
| A | WISTUBA-HAMPRECHT, Kilian et al. Phenotypic characterization and prognostic impact of circulating γδ and αβ T-cells in metastatic malignant melanoma. International Journal of Cancer. 27 August 2015 (online publication date), vol. 138, pp. 698-704.<br><br>        See entire document. | 1-24 |
| PX | LEE, Sung-Woo et al. CD8+ TILs in NSCLC differentiate into TEMRA via a bifurcated trajectory: deciphering immunogenicity of tumor antigens. Journal for ImmunoTherapy of Cancer. September 2021, vol. 9, no. 9, e002709, pp. 1-14.<br><br>        See abstract; pages 3-5, 7, 10 and 12-13; and figures 1 and 6. | 1-24 |

Form PCT/ISA/210 (second sheet) (July 2019)

<table>
<tr><th colspan="2">INTERNATIONAL SEARCH REPORT<br>Information on patent family members</th><th colspan="2">International application No.<br><br>PCT/KR2022/004909</th></tr>
<tr><th>Patent document<br>cited in search report</th><th>Publication date<br>(day/month/year)</th><th>Patent family member(s)</th><th>Publication date<br>(day/month/year)</th></tr>
<tr><td>US 2019-0178892 A1</td><td>13 June 2019</td><td>EP 1777523 A1</td><td>25 April 2007</td></tr>
<tr><td></td><td></td><td>EP 1943520 A1</td><td>16 July 2008</td></tr>
<tr><td></td><td></td><td>EP 1943520 B1</td><td>07 September 2011</td></tr>
<tr><td></td><td></td><td>EP 2241891 A1</td><td>20 October 2010</td></tr>
<tr><td></td><td></td><td>EP 2241891 B1</td><td>31 October 2012</td></tr>
<tr><td></td><td></td><td>EP 2420835 A2</td><td>22 February 2012</td></tr>
<tr><td></td><td></td><td>EP 2420835 B1</td><td>05 March 2014</td></tr>
<tr><td></td><td></td><td>EP 2733493 A1</td><td>21 May 2014</td></tr>
<tr><td></td><td></td><td>EP 2733493 B1</td><td>22 June 2016</td></tr>
<tr><td></td><td></td><td>EP 3088894 A1</td><td>02 November 2016</td></tr>
<tr><td></td><td></td><td>EP 3088894 B1</td><td>28 March 2018</td></tr>
<tr><td></td><td></td><td>EP 3364191 A1</td><td>22 August 2018</td></tr>
<tr><td></td><td></td><td>EP 3364191 B1</td><td>18 March 2020</td></tr>
<tr><td></td><td></td><td>EP 3715849 A1</td><td>30 September 2020</td></tr>
<tr><td></td><td></td><td>JP 2009-515148 A</td><td>09 April 2009</td></tr>
<tr><td></td><td></td><td>JP 2012-154944 A</td><td>16 August 2012</td></tr>
<tr><td></td><td></td><td>JP 2014-158500 A</td><td>04 September 2014</td></tr>
<tr><td></td><td></td><td>JP 5256038 B2</td><td>07 August 2013</td></tr>
<tr><td></td><td></td><td>JP 5789216 B2</td><td>07 October 2015</td></tr>
<tr><td></td><td></td><td>JP 5921602 B2</td><td>24 May 2016</td></tr>
<tr><td></td><td></td><td>US 10191059 B2</td><td>29 January 2019</td></tr>
<tr><td></td><td></td><td>US 11300569 B2</td><td>12 April 2022</td></tr>
<tr><td></td><td></td><td>US 2009-0215053 A1</td><td>27 August 2009</td></tr>
<tr><td></td><td></td><td>US 2014-0057257 A1</td><td>27 February 2014</td></tr>
<tr><td></td><td></td><td>US 2015-0268245 A1</td><td>24 September 2015</td></tr>
<tr><td></td><td></td><td>US 8481271 B2</td><td>09 July 2013</td></tr>
<tr><td></td><td></td><td>WO 2007-045996 A1</td><td>26 April 2007</td></tr>
<tr><td>WO 2012-038068 A2</td><td>29 March 2012</td><td>EP 2619576 A2</td><td>31 July 2013</td></tr>
<tr><td></td><td></td><td>EP 2619576 B1</td><td>10 June 2020</td></tr>
<tr><td></td><td></td><td>US 2013-0330325 A1</td><td>12 December 2013</td></tr>
<tr><td></td><td></td><td>US 9726676 B2</td><td>08 August 2017</td></tr>
<tr><td></td><td></td><td>WO 2012-038068 A3</td><td>31 May 2012</td></tr>
</table>

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020210044488
- KR 1020220042365

- KR 102099593

**Non-patent literature cited in the description**

- **ROSENBERG, S.A.** *J Immunol,* 2014, vol. 192 (12), 5451-8
- **BRANDENBURG, S. et al.** *Eur J Immunol,* 2008, vol. 38 (6), 1643-53

- **FACCIABENE, A. et al.** *Cancer Res,* 2012, vol. 72 (9), 2162-71